# EUROPEAN PATENT APPLICATION

(11) **EP 4 641 202 A2**
(43) Date of publication of application: **29.10.2025**
(21) Application number: 25187665.2
(22) Date of filing: 25.05.2023
(51) Int. Cl.: G01N 33/574

(54) **COMPOSITIONS AND METHODS FOR MODULATION OF IMMUNE RESPONSES**

(30) Priority: 25.05.2022 US 202263345765 P
(62) Divisional of application: 23733180.6
(71) Applicant: Flagship Pioneering Innovations VII, LLC, Cambridge, MA 02142 (US)
(72) Inventor: MANI, Vinidhra, Cambridge (US); WEINSTEIN, Erica, Gabrielle, Cambridge (US); ECHELARD, Yann Paul Guy Régis, Cambridge (US); KAHVEJIAN, Avak, Cambridge (US); PENDERGRAST, Patrick, Shannon, Cambridge (US); OUSPENSKAIA, Tamara, Cambridge (US); BILLINGS, Gabriel Humphrey, Cambridge (US); GRISHKEVICH, Vlad, Cambridge (US); BAEK, Sungmin, Cambridge (US)
(74) Representative: Maiwald GmbH

(57) **Abstract**

The disclosure provides, in various embodiments, compositions, such as polypeptides, polynucleotides, gene editing system, small molecules, vectors or host cells, that comprises and/or modulate expression or activity of immune regulation-associated proteins. The disclosure also provides, in various embodiments, methods of treating aging, senescence, fibrosis, autoimmunity, cancer, an infection, and/or an immunological disease using an agent that comprises and/or modulates expression or activity of an immune regulation-associated protein and methods of identifying said agent.

## Description

### INCORPORATION BY REFERENCE OF MATERIAL

This application claims the benefit of U.S. Provisional Application No. 63/345,765, filed on May 25, 2022. The entire teachings of the above application are incorporated herein by reference.

### INCORPORATION BY REFERENCE OF MATERIAL IN XML

This application incorporates by reference the Sequence Listing contained in the following eXtensible Markup Language (XML) file being submitted concurrently herewith:
a) File name: 57081055002.xml; created May 23, 2023, 40,525,913 Bytes in size.

### BACKGROUND

The immune system's major function is to mediate protective immunity against pathogens, such as viruses, bacteria, fungi, parasites, and tumors. A variety of immune subversion mechanisms are often enhanced during cancer and chronic infections. Insufficient immune responses can lead to immunodeficiencies, susceptibility to infection, and cancer (e.g., tumor) progression. However, without tight regulation, the immune system can also promote acute and chronic inflammation, leading to immunopathology or autoimmunity. Therefore, there is a critical need to identify additional and novel therapeutic targets for the regulation of an immune cell.

### SUMMARY

The disclosure provided herein is based, in part, on the identification of non-canonical protein targets (e.g., proteins encoded by non-canonical open reading frames (ORFs)) for the regulation of an immune response.

In one aspect, the present disclosure relates to an agent that comprises and/or modulates (e.g., increases or decreases) the expression and/or activity of a target protein identified herein (e.g., a target protein listed in the Sequence Listing), or a variant thereof. In some embodiments, the agent comprises a target protein identified herein (e.g., a target protein listed in the Sequence Listing), or a variant thereof. In certain embodiments, the agent modulates (e.g., increases or decreases) the expression and/or activity of a target protein identified herein (e.g., a target protein listed in the Sequence Listing, or a variant thereof). In some embodiments, the agent comprises, consists essentially of, or consists of a polypeptide, a polynucleotide, a gene editing system, a small molecule, or a cell (e.g., a cell therapy). The agent can be an inhibitor or an activator of a target protein identified herein. In some embodiments, the agent modulates the expression of a target protein identified herein. In some embodiments, the agent modulates the activity of a target protein identified herein.

In another aspect, the disclosure provides a pharmaceutical composition comprising a target protein identified herein, and a pharmaceutically acceptable carrier.

In another aspect, the disclosure provides a pharmaceutical composition comprising an agent that modulates the expression or activity of a target protein identified herein, and a pharmaceutically acceptable carrier.

In other aspects, the disclosure relates to a polynucleotide encoding a polypeptide described herein, an expression vector comprising a polynucleotide encoding a polypeptide described herein, and a host cell comprising a polynucleotide encoding a polypeptide described herein.

In another aspect, the disclosure provides a method of detecting a disease or condition, or determining a likelihood of developing the disease or condition in a subject, comprising quantifying an expression or activity of a target protein in a sample from the subject, wherein the level of expression or activity of the target protein in the sample is indicative of the likelihood of developing the disease or condition in the subject, wherein the disease or condition is selected from aging, senescence, fibrosis, autoimmunity, cancer (e.g., tumor), an infection, an immunological disease (e.g., inflammation and/or an autoimmune disease), or a combination thereof.

In another aspect, the disclosure provides a method of preparing a sample that is useful for determining a likelihood of developing a disease or condition in a subject, comprising:
a) obtaining or having obtained a sample from the subject;
b) adding a protease inhibitor, a control peptide, a standard peptide, or a combination thereof to the sample to prepare a sample that is useful for detecting a likelihood of developing cancer; and
c) quantifying an expression or activity of a target protein in the sample prepared in step b),
wherein the disease or condition is selected from aging, senescence, fibrosis, autoimmunity, cancer (e.g., tumor), an infection, an immunological disease (e.g., inflammation and/or an autoimmune disease), or a combination thereof.

In some embodiments, the method further comprises treating a subject who is predicted to have a likelihood of developing the disease or condition, comprising administering to the subject an effective amount of an agent that comprises and/or modulates the expression or activity of the target protein identified herein, or a pharmaceutical composition comprising the agent.

In another aspect, the disclosure provides a method of treating a disease or condition in a subject in need thereof (e.g., a human subject having a cancer), comprising administering to the subject an effective amount of an agent that comprises and/or modulates the expression or activity of a target protein identified herein, or a pharmaceutical composition comprising the agent.

In another aspect, the disclosure provides a method of selecting a subject suitable for treatment of a disease or condition, comprising quantifying an expression or activity of a target protein in a sample from the subject, and selecting the subject suitable for treatment of the disease or condition according to the level of expression or activity of the target protein in the sample, wherein the disease or condition is selected from aging, senescence, fibrosis, autoimmunity, cancer (e.g., tumor), an infection, an immunological disease (e.g., inflammation and/or an autoimmune disease), or a combination thereof.

In another aspect, the disclosure provides a method of modulating the expression or activity of a target protein identified in the Sequence Listing, or a variant thereof in a cell (e.g., a cancer cell, such as a cancer cell in a subject), comprising contacting the cell (e.g., *in vitro, ex vivo,* or *in vivo*) with an agent that comprises and/or modulates the expression or activity of a target protein identified herein, or a pharmaceutical composition comprising the agent.

In another aspect, the disclosure provides a method of identifying an agent that modulates the expression or activity of a target protein identified herein, comprising:
a) contacting the target protein with an agent; and
b) determining whether the agent modulates the expression or activity of the target protein,
wherein a difference in the expression or activity of the target protein that has been contacted with the agent compared to a reference for the expression or activity of the target protein indicates that the agent modulates the expression or activity of the target protein.

### BRIEF DESCRIPTION OF THE DRAWINGS

The foregoing will be apparent from the following more particular description of example embodiments, as illustrated in the accompanying drawings in which like reference characters refer to the same parts throughout the different views. The drawings are not necessarily to scale, emphasis instead being placed upon illustrating embodiments.

FIG. 1 shows dose dependent reduction of hTNFa when compared to control (TA_H2O) on CD3/CD28 (TransAct) activated PBMCs. *** = p < 0.0002 and **** = p <0.0001 compared to respective controls by two-way ANOVA.

FIG. 2 shows lack of IL-1b inhibition on CD3/CD28 (TransAct) activated PBMCs when compared to control (TA_H2O). Positive control shows significant inhibition. ****= p <0.0001 compared to respective controls by two-way ANOVA.

FIG. 3 shows inhibition of Interferon Regulatory Factor (IRF) pathway by SEQ ID NO: 37413 protein treatment in THP-1 dual reporter cells. THP-1 dual reporter cells were incubated with increasing concentrations of the peptide (0, 2.5, 5, 7.5, and 10 µM) in the presence of lipopolysaccharide (LPS) for 24 hours. The relative IRF activity was assessed by measuring the reporter gene expression. Data are presented as mean ± SD of 10 technical replicates. Asterisks denote statistical significance compared to LPS-only treated cells (**p < 0.01, ****p < 0.0001).

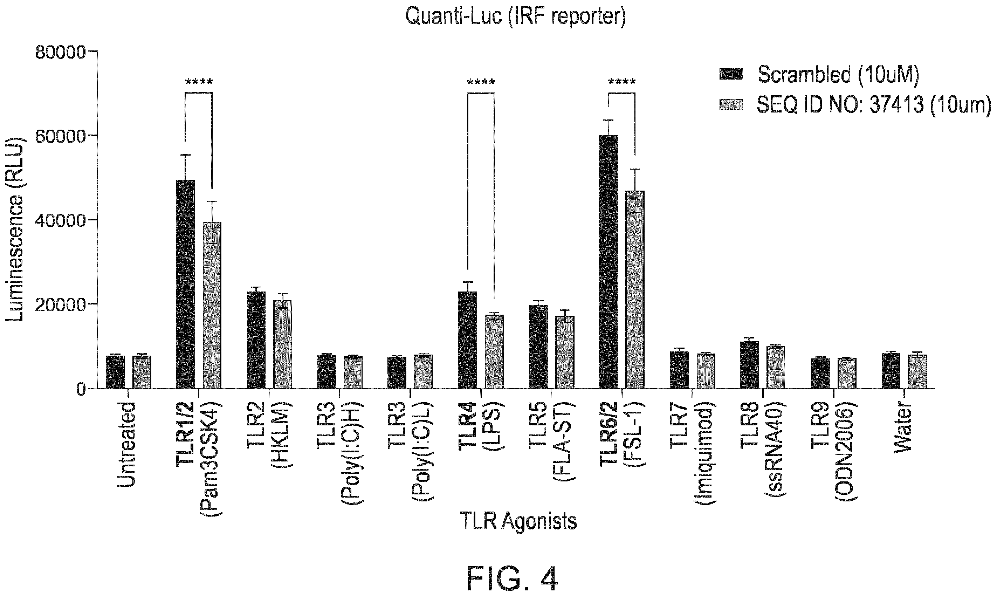

FIG. 4 shows selective inhibition of IRF response by SEQ ID NO: 37413 protein co-treatment with specific TLR agonists in THP-1 dual reporter cells. THP-1 dual reporter cells were treated with 10 different TLR agonists (TLR1/2, TLR2, TLR3, TLR4, TLR5, TLR6/2, TLR7, TLR8, and TLR9) in the presence of the SEQ ID NO: 37413 protein (10 µM) or scramble peptide for 24 hours. The relative IRF activity was assessed by measuring the reporter gene expression. Data are presented as mean ± SD of 10 technical replicates per treatment group from a representative experiment. Asterisks denote statistical significance compared to respective TLR agonist with scramble peptide treated cells (****p < 0.0001). The protein inhibited IRF response when co-treated with TLR1/2, TLR4, and TLR6/2 agonists, while no significant effect was observed with TLR2, TLR3, TLR5, TLR7, TLR8, or TLR9 agonists.

FIG. 5A shows UMAP visualization of cluster analysis reveals 10 distinct PBMC cell subtypes, while FIG. 5B shows bar graphs display the count of upregulated and downregulated genes when treated with SEQ ID NO: 37413 protein compared to the scrambled protein control.

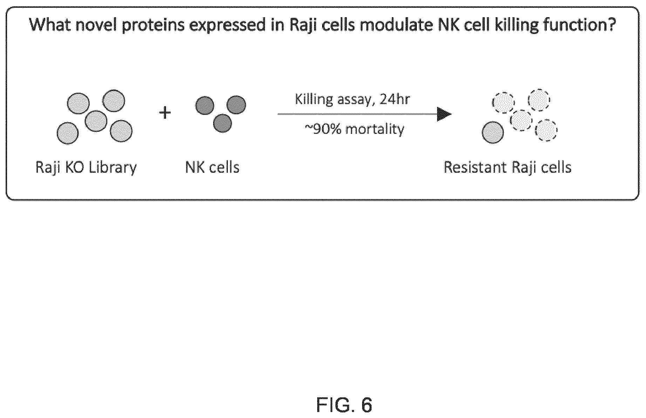

FIG. 6 shows screen design to validate the function of novel proteins in activation of Raji cell killing by primary NK cells.

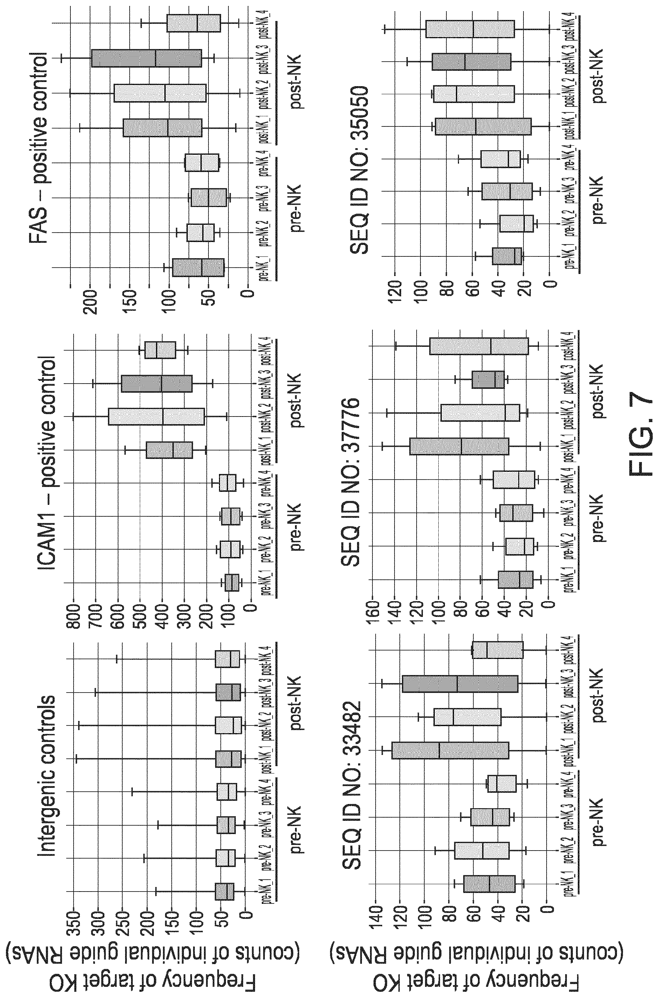

FIG. 7 shows CRISPR mediated KO of known controls ICAM1 and FAS are enriched in post-NK Raji cells supporting their role in potentiating NK cytotoxicity. Similar enrichment is observed for three exemplary novel proteins SEQ ID NO: 33482, SEQ ID NO: 33482, SEQ ID NO: 37776 and SEQ ID NO: 35050. As expected, there is no enrichment of intergenic (neutral) control guide RNAs. Boxplots summarize effects for numerous guide RNAs targeting each target (n=4); effect across 4 biological replicates for pre-NK and post-NK samples is shown.

FIG. 8 shows screen design to validate novel proteins function on inhibition of Raji cells killing by primary NK cells.

FIG. 9 shows CRISPR mediated KO of known controls B2M and TAP1 are depleted in post-NK Raji cells supporting their role in protecting cells against NK cytotoxicity. Similar depletion is observed in three exemplary novel proteins SEQ ID NO: 2013, SEQ ID NO: 35586 and SEQ ID NO: 36545. As expected, there is no depletion of intergenic (neutral) control guide RNAs. Boxplots summarize effects for numerous guide RNAs targeting each target (n=4); effect across 4 biological replicates for pre-NK and post-NK samples is shown.

### DETAILED DESCRIPTION

A description of example embodiments follows.

### Target Proteins

In one aspect, the disclosure provides a target protein identified herein (e.g., a target protein comprising an amino acid sequence selected from SEQ ID NO: 33885, SEQ ID NO: 37413, SEQ ID NO: 33482, SEQ ID NO: 36545, SEQ ID NO: 2013, SEQ ID NO: 35586, SEQ ID NO: 35050). As used herein, the expressions "target protein identified herein" and "target protein of the disclosure" include the polypeptides disclosed in the Sequence Listing. The target protein can be produced recombinantly (e.g., via DNA or mRNA) or synthetically.

In some embodiments, the target protein is an extracellular protein (e.g., a secreted protein, such as a ligand). In certain embodiments, the target protein is a transmembrane protein (e.g., a receptor). In particular embodiments, the target protein is membrane bound and extracellular, but is not transmembrane. In more particular embodiments, the target protein is embedded in a membrane, but is not transmembrane.

In various embodiments, the target protein is a protein comprising an amino acid sequence set forth in the Sequence Listing. In some embodiments, the target protein consists of an amino acid sequence set forth in the Sequence Listing. In some embodiments, the target protein comprises an amino acid sequence having one amino acid substitution relative to an amino acid sequence set forth in the Sequence Listing, wherein the substitution is substitution of an N-terminal residue in an amino acid sequence in the Sequence Listing with a methionine (Met) residue. In some embodiments, the target protein consists of an amino acid sequence having one amino acid substitution relative to an amino acid sequence set forth in the Sequence Listing, wherein the substitution is substitution of an N-terminal residue in an amino acid sequence in the Sequence Listing with a methionine (Met) residue. In some embodiments, the target protein comprises an amino acid sequence set forth in the Sequence Listing and further comprises a methionine (Met) residue at its N-terminus. In some embodiments, the target protein consists of an amino acid sequence set forth in the Sequence Listing and a methionine (Met) residue at its N-terminus.

Certain of the target proteins in the Sequence Listing have been identified as being differentially expressed (e.g., upregulated or downregulated) in a disease and/or condition state selected from aging, senescence, fibrosis, autoimmunity, cancer (e.g., tumor), an infection, an immunological disease (e.g., inflammation and/or an autoimmune disease), or a combination thereof compared to a reference state (e.g., normal state) such that modulation of the level and/or activity of the target protein acts to treat, ameliorate, and/or prevent the development of the disease or condition.

As used herein, the term "differential expression," refers to at least one recognizable difference in protein expression. It may be a quantitatively measurable, semi-quantitatively estimable or qualitatively detectable difference in protein expression. Thus, a differentially expressed protein, or "DEP," may have a higher expression level in a reference state (e.g., normal state) than in a disease state, in which the DEP has a lower expression level or is not expressed at all. Conversely, a DEP may have a higher expression level in a disease state than in a reference state (e.g., normal state), in which the DEP has a lower expression level or is not expressed at all. Further, expression may be regarded as differential if the DEP is recognizably changed (e.g., mutated) between two states under comparison. Recognizable changes can include amino acid substitutions, insertions, and/or deletions, including N- and C-terminal truncations, as well as modifications (e.g., post-translational modifications).

As used herein, the term "reference" refers to a standard used for comparison purpose(s). A person skilled in the art can select an appropriate reference for a particular comparison purpose(s). Thus, for example, a reference for a disease state may be a normal, healthy state; a reference for a mutated protein may be the non-mutated protein; a reference for a disease treatment may be no treatment or may be a standard of care treatment. In some embodiments, particularly embodiments involving methods of identifying an agent that modulates the expression and/or activity of a target protein, the reference is the activity and/or expression of the target protein in the absence of the agent. In some embodiments, a reference is based on a predetermined level, e.g., based on functional expression or empirical assays. In some embodiments, a reference obtained from one cell, sample or subject (e.g., a cell or sample from a healthy subject, a subject that does not have a particular disease; a healthy subject, a subject who does not have the particular disease). In some embodiments, a reference is obtained from more than one (e.g., a population of) cell, sample or subject (e.g., a cell or sample from a healthy subject, a subject that does not have a particular disease; a healthy subject, a subject who does not have a particular disease), such as 2, 3, 4, 5, 10, 20, 30, 50, 100 or more, or a statistically significant number of cells, samples or healthy subjects. A reference obtained from more than one cell, sample or subject can be represented as a statistic (e.g., an average or median).

In some embodiments, the protein is used as a marker of an immune and/or a disease state, for example, an exhausted T cell, a CD4⁺ T cell (e.g., Th1, Th2, Th17), a CD 8⁺ T cell, or a combination thereof.

In certain embodiments, the target protein has a higher expression level in an activated immune cell. In some embodiments, the target protein has an expression level in an activated immune cell that is at least about 0.5-fold higher, e.g., at least about: 0.6-, 0.7-, 0.8-, 0.9-, 1.0-, 1.1-, 1.2-, 1.3-, 1.4-, 1.5-, 1.6-, 1.7-, 1.8-, 1.9-, 2.0-, 2.5-, 3-, 3.5-, 4-, 5-, 6-, 7-, 8-, 9- or 10-fold higher (e.g., 50-fold higher, 100-fold higher) than the target protein expression level in a reference (e.g., a not activated immune cell of the same type).

In certain embodiments, the target protein has a lower expression level in an activated immune cell. In some embodiments, the target protein has an expression level in an activated immune cell that is at least about 0.5-fold lower, e.g., at least about: 0.6-, 0.7-, 0.8-, 0.9-, 1.0-, 1.1-, 1.2-, 1.3-, 1.4-, 1.5-, 1.6-, 1.7-, 1.8-, 1.9-, 2.0-, 2.5-, 3-, 3.5-, 4-, 5-, 6-, 7-, 8-, 9- or 10-fold lower (e.g., 50-fold lower, 100-fold lower) than the target protein expression level in a reference (e.g., a not activated immune cell of the same type).

In some embodiments, the target protein has an expression level in a disease (e.g., as determined from a sample from a cell or tissue of a subject having the disease) that is at least about 0.5-fold higher, e.g., at least about: 0.6-, 0.7-, 0.8-, 0.9-, 1.0-, 1.1-, 1.2-, 1.3-, 1.4-, 1.5-, 1.6-, 1.7-, 1.8-, 1.9-, 2.0-, 2.5-, 3-, 3.5-, 4-, 5-, 6-, 7-, 8-, 9- or 10-fold higher (e.g., 50 fold higher, 100-fold higher) than the target protein expression level in a reference (e.g., a sample from a cell or tissue of a subject who does not have the disease).

In some embodiments, the target protein has an expression level in a disease (e.g., as determined from a sample comprising or obtained from a cell or tissue of a subject having the disease) that is at least about 0.5-fold lower, e.g. at least about: 0.6-, 0.7-, 0.8-, 0.9-, 1.0-, 1.1-, 1.2-, 1.3-, 1.4-, 1.5-, 1.6-, 1.7-, 1.8-, 1.9-, 2.0-, 2.5-, 3-, 3.5-, 4-, 5-, 6-, 7-, 8-, 9- or 10-fold lower (e.g., 50-fold lower, 100-fold lower) than the target protein expression level in a reference (e.g., a sample from a cell or tissue of a subject who does not have the disease). In some embodiments, the target protein is not expressed, or is expressed at an undetectable level, in a disease (e.g., as determined from a sample comprising or obtained from a cell or tissue of a subject having the disease).

In some embodiments, the target protein has a transcript level in a disease (e.g., as determined from a sample from a cell or tissue of a subject having the disease) that is at least about 0.5-fold higher, e.g., at least about: 0.6-, 0.7-, 0.8-, 0.9-, 1.0-, 1.1-, 1.2-, 1.3-, 1.4-, 1.5-, 1.6-, 1.7-, 1.8-, 1.9-, 2.0-, 2.5-, 3-, 3.5-, 4-, 5-, 6-, 7-, 8-, 9- or 10-fold higher (e.g., 50 fold higher, 100-fold higher) than the target protein transcript level in a reference (e.g., a sample from a cell or tissue of a subject who does not have the disease). In particular embodiments, an increase in the transcript level of a target protein contributes to (e.g., results in) a disease or condition described herein.

In some embodiments, the target protein has a transcript level in a disease (e.g., as determined from a sample comprising or obtained from a cell or tissue of a subject having the disease) that is at least about 0.5-fold lower, e.g. at least about: 0.6-, 0.7-, 0.8-, 0.9-, 1.0-, 1.1-, 1.2-, 1.3-, 1.4-, 1.5-, 1.6-, 1.7-, 1.8-, 1.9-, 2.0-, 2.5-, 3-, 3.5-, 4-, 5-, 6-, 7-, 8-, 9- or 10-fold lower (e.g., 50-fold lower, 100-fold lower) than the target protein transcript level in a reference (e.g., a sample from a cell or tissue of a subject who does not have the disease). In some embodiments, the transcript of the target protein is not expressed, or is expressed at an undetectable level, in a disease (e.g., as determined from a sample comprising or obtained from a cell or tissue of a subject having the disease). In particular embodiments, a decrease in the transcript level of a target protein contributes to (e.g., results in) a disease or condition described herein.

In particular embodiments, the gene encoding the target protein comprises at least one mutation (e.g., a fusion, a deletion, an insertion, a point mutation, and/or an expansion of amino-acid repeats) in a disease described herein.

Non-limiting examples of (biological) samples include blood, blood components (e.g., serum or plasma), urine, saliva, amniotic fluid, cerebrospinal fluid, tissue (e.g., a biopsy or microbiopsy), pancreatic fluid, chorionic villus sample, and cells, etc., isolated from a subject.

In some embodiments, the target protein is translated from a non-coding RNA. In some embodiments, the non-coding RNA is a long intergenic non-coding RNA (lincRNA). In certain embodiments, the non-coding RNA is a long noncoding RNA (lncRNA). In some embodiments, the non-coding RNA is a microRNA (miRNA or miR).

In some embodiments, the target protein is translated from a non-exonic element in an unprocessed precursor mRNA (pre-mRNA). In some embodiments, the non-exonic element is an intron in a pre-mRNA. In some embodiments, the non-exonic element is a 5'-untranslated region (5'-UTR) in a pre-mRNA. In some embodiments, the non-exonic element is a 3'-untranslated region (3'-UTR) in a pre-mRNA.

In some embodiments, the target protein has a length of 2,000 amino acids or less, e.g., 1000 amino acids or less, 750 amino acids or less, 500 amino acids or less, 250 amino acids or less, 150 amino acids or less or 100 amino acids or less. In some embodiments, the target protein has a length of 7 amino acids or more, e.g., 8, 9, 10, 15, 18, 25, 50, 75 or 100 amino acids or more. In certain embodiments, the target protein has a length of from about 50 to about 200 amino acids, e.g., from about 100 to about 150 amino acids. In particular embodiments, the target protein has a length of 7 amino acids or more. In more particular embodiments, the target protein has a length of about 18 amino acids.

In some embodiments, a target protein of the disclosure is a modulator of one or more GPCRs. In some embodiments, a target protein is an agonist of one or more GPCRs. In some embodiments, a target protein is an antagonist of one or more GPCRs. In some embodiments, a target protein is a direct modulator of one or more GPCRs, such as a ligand for one or more GPCRs. In some embodiments, a target protein is an indirect modulator of one or more GPCRs.

The expression and/or activity of various GPCRs have been linked to different diseases/disorders, conditions and indications, including those set forth in Table A (see, e.g., Kenakin, T., Biased Receptor Signaling in Drug Discovery, Pharmacol Rev 71:267-315, April 2019; Harmar, A.J., et al., IUPHAR-DB: the IUPHAR database of G protein-coupled receptors and ion channels, Nucleic Acids Research, 2009, Vol. 37; and Davenport AP, Scully CCG, de Graaf C, Brown AJH, and Maguire JJ. Advances in therapeutic peptides targeting G protein-coupled receptors. Nat Rev Drug Discov. 2020 Jun.19(6):389-413; the contents of each are incorporated herein by reference in their entirety). Accordingly, in some embodiments, a target protein disclosed herein, which is a modulator of a GPCR, is useful for treating and/or diagnosing one or more diseases/disorders, conditions and/or indications, such as cancer or a pre-cancerous condition, or any of the diseases/disorders, conditions and indications listed in Table A, which are known to be associated with GPCR expression and/or activity.

**Table A. GPCRs Associated with Disease**

| **Disease group** | **GPCRs Associated with Disease Group** |
|---|---|
| acute disease | ADRB1, P2RY12, AGTR1 |
| breast disease | GNRHR |
| cancer or benign tumor | ADRB2, ADRA1A, TSHR, SSTR3, SSTR1, GNRHR, CXCR4, CASR, GCGR, ADRA1D, SSTR4, ADRA1B, OPRM1, SMO, TACR1, OPRK1, SSTR2, ADRB1, MC1R, SSTR5 |
| cardiovascular disease | ADRB3, OPRD1, ADRB2, ADRA1A, AGTR1, ADORA1, TSHR, HTR1D, PTGIR, HTR4, DRD2, F2R, HTR1B, FZD4, EDNRB, ADRA1D, ADORA2A, HTR1F, ADORA3, ADORA2B, CELSR1, CALCRL, ADRA1B, BDKRB2, OPRM1, ADRA2B, GLP1R, AVPR1A, P2RY12, OPRK1, PTGFR, ADRB1, ADRA2A, AVPR2, ADRA2C, EDNRA |
| chronic disease | ADORA3, ADORA2B, ADGRG6, ADRB2, CASR, GABBR1, AGTR1, GABBR2, ADORA1, CHRM3, CHRM1, ADORA2A |
| connective tissue disease | ADRB3, OPRD1, MC2R, OPRK1, CASR, PTGER3, OPRM1, ADRA2A, ADRA2B, ADRA2C, GNRHR |
| digestive system disease | CALCR, AGTR1, MTNR1B, GIPR, CHRM3, SSTR3, SSTR1, HTR4, PTGER3, GCGR, CHRM1, EDNRB, HRH2, DRD3, OPRM1, GLP1R, P2RY12, S1PR1, SSTR2, HTR1A, DRD4, MC1R, EDNRA, GLP2R, SSTR5 |
| disorder of development or morphogenesis | CALCR, S1PR4, AGTR1, GABBR2, CHRM3, GNRHR, KISS1R, S1PR3, FZD2, PTH1R, HRH1, S1PR2, EDNRB, LHCGR, ADGRG2, ADGRG6, CELSR1, GABBR1, SMO, S1PR1, MC2R, S1PR5, MC1R, EDNRA |
| disorder of visual system | ADRB3, ADRB2, GPR179, GPR143, P2RY2, OPN1LW, CHRM3, P2RY4, OPN1SW, GRM6, CASR, HRH1, CHRM1, FZD4, EDNRB, RHO, ADRA2B, ADGRV1, FZD5, CHRM2, P2RY1, PTGFR, ADRB1, ADRA2A, ADRA2C, P2RY6, OPN1MW |
| endocrine system disease | CALCR, AGTR1, MTNR1B, MC4R, GIPR, TSHR, SSTR3, SSTR1, TACR3, GNRHR, FSHR, KISS1R, PTH1R, CASR, GCGR, GHRHR, LHCGR, DRD3, SSTR4, GHSR, GLP1R, TACR1, P2RY12, MC2R, SSTR2, PROKR2, DRD4, MC1R, EDNRA, CHRM1, SSTR5 |
| genetic disorder | CNR1, GABBR2, MC4R, SSTR3, SSTR1, KISS1R, PTH1R, GHRHR, ADRA2B, GRM1, AVPR1A, P2RY12, HTR1A, PROKR2, OPN1MW, TBXA2R, ADRA1A, AGTR1, MTNR1B, OPN1LW, CHRM3, GRM7, HTR1D, GPR68, S1PR2, RHO, LHCGR, ADORA2A, ADORA3, ADORA2B, SSTR4, BDKRB2, LPAR6, ADGRV1, S1PR1, SSTR2, CHRM2, PTGFR, HRH3, CALCR, HTR2A, GPR143, MTNR1A, TACR3, HTR4, GNRHR, FSHR, GRM6, FZD2, CASR, EDNRB, ADRA1D, ADGRG2, ADGRG6, GABBR1, ADRA1B, OPRM1, SMO, FZD6, MC2R, ADRA2A, AVPR2, ADRA2C, CHRM1, SSTR5, ADRB3, GPR179, ADORA1, TSHR, OPN1SW, DRD2, CXCR4, FZD4, AVPR1B, HRH2, DRD3, CELSR1, CCR5, HTR2C, DRD4, MC1R, EDNRA |
| head and neck disorder | ADRB3, ADRB2, GPR179, ADRA1A, GPR143, P2RY2, OPN1LW, CHRM3, P2RY4, OPN1SW, GRM6, CASR, HRH1, FZD4, EDNRB, ADRA1D, RHO, OPN1MW, ADRA1B, ADRA2B, ADGRV1, FZD5, CHRM2, P2RY1, PTGFR, ADRB1, ADRA2A, ADRA2C, P2RY6, CHRM1 |
| hematologic disease | P2RY12, MC2R, CXCR4, TBXA2R, OPRM1, DRD4, AVPR2, AVPR1B, AVPR1A, SMO, DRD3 |
| idiopathic disease | HTR2A, CASR, HRH1, CNR1, GABBR2, EDNRB, EDNRA, HTR2C |
| immune system disease | ADRB3, CALCR, ADRA1A, S1PR4, GABBR2, TSHR, CHRM3, S1PR3, CXCR4, HRH1, S1PR2, GCGR, ADRA1D, CYSLTR1, CELSR1, GABBR1, ADRA1B, ADRA2B, GLP1R, CCR5, S1PR1, MC2R, CHRM2, S1PR5, PTGFR, ADRA2A, ADRA2C, CHRM1 |
| inflammatory disease | ADRB3, OPRD1, ADRB2, ADRA1A, AGTR1, P2RY2, TSHR, CHRM3, P2RY4, HRH1, PTGER3, ADRA1D, CYSLTR1, ADRA1B, BDKRB2, OPRM1, ADRA2B, S1PR1, MC2R, OPRK1, CHRM2, P2RY1, ADGRE2, PTGFR, ADRB1, ADRA2A, ADRA2C, P2RY6, CHRM1 |
| injury | PTH1R, HRH1, GABBR1, PTGER3, GABBR2, AVPR2, AVPR1B, AVPR1A, HRH2, CHRM1 |
| integumentary system disease | ADRB3, HRH1, ADRA1A, GNRHR, ADRA1B, BDKRB2, ADGRE2, LPAR6, EDNRA, ADRA2A, ADRA2B, EDNRB, MC1R, PTGFR, ADRA1D, ADRA2C, SMO, FZD6 |
| metabolic disease | ADRB3, OPRD1, CALCR, HTR2A, OXTR, CNR1, GPR143, AGTR1, GABBR2, MC4R, MTNR1A, GIPR, CHRM3, TSHR, MTNR1B, SSTR3, SSTR1, DRD2, CASR, GCGR, DRD3, ADORA2A, SSTR4, OPRM1, GRM1, GLP1R, HTR2C, P2RY12, OPRK1, SSTR2, CHRM2, DRD4, ADRA2A, MC1R, EDNRA, CHRM1, SSTR5 |
| musculoskeletal system disease | ADRB3, OPRD1, ADRB2, CALCR, HTR2A, ADRA1A, AGTR1, GABBR2, FZD2, PTH1R, CASR, PTGER3, EDNRB, ADRA1D, GABBR1, ADRA1B, OPRM1, ADRA2B, SMO, HTR2C, MC2R, OPRK1, ADRB1, ADRA2A, ADRA2C, EDNRA |
| nervous system disease | OPRD1,HCRTR1,DRD1,GRM3,CNR1,GABBR2,SSTR3,ADGRG1,S STR1,GHRHR,HTR1F,HTR7,ADRA2B,GRM1,HCRTR2,P2RY12,HT R1A,S1PR5,OPN1MW,ADRB2,HTR2B,ADRA1A,AGTR1,MTNR1B, OPN1LW,HTR6,CHRM3,GRM7,HTR1D,S1PR3,S1PR2,RHO,ADOR A2A,ADORA3,ADORA2B,SSTR4,DRD5,CALCRL,ADGRV1,HTR1 E,S1PR1,SSTR2,CHRM2,HTR5A,HRH3,HTR2A,OXTR,GPR143,MT NR1A,HTR4,GRM6,CASR,HRH1,HTR1B,ADRA1D,GABBR1,ADR A1B,OPRM1,SMO,MC2R,OPRK1,ADRA2A,ADRA2C,CHRM1,SST R5,GPR179,S1PR4,ADORA1,OPN1SW,DRD2,FZD4,DRD3,CYSLT R1,CELSR1,HTR2C,DRD4,ADRB1 |
| obstetric disorder | ADRB3, OXTR, ADRA1A, PTGER3, ADRA1B, TSHR, ADRA2A, ADRA2B, GLP1R, ADRA1D, ADRA2C |
| otorhinolaryngol ogic disease | ADRB3, ADRB2, CYSLTR1, HRH1, ADRA1A, CHRM2, ADRA1B, CHRM3, ADRA2A, ADRA2B, ADRB1, ADRA1D, ADRA2C, EDNRA, CHRM1 |
| psychiatric disorder | OPRD1, HCRTR1, HTR2A, DRD1, OXTR, HTR2B, ADRA1A, HRH3, GRM3, GABBR2, MTNR1A, HTR6, MTNR1B, HTR1D, HTR4, DRD2, HRH1, HTR1B, AVPR1B, DRD3, HTR1F, CYSLTR1, DRD5, GABBR1, OPRM1, HTR7, ADRA2B, GRM1, HCRTR2, AVPR1A, HTR2C, HTR1E, OPRK1, HTR1A, DRD4, ADRA2A, AVPR2, HTR5A, ADRA2C, CHRM1 |
| reproductive system disease | ADRA1A,SSTR3,SSTR1,TACR3,GNRHR,FSHR,KISS1R,ADRA1D, LHCGR,ADGRG2,DRD3,SSTR4,PTGER2,ADRA1B,PTGER1,CCR5, TACR1, SSTR2,PROKR2,DRD4,AVPR2, SSTR5 |
| respiratory system disease | ADRB3,ADRB2,ADRA1A,AGTR1,ADORA1,CHRM3,PTGIR,CASR ,HRH1,EDNRB,ADRA1D,ADORA2A,ADORA3,ADORA2B,ADGR G6,CYSLTR1,ADRA1B,ADRA2B,CHRM2,ADRB1,ADRA2A,ADR A2C,EDNRA,CHRM1 |
| syndromic disease | HTR2A,P2RY2,GABBR2,CHRM3,SSTR3,P2RY4,SSTR1,HTR4,GN RHR,FSHR,KISS1R,DRD2,FZD2,CASR,EDNRB,LHCGR,DRD3,CY SLTR1,SSTR4,GABBR1,OPRM1,ADGRV1,HTR2C,P2RY12,MC2R, SSTR2,HTR1A,PROKR2,P2RY1,DRD4,P2RY6,CHRM1,SSTR5 |
| upper digestive tract disorder | HTR4, HRH2 |
| urinary system disease | ADRB3, ADRA1A, AGTR1, TSHR, CHRM3, CASR, EDNRB, AVPR1B, ADRA1D, ADORA2A, ADORA2B, ADRA1B, OPRM1, ADRA2B, AVPR1A, MC2R, CHRM2, ADRA2A, AVPR2, ADRA2C, EDNRA |

### Agents that Modulate Target Proteins

Provided herein are agents that modulate the expression of a target protein disclosed herein, such as a target protein in the Sequence Listing, a variant thereof, or a fragment thereof (e.g., a biologically active fragment of a target protein). The expression of the target protein or variant or fragment thereof can be modulated by a wide range of processes, directly or indirectly, leading to an increase or a decrease of the target protein level. Non-limiting examples include altering: the copy number of the gene encoding the target protein, transcriptional initiation, elongation or termination, RNA processing, RNA stability (e.g., mRNA stability), RNA degradation, translation initiation, post-translational modification of a protein, protein stability, protein degradation (e.g., cleavage, such as protease cleavage), or a combination of the foregoing.

In some embodiments, the agent modulates (e.g., increases or decreases) the expression of a gene or gene transcript encoding the target protein. In some embodiments, the agent modulates the expression or activity of the target protein. In some embodiments, the agent decreases (e.g., inhibits, reduces or neutralizes) the activity of the target protein. In some embodiments, the agent increases (e.g., activates) the activity of the target protein. In some embodiments, the agent decreases (e.g., inhibits or downregulates) the expression of the target protein. In other embodiments, the agent increases (e.g., activates or upregulates) the expression of the target protein.

As used herein, the term "increasing" or "increase" refers to modulation that results in a higher level of expression, activity, function or a combination thereof of the target protein, or a metric (e.g., cancer cell death or DNA methylation of a target site), relative to a reference (e.g., the level prior to or in an absence of modulation by the agent). In some embodiments, the agent increases the expression or activity of the target protein, or the metric, by at least about 5% relative to the reference, e.g., by at least about: 10%, 15%, 20%, 25%, 30%, 35%, 40%, 45%, 50%, 55%, 60%, 65%, 70%, 75%, 80%, 85%, 90%, 95% or 98% relative to the reference.

As used herein, the term "decreasing" or "decrease" refers to modulation that results in a lower level of expression, activity, function or a combination thereof of the target protein, or a metric (e.g., cancer cell death or DNA methylation of a target site), relative to a reference (e.g., the level prior to or in an absence of modulation by the agent). In some embodiments, the agent decreases the expression or activity of the target protein, or the metric, by at least about 5% relative to the reference, e.g., by at least about: 10%, 15%, 20%, 25%, 30%, 35%, 40%, 45%, 50%, 55%, 60%, 65%, 70%, 75%, 80%, 85%, 90%, 95% or 98% relative to the reference.

Non-limiting examples of the metric include energy production or energy conversion in the liver (e.g., modulating ATP synthesis, B-oxidation, oxidation of metabolites derived from glycolysis, oxidation of metabolites derived from amino acids), mitochondrial transcription, mitochondrial ribosome assembly, mitochondrial translation, mitochondrial thermogenesis, hormonal signaling (e.g., mitochondrial estrogen receptor (mtER) signaling), redox maintenance (e.g., NADH and/or FADH₂), cell cycle regulation, cell migration, cell morphology, apoptosis, necrosis, membrane potential, ion (e.g., calcium or zinc) storage, ion (e.g., calcium or zinc) homeostasis, metabolite synthesis (e.g., heme biosynthesis or steroid biosynthesis), nutrient sensing, unfolded protein stress response pathway, signaling processes (e.g., calcium signaling).

In some embodiments, the level of expression, activity, function or a combination thereof of the target protein, or the metric, is measured after the agent is contacted with (e.g., a cell) or administered (e.g., to a subject) for at least about 1 day, e.g., at least about: 2 days, 3 days, 4 days, 5 days, 6 days, 8 days, 9 days, 10 days, 1 week, 2 weeks, 3 weeks, 1 month, 2 months, 3 months, 4 months, 5 months or 6 months, e.g., after a treatment regimen has begun.

In some embodiments, the agent comprises, consists essentially of or consists of a polypeptide, a polynucleotide, a gene editing system, a small molecule, or a cell (e.g., a cell therapy).

In some embodiments, the target protein activates an immune cell, and the agent modulates (e.g., increases or decreases) the level of expression, activity, function or a combination thereof, of the target protein. In some embodiments, the target protein inhibits an immune cell (e.g., inhibits activation of an immune cell, induces immune cell death (e.g., apoptosis), or a combination thereof), and the agent modulates (e.g., increases or decreases) the level of expression, activity, function or a combination thereof of the target protein.

In certain embodiments, the agent modulates (e.g., increases or decreases) the level of expression, activity, function, or a combination thereof, of the target protein in a cancer cell (e.g., a metastatic cancer cell), a cell (e.g., stromal cell) in a tumor micro-environment, a target cell of an inflammatory response (e.g., an epithelial cell, an endothelial cell, a stem cell or a non-immune cell)), an immune cell (e.g., an effector T cell, a helper T cell, a Th1 cell, a Th2 cell, a Th17 cell, a B cell, an natural killer (NK) cell, an innate lymphoid cell (e.g., an ILC1 cell, an ILC2 cell, an ILC3 cell), a macrophage (e.g., an M1 macrophage, an M2 macrophage), a monocyte, and/or an antigen presenting cell (e.g., a dendritic cell), or a combination of the foregoing. In certain embodiments, the agent modulates the level of expression, activity, function or a combination thereof of the target protein in a tumor, a tumor microenvironment, a site of metastasis, a lymph node, a spleen, a secondary lymphoid organ, a tertiary lymphoid organ, a barrier tissue, skin, gut, an airway, a wound, another immune tissue, a non-immune tissue, or a combination of the foregoing.

In some embodiments, the agent modulates (e.g., increases or decreases) inflammation, decreases the level of an auto-antibody, increases an organ function, decreases the rate or number of relapses or flare-ups, decreases a viral load, controls infection, or a combination of the foregoing.

In some embodiments, the agent induces downregulation of the target protein (e.g., increases target-protein degradation); prevents multimerization (e.g., dimerization) of the target protein; sequesters a target protein (e.g., a secreted target protein); modulates (e.g., agonizes, antagonizes or disrupts) a known function of the target protein; decreases binding between the target protein and a binding partner (e.g., via steric hinderance); modulates (e.g., increases or decreases) downstream cell signaling; induces phagocytosis of a cell expressing the target protein; or a combination of the foregoing. In certain embodiments, the agent lacks agonistic activity toward the target protein. In certain embodiments, the agent has agonistic activity toward the target protein. In some embodiments, the agent lacks antagonistic activity toward the target protein. In some embodiments, the agent has antagonistic activity toward the target protein. In particular embodiments, the agent binds to at least one residue of the target protein that is involved in binding to a binding partner. In some embodiments, the agent binds to one or more binding sites and/or domains of the target protein involved in binding of the target protein to the binding partner.

Non-limiting examples of binding partners include cytokine receptors and immune signaling receptors.

In some embodiments, the agent induces downregulation of a binding partner of the target protein; sequesters a binding partner (e.g., a secreted binding partner) of the target protein; prevents multimerization (e.g., dimerization) of a binding partner of the target protein; sequesters a binding partner of the target protein (e.g., a secreted binding partner); modulates (e.g., agonizes, antagonizes or disrupts) a known function of a binding partner of the target protein; decreases binding between the target protein and a binding partner (e.g., via steric hinderance); modulates (e.g., increases or decreases) downstream cell signaling; or a combination of the foregoing. In certain embodiments, the agent lacks agonistic activity toward a binding partner of the target protein. In certain embodiments, the agent has agonistic activity toward a binding partner of the target protein. In some embodiments, the agent lacks antagonistic activity toward a binding partner of the target protein. In some embodiments, the agent has antagonistic activity toward a binding partner of the target protein. In particular embodiments, the agent further binds to at least one residue of a binding partner of the target protein that is involved in binding between the target protein and the binding partner. In more particular embodiments, the agent further binds to one or more binding sites and/or domains of a binding partner of the target protein involved in binding between the target protein and the binding partner.

In some embodiments, the agent modulates (e.g., activates or inhibits) immune signaling, cytokine signaling, inflammatory signaling, or a combination of the foregoing.

In some embodiments, the agent enhances a signal involved in T cell activation and/or survival. In certain embodiments, the agent activates a stimulatory checkpoint molecule. Non-limiting examples of stimulatory checkpoint molecules include CD27, CD28, CD40, CD122, CD137, OX40, GITR, inducible T-cell costimulator (ICOS). In particular embodiments, the agent is an agonist to CD28.

In some embodiments, the agent reduces a signal involved in T cell anergy and/or exhaustion. In certain embodiments, the agent inhibits an inhibitory checkpoint molecule. Non-limiting examples of inhibitory checkpoint molecules include PD-1, PD-L1, PD-L2, TIM-3, LAG-3, CTLA-4, A2AR, CD276, B7-H4, BTLA, IDO, KIR, NOX2, VISTA, SIGLECs 7 and SIGLECs 9. In particular embodiments, the agent is an inhibitor (e.g., a blocking antibody) to PD-1.

In some embodiments, the agent further modulates (e.g., increases or decreases) the level of expression, activity, function, or a combination thereof, of a checkpoint protein other than the target protein.

In certain embodiments, the agent modulates (e.g., increases or decreases) the level of expression, activity, function, or a combination thereof, of a variant of a target protein disclosed herein. In some embodiments, the variant comprises an amino acid sequence that is at least 70% identical to the amino acid sequence of a target protein disclosed herein. For example, the sequence identity to the variant can be at least about: 75%, 80%, 85%, 90%, 91%, 92%, 93%, 94%, 95%, 96%, 97%, 98% or 99%. In some embodiments, the sequence identity is about: 70%, 75%, 80%, 85%, 90%, 91%, 92%, 93%, 94%, 95%, 96%, 97%, 98% or 99%. In some embodiments, the sequence identity is about: 70-99%, 75-99%, 75-95%, 80-99%, 80-98%, 80-95%, 80-90%, 85-98%, 85-97%, 85-90%, 90-97%, 90-96%, 90-85%, 90-80% or 95-99%. In some embodiments, a variant comprises an amino acid sequence that is at least about 75%, at least about 80%, at least about 85%, at least about 90%, at least about 95%, or at least about 98% identical to the amino acid sequence of a target protein disclosed herein.

As used herein, the term "sequence identity," refers to the extent to which two nucleotide sequences, or two amino acid sequences, have the same residues at the same positions when the sequences are aligned to achieve a maximal level of identity, expressed as a percentage. For sequence alignment and comparison, typically one sequence is designated as a reference sequence, to which a test sequences are compared. The sequence identity between reference and test sequences is expressed as the percentage of positions across the entire length of the reference sequence where the reference and test sequences share the same nucleotide or amino acid upon alignment of the reference and test sequences to achieve a maximal level of identity. As an example, two sequences are considered to have 70% sequence identity when, upon alignment to achieve a maximal level of identity, the test sequence has the same nucleotide or amino acid residue at 70% of the same positions over the entire length of the reference sequence.

Alignment of sequences for comparison to achieve maximal levels of identity can be readily performed by a person of ordinary skill in the art using an appropriate alignment method or algorithm. In some instances, the alignment can include introduced gaps to provide for the maximal level of identity. Examples include the local homology algorithm of Smith & Waterman, Adv. Appl. Math. 2:482 (1981), the homology alignment algorithm of Needleman & Wunsch, J. Mol. Biol. 48:443 (1970), the search for similarity method of Pearson & Lipman, Proc. Nat'l. Acad. Sci. USA 85:2444 (1988), computerized implementations of these algorithms (GAP, BESTFIT, FASTA, and TFASTA in the Wisconsin Genetics Software Package, Genetics Computer Group, 575 Science Dr., Madison, Wis.), and visual inspection (see generally Ausubel et al., Current Protocols in Molecular Biology).

When using a sequence comparison algorithm, test and reference sequences are input into a computer, subsequent coordinates are designated, if necessary, and sequence algorithm program parameters are designated. The sequence comparison algorithm then calculates the percent sequence identity for the test sequence(s) relative to the reference sequence, based on the designated program parameters. A commonly used tool for determining percent sequence identity is Protein Basic Local Alignment Search Tool (BLASTP) available through National Center for Biotechnology Information, National Library of Medicine, of the United States National Institutes of Health. (Altschul et al., 1990).

In some embodiments, the amino acid sequence of a variant of a target polypeptide disclosed herein comprises at least one amino acid substitution relative to the amino acid sequence of the target protein. In some embodiments, the number of amino acid substitutions in a variant relative to the amino acid sequence of a target protein disclosed herein is at least about: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60. In some embodiments, the number of amino acid substitutions is at least about: 5, 6, 10, 15, 20, 25, 30, 35, 40, 45, 50, 55 or 60. In some embodiments, the number of amino acid substitutions is up to about: 60, 55, 50, 45, 40, 35, 30, 25, 20, 15, 10, 6 or 5. In some embodiments, the number of amino acid substitutions is about: 1-60, 1-55, 2-55, 2-50, 3-50, 3-45, 4-45, 4-40, 5-40, 5-35, 6-35, 6-30, 7-30, 7-25, 8-25, 8-20, 9-20, 9-15, 10-15, 5-60, 10-60, 10-55, 15-55, 15-50, 20-50, 20-45, 25-45, 25-40 or 30-40. In some embodiments, the number of amino acid substitutions is about: 10-35, 10-33, 11-33, 11-31, 12-31, 12-29, 13-29, 13-27, 14-27 or 14-25.

The amino acid substitution(s) in a variant can be substitutions with a canonical amino acid or a non-canonical amino acid. Non-canonical amino acids include, but are not limited to D amino acids, such as D versions of the canonical L-amino acids.

In some embodiments, an amino acid substitution is a conservative substitution. The term "conservative amino acid substitution(s)" or "conservative substitution(s)" refers to an amino acid substitution having a value of 0 or greater in BLOSUM62.

In some embodiments, an amino acid substitution is a highly conservative substitution. The term "highly conservative amino acid substitution(s)" or "highly conservative substitution(s)" refers to an amino acid substitution having a value of at least 1 *(e.g.,* at least 2) in BLOSUM62.

In some embodiments, a variant of a target protein of the disclosure comprises about 5-60 amino acid substitutions, relative to the amino acid sequence of a target protein disclosed herein. In some embodiments, the amino acid substitutions include at least one conservative substitution. In some embodiments, the amino acid substitutions include at least one highly conservative substitution.

### A. Polypeptide Agents

The term "polypeptide" "peptide" or "protein" denotes a polymer of at least two amino acids covalently linked by an amide bond, regardless of length or post-translational modification (e.g., glycosylation or phosphorylation). A protein, peptide or polypeptide can comprise any suitable L-and/or D-amino acid, for example, common α-amino acids (*e.g.,* alanine, glycine, valine), non-α-amino acids (e.g., β-alanine, 4-aminobutyric acid, 6-aminocaproic acid, sarcosine, statine), and unusual amino acids (e.g., citrulline, homocitruline, homoserine, norleucine, norvaline, ornithine). The amino, carboxyl and/or other functional groups on a peptide can be free (e.g., unmodified) or protected with a suitable protecting group. Suitable protecting groups for amino and carboxyl groups, and methods for adding or removing protecting groups are known in the art and are disclosed in, for example, Green and Wuts, "Protecting Groups in Organic Synthesis, " John Wiley and Sons, 1991. The functional groups of a protein, peptide or polypeptide can also be derivatized *(e.g.,* alkylated) or labeled *(e.g.,* with a detectable label, such as a fluorogen or a hapten) using methods known in the art. A protein, peptide or polypeptide can comprise one or more modifications (e.g., amino acid linkers, acylation, acetylation, amidation, methylation, terminal modifiers *(e.g.,* cyclizing modifications), *N*-methyl-α-amino group substitution), if desired. In addition, a protein, peptide or polypeptide can be an analog of a known and/or naturally-occurring peptide, for example, a peptide analog having conservative amino acid residue substitution(s).

In some embodiments, the agent comprises a polypeptide. In some embodiments, the polypeptide is an isolated polypeptide (e.g., isolated or extracted from a biological sample or source). In some embodiments, the polypeptide is a recombinant polypeptide. In some embodiments, the polypeptide is an inhibitor (e.g., a direct inhibitor or an indirect inhibitor) of the expression and/or activity of a target protein disclosed herein. In some embodiments, the polypeptide is an activator (e.g., a direct activator or an indirect activator) of the expression and/or activity of a target protein disclosed herein. In some embodiments, the polypeptide decreases the expression or activity of the target protein disclosed herein. In other embodiments, the polypeptide increases the expression or activity of the target protein disclosed herein. In some embodiments, the polypeptide is a target protein disclosed herein, or a portion thereof (e.g., a biologically active portion thereof, such as a biologically active fragment of the target protein).

In some embodiments, the polypeptide is an immunoglobulin molecule, such as an antibody (e.g., a whole antibody, an intact antibody) or an antigen-binding fragment of an antibody. In some embodiments, the antibody or antigen-binding fragment thereof binds to the target protein. In some embodiments, the antibody or antigen-binding fragment thereof binds to a protein capable of modulating the expression or activity of the target protein.

In some embodiments, the polypeptide is an antibody. As used herein, the term "antibody" refers to an immunoglobulin molecule capable of specific binding to a target, such as a carbohydrate, polynucleotide, lipid, polypeptide, etc., through at least one antigen recognition site, located in the variable region of the immunoglobulin molecule. As used herein, the term "antibody" refers to a full-length antibody comprising two heavy (H) chains and two light (L) chains inter-connected by disulfide bonds or multimers thereof (for example, IgM). Each heavy chain comprises a heavy chain variable region (V_{H}) and a heavy chain constant region (comprising domains CH1, hinge CH2 and CH3). Each light chain comprises a light chain variable region (V_{L}) and a light chain constant region (CL). The V_{H} and the V_{L} regions may be further subdivided into regions of hypervariability, termed complementarity determining regions (CDR), interspersed within framework regions (FR). V_{H} and V_{L} each comprises three CDRs and four FR segments, arranged from the amino-terminus to the carboxy-terminus in the following order: FR1, CDR1, FR2, CDR2, FR3, CDR3, and FR4. The antibody can be of any species, such as a rodent (e.g., murine, rat, guinea pig) antibody, a human antibody, or the antibody can be a humanized antibody or chimeric antibody.

In some embodiments, the antibody comprises an IgA (e.g., IgA1 or IgA2) heavy chain constant region, an IgD heavy chain constant region, an IgE heavy chain constant region, an IgG (e.g., IgG1, IgG2 (e.g., IgG2a, IgG2b or IgG2c), IgG3 or IgG4) heavy chain constant region or an IgM heavy chain constant region. In some embodiments, the antibody comprises an IgG heavy chain constant region. In some embodiments, the antibody comprises a κ light chain constant region. In some embodiments, the antibody comprises a λ light chain constant region.

In some embodiments, the antibody is a polyclonal antibody. In some embodiments, the antibody is a monoclonal antibody. In some embodiments, the antibody is human or chimeric. In some embodiments, the antibody is primatized (e.g., humanized). In some embodiments, the antibody is multispecific, e.g., bi-, tri-, or quad-specific. In some embodiments, the antibody is a heteroconjugate antibody.

In some embodiments, the polypeptide agent is an antigen-binding fragment of an immunoglobulin molecule (e.g., antibody). The term "antigen-binding fragment" refers to a portion of an immunoglobulin molecule (e.g., antibody) that retains the antigen binding properties of the parental full-length antibody. Non-limiting examples of antigen-binding fragments include a V_{H} region, a V_{L} region, an Fab fragment, an F(ab')₂ fragment, an Fd fragment, an Fv fragment, and a domain antibody (dAb) consisting of one V_{H} domain or one V_{L} domain, etc. V_{H} and V_{L} domains may be linked together via a synthetic linker to form various types of single-chain antibody designs in which the V_{H}/V_{L} domains pair intramolecularly, or intermolecularly in those cases when the V_{H} and V_{L} domains are expressed by separate chains, to form a monovalent antigen binding site, such as single chain Fv (scFv) or diabody. In some embodiments, the polypeptide disclosed herein is an antigen binding fragment selected from Fab, Fab', F(ab')₂, Fd, Fv, disulfide-linked Fvs (sdFv, e.g., diabody, triabody or tetrabody), scFv, SMIP or rlgG. In some embodiments, the polypeptide is a scFv. Antigen-binding fragments can be produced by recombinant DNA techniques, enzymatic or chemical cleavage of intact immunoglobulins, or, in certain cases, by chemical peptide synthesis procedures known in the art.

Polypeptide agents (e.g., monoclonal antibodies) can be monovalent, bivalent or multivalent. A monoclonal antibody can be monospecific or multispecific (e.g., bispecific). Monospecific antibodies bind one antigenic epitope. A multispecific antibody, such as a bispecific antibody or a trispecific antibody, is included in the term monoclonal antibody.

"Multispecific" refers to an antibody that specifically binds at least two distinct antigens or at least two distinct epitopes within the antigens, for example three, four or five distinct antigens or epitopes. "Bispecific" refers to an antibody that specifically binds two distinct antigens or two distinct epitopes within the same antigen.

"Isolated antibody" refers to an antibody or an antigen-binding fragment thereof that is substantially free of other antibodies having different antigenic specificities (e.g., an isolated anti-target protein antibody is substantially free of antibodies that specifically bind antigens other than the target protein). In the case of a bispecific antibody, the bispecific antibody specifically binds two antigens of interest, and is substantially free of antibodies that specifically bind antigens other than the two antigens of interest. In some embodiments, the polypeptide agent (e.g., monoclonal antibody) is at least 80% pure, *e.g.,* about: 80%, 81%, 82%, 83%, 84%, 85%, 86%, 87%, 88%, 89%, 90%, 91%, 92%, 93%, 94%, 95%, 96%, 97%, 98%, 99% or 100% pure.

In some embodiments, the polypeptide is an antagonist antibody that binds to the target protein (e.g., a target protein whose expression or activity is increased in a cancer state relative to a reference state). In some embodiments, the antibody described herein is an antagonist antibody that binds to a protein capable of modulating the expression or activity of the target protein. As used herein, the term "antagonist antibody" refers to an antibody that, upon binding to an antigen (e.g., the target protein or a protein capable of modulating the expression or activity of the target protein), reduces (e.g., inhibits) the function of the antigen. In some embodiments, the antigen is a receptor, and the antagonist antibody binds to the ligand-binding domain of the receptor. In some embodiments, the antigen is a transmembrane protein, and the antagonist antibody binds to the extracellular region of the transmembrane protein. In some embodiments, the antigen is an enzyme or a signaling molecule, and the antagonist antibody reduces the activity of the enzyme or attenuate a signal transduction pathway mediated by the signaling molecule. In some embodiments, the antagonist antibody reduces antigen function by at least about 10%, e.g., by at least about: 15%, 20%, 25%, 30%, 35%, 40%, 45%, 50%, 55%, 60%, 65%, 70%, 75%, 80%, 90%, 95%, 98% or 99%.

In some embodiments, the polypeptide is an agonist antibody that binds to the target protein (e.g., a target protein whose expression or activity is decreased in a cancer state relative to a reference state). In some embodiments, the antibody is an agonist antibody that binds to a protein capable of modulating the expression or activity of the target protein. As used herein, the term "agonist antibody" refers to an antibody that, upon binding to an antigen (e.g., the target protein or a protein capable of modulating the expression or activity of the target protein), increases the function of the antigen. In some embodiments, the antigen is a receptor, and the agonist antibody binds to the ligand-binding domain of the receptor. In some embodiments, the antigen is a transmembrane protein, and the agonist antibody binds to the extracellular region of the transmembrane protein. In some embodiments, the antigen is an enzyme or a signaling molecule, and the agonist antibody increases the activity of the enzyme or activates a signal transduction pathway mediated by the signaling molecule. In some embodiments, the agonist antibody increases antigen function by at least about 10%, e.g., by at least about: 15%, 20%, 25%, 30%, 35%, 40%, 45%, 50%, 55%, 60%, 65%, 70%, 75%, 80%, 90%, 95%, 100%, 200%, 300%, 400%, 500%, 600%, 700%, 800%, 900% or 1,000%.

In some embodiments, the agonist antibody does not exert at least one of the following functional properties: reducing (e.g., inhibiting) the activity of the antigen; inducing antibody-dependent cell killing of a cell expressing the antigen (e.g., by natural killer (NK) cells, monocytes, macrophages, neutrophils, dendritic cells, or eosinophils); inducing phagocytosis of a cell expressing the antigen (e.g., by macrophage); inducing opsonization of a cell expressing the antigen; and inducing downregulation of the antigen on a cell surface (e.g., by hyper-crosslinking or clustering the antigen to induce internalization and degradation).

Suitable techniques, assays and reagents for making and using therapeutic antibodies against an antigen are known in the art. *See,* for example, Therapeutic Monoclonal Antibodies: From Bench to Clinic (Zhiqiang An eds., 1st ed. 2009); Antibodies: A Laboratory Manual (Edward A. Greenfield eds., 2d ed. 2013); Ferrara et al., Using Phage and Yeast Display to Select Hundreds of Monoclonal Antibodies: Application to Antigen 85, a Tuberculosis Biomarker, PLoS ONE 7(11): e49535 (2012), for methods of making recombinant antibodies, including antibody engineering, use of degenerate oligonucleotides, 5'-RACE, phage display, and mutagenesis; antibody testing and characterization; antibody pharmacokinetics and pharmacodynamics; antibody purification and storage; and screening and labeling techniques.

In some embodiments, the polypeptide is an antibody mimetic that binds a target protein disclosed herein. The term "antibody mimetic" refers to polypeptides capable of mimicking an antibody's ability to bind an antigen, but structurally differ from native antibody structures. Non-limiting examples of antibody mimetics include Adnectins, Affibodies, Affilins, Affimers, Affitins, Alphabodies, Anticalins, Avimers, DARPins, Fynomers, Kunitz domain peptides, monobodies, nanobodies, nanoCLAMPs, and Versabodies.

In some embodiments (e.g., when the expression or activity of the target protein is decreased in a disease state relative to a reference state), the agent is a polypeptide (e.g., isolated polypeptide) that comprises an amino acid sequence that is at least 70% identical to at least a portion (e.g., a biologically active portion or fragment) of the target protein. For example, the percent identity can be at least about: 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% to the full-length target protein or a biologically active portion or fragment thereof. In some embodiments, the polypeptide comprises the amino acid sequence of a full-length target protein. In some embodiments, the polypeptide comprising the amino acid sequence of a full-length target protein is a recombinant polypeptide. In some embodiments, the polypeptide comprising the amino acid sequence of a full-length target protein is a synthetic polypeptide.

In some embodiments, the polypeptide (e.g., isolated polypeptide) comprises an amino acid sequence having at least 1 amino acid substitution relative to the target protein. For example, the number of amino acid substitutions can be at least about: 2, 3, 4, 5, 6, 7, 8, 9, 10, 11, 12, 13, 14, 15, 16, 17, 18, 19 or 20, or about: 1-20, 1-19, 2-19, 2-18, 2-17, 3-17, 3-16, 4-16, 4-15, 5-15, 5-14, 6-14, 6-13, 7-13, 7-12, 8-12, 8-11 or 9-11. In some embodiments, the amino acid substitutions are conservative substitutions. In some embodiments, the amino acid substitutions are highly conservative substitutions.

In some embodiments, the polypeptide (e.g., isolated polypeptide) comprises an amino acid sequence that is at least 70% identical to at least a portion of a protein capable of modulating the expression or activity of the target protein. For example, the percent identity can be at least about: 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%. In some embodiments, the polypeptide comprises the amino acid sequence of a protein capable of modulating the expression or activity of the target protein.

In some embodiments, the polypeptide (e.g., isolated polypeptide) comprises an amino acid sequence having at least 1 amino acid substitution relative to a protein capable of modulating the expression or activity of the target protein. For example, the number of amino acid substitutions can be at least about: 2, 3, 4, 5, 6, 7, 8, 9, 10, 11, 12, 13, 14, 15, 16, 17, 18, 19 or 20, or about: 1-20, 1-19, 2-19, 2-18, 2-17, 3-17, 3-16, 4-16, 4-15, 5-15, 5-14, 6-14, 6-13, 7-13, 7-12, 8-12, 8-11 or 9-11. In some embodiments, the amino acid substitutions are conservative substitutions. In some embodiments, the amino acid substitutions are highly conservative substitutions.

In some embodiments, the polypeptide is a cell-penetrating peptide. In certain embodiments, the polypeptide is linked to a cell-penetrating peptide. Suitable cell-penetrating peptide sequences can be protein-derived, designed or, chimeric (modified). *See,* e.g., Regberg, et al., Applications of cell-penetrating peptides for tumor targeting and future cancer therapies, Pharmaceuticals 5(9): 991-1007 (2012). Non-limiting examples of cell-penetrating peptides include TAT (48-60), Penetratin, pVEC, MPG8, Transportan, Transportan10, PepFect3, PepFect 6, PepFect 14, Polyarginine, Stearyl-polyarginine, Pep-1, Pep-3, CADY, YTA2, YTA4, SynB 1, SynB3, Maurocalcine and PTD4.

In some embodiments, the polypeptide is a circulating factor (e.g., a cytokine).

In some embodiments, the biological properties (e.g., biological activities or half-life) of the polypeptide (e.g., isolated polypeptide) and the target protein are similar. Non-limiting examples of biological activities include enzymatic activities or properties (e.g., selectivity, steady-state or kinetics), binding activities (e.g., nucleic acid (DNA, RNA) binding protein binding) or properties (e.g., specificity, affinity or kinetics), cell signaling activities, immunological activities, and structural activity (e.g., cell adhesion), among others. Non-limiting examples of enzyme activities include transferase activity (e.g., transfers functional groups from one molecule to another), oxioreductase activity (e.g., catalyzes oxidation-reduction reactions), hydrolase activity (e.g., cleaves chemical bonds via hydrolysis), lyase activity (e.g., generate a double bond), ligase activity (e.g., joining two molecules via a covalent bond), and isomerase activity (e.g., catalyzes structural changes within a molecule from one isomer to another).

In some embodiments, the polypeptide (e.g., isolated polypeptide) is a recombinant protein. In other embodiments, the polypeptide (e.g., isolated polypeptide) is a synthetic protein. Methods of producing therapeutic polypeptides are known in the art. *See,* e.g., Therapeutic Proteins: Methods and Protocols (Mark C. Smales & David C James eds., 2005*);* Pharmaceutical Biotechnology: Fundamentals and Applications (Daan J. A. Crommelin, Robert D. Sindelar & Bernd Meibohm eds., 2013). Polypeptides can be expressed recombinantly using mammalian cells, insect cells, yeast or bacteria *etc.,* under the control of appropriate promoters.

In some embodiments, the polypeptides described herein (e.g., target proteins, or a portion thereof, polypeptide agents that modulate target proteins) are modified, for example, by cleavage (e.g., protease cleavage) or post-translational modification. In certain embodiments, the modification(s) will affect the activity of the polypeptide, for example, by making an inactive polypeptide active or by altering (e.g., increasing, decreasing) the level of activity of a polypeptide. In particular embodiments, a polypeptide described herein is provided as a prodrug, e.g., that can be converted (e.g., by proteolytic cleavage, post-translational modification) to an active polypeptide in vivo. In some embodiments, the polypeptide includes a post-translational modification or other chemical modification. Non-limiting examples of post-translational modifications include acetylation, amidation, formylation, glycosylation, hydroxylation, methylation, myristoylation, phosphorylation, deamidation, prenylation (e.g., farnesylation, geranylation, etc.), ubiquitylation, ribosylation and sulphation. Phosphorylation can occur on an amino acid such as tyrosine, serine, threonine, or histidine.

In some embodiments, the polypeptide is joined to a heterologous peptide or protein (e.g., via a covalent bond such as a peptide bond, or a non-covalent bond), such as in a conjugate or fusion protein. In some embodiments, the polypeptide comprises a tag (e.g., a detectable label, such as a fluorophore or enzyme, or a purification tag, such as an epitope tag).

In some embodiments, the polypeptide comprises one or more neoantigens selected from the Sequence Listing, or a variant thereof. As used herein, the term "neoantigen" refers to a tumor antigen that arises from a target protein described herein. In some embodiments, the neoantigen is a cancer-specific neoantigen. There are a variety of ways to produce a neoantigen. For example, a neoantigen may be produced *in vitro* as a polypeptide before being formulated into a neoplasia vaccine or immunogenic pharmaceutical composition. In some embodiments, the immunogenic pharmaceutical composition comprises an effective amount of one or more neoantigens or pharmaceutically acceptable salt(s) thereof. In some embodiments, the immunogenic pharmaceutical composition further comprises a pharmaceutically acceptable carrier, excipient, adjuvant or additive.

Alternatively, a neoantigen may be produced *in vivo* by introducing a polynucleotide or an expression vector (e.g., a viral expression vector) encoding the neoantigen into a cell or tissue (e.g., of a subject in need). In certain embodiments, the polypeptide comprises at least two neoantigens. In some embodiments, the polypeptide comprises a T cell enhancer amino acid sequence. In some embodiments, the T cell enhancer is selected from the group consisting of an invariant chain, a leader sequence of tissue-type plasminogen activator, a PEST sequence, a cyclin destruction box, a ubiquitination signal, and a SUMOylation signal.

### B. Polynucleotide Agents

In some embodiments, the agent comprises a polynucleotide, or an analog or derivative thereof. In some embodiments, the polynucleotide, or the analog or derivative thereof, is an inhibitor of the target protein. In some embodiments, the polynucleotide, or the analog or derivative thereof, is an activator of the target protein. In some embodiments, the polynucleotide, or the analog or derivative thereof, decreases (e.g., reduces or neutralizes) the expression or activity of the target protein. In other embodiments, the polynucleotide, or the analog or derivative thereof, increases the expression or activity of the target protein.

The polynucleotides can have sequences containing naturally occurring ribonucleotide or deoxyribonucleotide monomers, non-naturally occurring nucleotides, or combinations thereof. Accordingly, polynucleotides can include, for example, nucleotides comprising naturally occurring bases (e.g., A, G, C, or T) and nucleotides comprising modified bases (e.g., 7-deazaguanosine, inosine, or methylated nucleotides, such as 5-methyl dCTP and 5-hydroxymethyl cytosine). In some embodiments, the polynucleotide comprises at least one modified nucleotide. Non-limiting examples of modified nucleotides include 2'-fluoro, 2'-o-methyl, 2'-deoxy, unlocked nucleic acid, 2'-hydroxy, phosphorothioate, 2'-thiouridine, 4'-thiouridine and 2'-deoxyuridine. In some embodiments, the modification increases nuclease resistance, increases serum stability, decrease immunogenicity, or a combination of the foregoing.

In some embodiments, the polynucleotide is a DNA molecule. In some embodiments, the polynucleotide is an RNA molecule. In some embodiments, the polynucleotide is a vector (e.g., expression vector, plasmid).

In some embodiments, the polynucleotide comprises an analog or a derivative of a polynucleotide. In some embodiments, the analog or derivative is a peptide nucleic acid (PNA). In some embodiments, the analog or derivative is a locked nucleic acid (LNA). In some embodiments, the analog or derivative is a morpholino oligonucleotide. In some embodiments, the analog or derivative comprises one or more phosphorothioate-linkages. In some embodiments, the agent comprises a deoxyribonucleic guanidine (DNG) nucleotide. In some embodiments, the agent comprises ribonucleic guanidine (RNG) nucleotide.

In some embodiments, the polynucleotide modulates the expression and/or activity of a nucleic acid encoding a target protein disclosed herein (e.g., a target protein in the Sequence Listing), or a portion thereof (e.g., a biologically active portion or fragment thereof).

In some embodiments, the polynucleotide comprises a nucleotide sequence that is complementary (e.g., fully complementary or partially complementary) to at least a portion of a gene or gene transcript encoding a target protein disclosed herein, such that the polynucleotide sequence is capable of hybridizing or annealing to the gene or gene transcript (e.g., under physiological conditions). In other embodiments, the polynucleotide comprises a nucleotide sequence that is complementary to at least a portion of a gene or gene transcript encoding a protein that is capable of modulating the expression or activity of a target protein disclosed herein.

In some embodiments, the polynucleotide encodes a target protein disclosed herein, or a variant thereof (e.g., a biologically active variant thereof), or a portion thereof (e.g., a biologically active portion or fragment thereof).

In some embodiments, the nucleic acid that encodes the target protein, or variant thereof, or portion (e.g., fragment) thereof, is a gene sequence or a portion thereof. In some embodiments, the encoding nucleic acid is an unprocessed RNA transcript (e.g., pre-mRNA) or a portion thereof (e.g., a 5'-UTR, a 3'-UTR, an intron). In some embodiments the encoding nucleic acid is a mRNA molecule or a portion thereof. In some embodiments the encoding nucleic acid is present in a non-coding RNA (e.g., a long intergenic non-coding RNA (lincRNA), long noncoding RNA (lncRNA), or miRNA).

The encoding nucleic acid can comprise a canonical open reading frame (ORF) or a non-canonical ORF. In certain embodiments, the encoding nucleic acid comprises a non-canonical ORF.

The polynucleotides can be single stranded (ss) or double stranded (ds). In some embodiments, the polynucleotide is double stranded (ds). In some embodiments, the length of the ds polynucleotide is about 15-50 base pairs, e.g., about: 15-45, 15-40, 15-35, 15-30, 15-25, 18-50, 18-45, 18-40, 18-35, 18-30, 18-25, 20-50, 20-45, 20-40, 20-35, 20-30, 20-25, 25-50, 25-45, 25-40, 25-35, 25-30, 30-50, 30-45, 30-40, 30-35, 35-50, 35-45, 35-40 or 40-50 base pairs. In some embodiments, the length of the polynucleotide is about 19-23 base pairs. In some embodiments, the length of the polynucleotide is about 21 base pairs.

In some embodiments, the polynucleotide is single stranded (ss). In some embodiments, the length of the ss polynucleotide is about 15-50 nucleotides, e.g., about: 15-45, 15-40, 15-35, 15-30, 15-25, 18-50, 18-45, 18-40, 18-35, 18-30, 18-25, 20-50, 20-45, 20-40, 20-35, 20-30, 20-25, 25-50, 25-45, 25-40, 25-35, 25-30, 30-50, 30-45, 30-40, 30-35, 35-50, 35-45, 35-40 or 40-50 nucleotides.

In some embodiments, the polynucleotide prevents the maturation of a newly-generated nuclear RNA transcript into an mRNA for transcription. In some embodiments, the polynucleotide comprises a nucleotide sequence that is complementary to a sequence at the boundary of an intron and an exon.

In some embodiments, the polynucleotide (e.g., an antisense oligonucleotide) can hybridize to an mRNA encoding the target protein (e.g., under physiological conditions). In some embodiments, the length of the polynucleotide is at least about 10 nucleotides, e.g., at least about: 15, 16, 17, 18, 19, 20, 21, 22, 23, 24, 25, 26, 27, 28, 29 or 30 nucleotides or about: 10-30, 15-30, 15-25, 20-25 nucleotides. In some embodiments, the polynucleotide is at least 75% identical to an antisense sequence of identical the targeted transcript, e.g., at least about: 80%, 85%, 90%, 95%, 96%, 97%, 98% or 99%.

In some embodiments, the polynucleotide further comprises an overhang sequence (e.g., unpaired, overhanging nucleotides which are not directly involved in the formation of the double helical structure by the core sequences). In some embodiments, the polynucleotide comprises a 3' overhang, a 5' overhang, or both. In some embodiments, the overhang is about 1-5 nucleotides. In some embodiments, the overhang comprises a modified ribonucleotide or deoxynucleotide, e.g., a thiophosphate, phosphorothioate or deoxynucleotide inverted (3' to 3' linked) nucleotide.

Non-limiting examples of polynucleotide agents suitable for use in the compositions, kits and methods described herein include a small interfering RNA (siRNA), a short hairpin RNA (shRNA), a microRNA (miRNA), an antagomir, an antisense DNA, an antisense RNA, a morpholino nucleic acid (MNA), a locked nucleic acid (LNA), a peptide nucleic acid (PNA), an aptamer and a guide RNA (gRNA).

In some embodiments, the polynucleotide inhibits gene expression (e.g., through the biological process of RNA interference (RNAi)). Polynucleotides appropriate for RNA interference can be readily designed and produced by a person of ordinary skill using techniques, assays and reagents known in the art, including computational tools. *See,* e.g., Pei et al. 2006, Reynolds et al. 2004, Khvorova et al. 2003, Schwarz et al. 2003, Ui-Tei et al. 2004, Heale et al. 2005, Chalk et al. 2004, Amarzguioui et al. 2004.

In some embodiments, the polynucleotide is a miRNA. In some embodiments, the miRNA is about 22 nucleotides in length. miRNAs bind to target sites on mRNA molecules and silence the mRNA, e.g., by causing cleavage of the mRNA, destabilization of the mRNA, or inhibition of translation of the mRNA.

In some embodiments, the polynucleotide is a siRNA. In some embodiments, the siRNA comprises a nucleotide sequence that is identical to about a 15-25 contiguous mRNA sequence encoding the target protein. In some embodiments, the siRNA is a double-stranded RNA molecule having about 19-25 base pairs. In some embodiments, the siRNA commences with the dinucleotide AA. In some embodiments, the siRNA has a GC-content of about 30-70%, e.g., about: 30-65%, 30-60%, 30-55%, 30-50%, 40-70%, 40-65%, 40-60%, 40-55%, 45-70%, 45-65%, 45-60% or 45%-55%.

In some embodiments, the polynucleotide is a shRNA. A shRNA is an RNA molecule including a hairpin turn that decreases expression of target genes via RNAi. shRNAs can be delivered to cells in the form of plasmids, e.g., viral or bacterial vectors, e.g., by transfection, electroporation, or transduction.

siRNAs and shRNAs resemble intermediates in the processing pathway of the endogenous microRNA (miRNA) genes *(see,* e.g., Bartel, Cell 116:281-97 (2004)). In some embodiments, the siRNA functions as an miRNA; in other embodiments, the miRNA functions as an siRNA *(see,* e.g., Zeng et al., Mol Cell 9:1327-33 (2002); Doench et al., and Genes Dev 17:438-42 (2003)). MicroRNAs, like siRNAs, use RISC to downregulate target genes, but unlike siRNAs, most animal miRNAs do not cleave the mRNA. Instead, miRNAs reduce protein output through translational suppression or polyA removal and mRNA degradation (see, e.g., Wu et al., Proc Natl Acad Sci USA 103:4034-39 (2006)).
Known miRNA binding sites are within mRNA 3' UTRs; miRNAs seem to target sites with near-perfect complementarity to nucleotides 2-8 from the miRNA's 5' end *(see,* e.g., Rajewsky, Nat Genet 38 Suppl: S8-13 (2006) and Lim et al., Nature 433:769-73 (2005)). This region is known as the seed region. Because siRNAs and miRNAs are interchangeable, exogenous siRNAs downregulate mRNAs with seed complementarity to the siRNA *(see,* e.g., Birmingham et al., Nat Methods 3:199-204 (2006)). Multiple target sites within a 3' UTR give stronger downregulation *(see,* e.g., Doench et al., Genes Dev 17: 438-42 (2003)).

In some embodiments, the polynucleotide is a messenger RNA (mRNA) or a circular RNA (circRNA) encoding a target protein disclosed herein or a variant thereof (e.g., a variant that is at least about 70% identical (e.g., at least about: 75%, 80%, 85%, 90%, 95%, 98% or 99% identical to the wild-type protein). In some embodiments, the mRNA is codon optimized (e.g., to improve efficacy of protein synthesis and limit mRNA destabilization by rare codons *(see,* e.g., Presnyak et al., Cell. 160 (6): 1111-24 (2015) and Thess et al., Mol Ther. 23(9): 1456-64 (2015)).

In some embodiments, a polynucleotide comprising RNA is chemically synthesized. In some embodiments, a polynucleotide comprising RNA is expressed recombinantly. In some embodiments, the RNA is transcribed *in vitro.* The making and using of RNA therapeutics are known in the art. *See,* for example, *RNA Therapeutics: Function,*

Design, and Delivery (Mouldy Sioud eds., 2010) and Kaczmarek et al., Advances in the delivery of RNA therapeutics: from concept to clinical reality, Genome Medicine 9:60 (2017).

In some embodiments, the mRNA is produced by *in vitro* transcription. In some embodiments, the mRNA is modified to optimize its activity. In some embodiments, the mRNA comprises a modified base, a 5' cap, a 5' cap analogue, an anti-reverse cap analog (ARCA), or a combination thereof.

In some embodiments, the mRNA comprises a poly(A) tail. In some embodiments, the poly(A) tail is about 100-200 nucleotides. In some embodiments, the poly(A) tail improves the expression and/or stability of the mRNA *(see,* e.g., Kaczmarek et al., Genome Medicine 9:60 (2017)).

In some embodiments, the mRNA comprises 5' cap. In some embodiments, the mRNA comprises a 5' cap analogue. In some embodiments, the 5' cap analogue is a 1,2-dithiodiphosphate-modified cap *(see,* e.g., Strenkowska et al., Nucleic Acids Res. 44:9578-90 (2016)).

In some embodiments, the mRNA comprises a modified 3' untranslated region (UTR), a 5' UTR, or both. In some embodiments, the modified UTR comprises sequences responsible for recruiting RNA-binding proteins (RBPs) and miRNAs (e.g., to enhance the level of protein product *(see,* e.g., Kaczmarek et al., Genome Medicine 9:60 (2017)). In some embodiments, the 3' UTR, 5' UTR, or both are modified to encode a regulatory element. In some embodiments, the regulatory element comprises a K-turn motif, a miRNA binding site, or a combination thereof to control RNA expression in a cell-specific manner *(see,* e.g., Wroblewska et al., Nat Biotechnol. 33:839-41 (2015)).

In some embodiments, the mRNA comprises an RNA base modification. In some embodiments, the mRNA comprises a pseudouridine. In some embodiments, the mRNA comprises a N1-methyl-pseudouridine (e.g., to mask immune-stimulatory activity and enhance translation initiation *(see,* e.g., Andries et al., J Control Release 217:337-44 (2015) and Svitkin et al., Nucleic Acids Res. 45:6023-36 (2017)).

In some embodiments, the RNA (e.g., mRNA) is a circular RNA.

Compositions and methods for producing mRNA are disclosed, *see,* e.g., in WO2016011306, WO2016014846, WO2016022914, WO2016077123, WO2016164762, WO2016201377, WO2017049275, US9937233, US8710200, US10022425, US9878056, US9572897, WO2010084371, US9353153, WO2015034925 and WO2019236673. Also *see,* e.g., Jemielity et al., RNA 9(9):1108-22 (2003); Mockey et al., Biochem Biophys Res Commun. 340: 1062-88 (2006); Strenkowska et al. Nucleic Acids Res. 44: 9578-90 (2016); Presnyak et al., Cell 160: 1111-24 (2015) and Kaczmarek et al., Genome Medicine 9:60 (2017)). In some embodiments, the mRNA is prepared in a lipid nanoparticle (LNP) formulation (e.g., for *in vivo* delivery, *see,* e.g., US Pat. 9,764,036, US Pat. 9,682,139, Kauffman et al., Nano Lett. 15: 7300-6 (2015) and Fenton et al., Adv Mater. 28: 2939-43 (2016)).

In some embodiments, the polynucleotide is an aptamer. In certain embodiments, the aptamer binds to a target protein disclosed herein. In particular embodiments, the aptamer binds to a binding partner of a target protein disclosed herein.

In some embodiments, the polynucleotide is linked (e.g., covalently) to a delivery polymer. In some embodiments, the link between the polynucleotide and the delivery polymer is reversible. In some embodiments, the polynucleotide is linked to the delivery polymer via a physiologically labile linker. In some embodiments, the physiologically labile linker is a disulfide bond.

In some embodiments, the polynucleotide is conjugated to the polymer in the presence of an excess of polymer. In some embodiments, the excess polymer is removed prior to administration (e.g., to a cell or a subject).

A person of ordinary skill in the art can readily make suitable polynucleotide agents for use in the compositions, kits and methods described herein using the gene locus information of the protein sequences contained in the Sequence Listing incorporated herein, such as the chromosomal location, starting and ending nucleotide positions, and polymorphism identifications.

### C. Agents Comprising Gene Editing Systems

In some embodiments, the agent comprises a gene editing system. In some embodiments, the gene editing system produces a deletion of nucleotides, a substitution of nucleotides, an addition of nucleotides or a combination of the foregoing, in a gene encoding a target protein.

In some embodiments, the gene editing system is a CRISPR/Cas system, a transposon-based gene editing system, or a transcription activator-like effector nuclease (TALEN) system. In some embodiments, the gene editing system is a CRISPR/Cas system. In some embodiments, the gene editing system is a class II CRISPR/Cas system.

In some embodiments, the gene editing system (e.g., the CRISPR/Cas system) reduces (e.g., decreases, inhibits) or eliminates (e.g., via gene knockout) the expression of the target protein. In some embodiments, the gene editing system (e.g., the CRISPR/Cas system) reduces (e.g., decreases, inhibits) or eliminates (e.g., via gene knockout) the expression of a protein capable of modulating the expression or activity of the target protein. In some embodiments, the gene editing system (e.g., the CRISPR/Cas system) increases (e.g., via gene knock-in or gene replacement) the expression of the target protein. In some embodiments, the gene editing system (e.g., the CRISPR/Cas system) increases (e.g., via gene knock-in or gene replacement) the expression of a protein capable of modulating the expression or activity of the target protein.

In some embodiments, the CRISPR system specifically catalyzes cleavage in a gene encoding the target protein, thereby inactivating said gene. Repairing nucleic acid strand breaks through non-homologous end joining (NHEJ) often results in changes to the DNA sequence at the site of the cleavage, resulting in small insertions or deletions (Indels). In some embodiments, NHEJ is used to knock out the gene encoding the target protein. In some embodiments, homology directed repair (HDR) is used to concurrently inactivate a gene encoding the target protein and insert a heterologous sequence into the inactivated locus. Cells in which a knockout and/or knockin event has occurred can be identified and/or selected by well-known methods in the art.

In some embodiments, the gene editing system comprises a single Cas endonuclease or a polynucleotide encoding the single Cas endonuclease. In some embodiments, the single Cas endonuclease is Cas9, Cpfl, C2C1 or C2C3. In some embodiments, the single Cas endonuclease is Cas9 (e.g., of *Streptococcus Pyogenes*)*.* In some embodiments, the single Cas endonuclease is Cpfl. In some embodiments, the Cpfl is AsCpf1 (from *Acidaminococcus sp.*) or LbCpf1 (from *Lachnospiraceae sp.*)*.* The choice of nuclease and gRNA(s) will typically be determined according to whether a deletion, a substitution, or an addition of nucleotide(s) to a targeted sequence is desired.

In some embodiments, the type II Cas endonuclease is Cas 9 (e.g., of *Streptococcuspyogenes*)*.* In some embodiments, the modified Cas 9 is nickase Cas9, dead Cas9 (dCas9) or eSpCas9. In some embodiments, the nickase Cas9 is Cas9 D10A. In some embodiments, the dCas9 is D10A or H840A. In some embodiments, the gene editing system comprises a double nickase Cas9 (e.g., to achieve more accurate genome editing, see, e.g., Ran et al., Cell 154: 1380-89 (2013). Wild-type Cas9 generates double-strand breaks (DSBs) at specific DNA sequences targeted by a gRNA. Nickase Cas9 generates only a single-strand break. dCas9 is catalytically inactive. In some embodiments, dCas9 is fused to a nuclease (e.g., a FokI to generate DSBs at target sequences homologous to two gRNAs). Various CRISPR/Cas9 plasmids are publicly available from the Addgene repository (Addgene, Cambridge, MA: addgene.org/crispr/).

In some embodiments, the gene editing system comprises:
a) a wild-type or modified type II Cas endonuclease or a polynucleotide encoding the wild-type or modified type II Cas endonuclease;
b) a CRISPR RNA ("crRNA"); and
c) a trans-activating crRNA ("tracrRNA").

In some embodiments, the crRNA comprises at least 1 "guide RNA" (sgRNA), e.g., at least: 2, 3 or 4 gRNAs. In some embodiments, the gRNA comprises a sequence that is identical to a portion of the gene sequence of the target protein. In some embodiments, the gRNA comprises a sequence that is identical to a portion of the gene sequence of a protein capable of modulating the expression or activity of the target protein. In some embodiments, the gRNA is at least about 16 nucleotides, e.g., at least about: 17, 18, 19, 20, 21, 22, 23, 24 or 25 nucleotides; or about: 16, 17, 18, 19, 20, 21, 22, 23, 24 or 25 nucleotides; or about: 16-24, 17-24, 17-23, 18-23, 18-22, 19-22 or 19-21 or 19, 20 or 21 nucleotides. In some embodiments, the sgRNA is chemically modified.

Design of gRNA sequences for gene editing are known in the art. *See,* for example, Cong et al., Science, 339: 819-23 (2013) and Ran et al., Nature Protocols 8: 2281-308 (2013). Cas9 requires at least about 16 or 17 nucleotides of gRNA sequence to cleave DNA, and Cpf1 requires at least about 16 nucleotides of gRNA sequence to cleave DNA. In practice, a gRNA sequence has a length of about 17-24 nucleotides (e.g., about: 19, 20 or 21 nucleotides) and is complementary to a target gene. Custom gRNA generators and algorithms are commercially available. Chemically modified sgRNAs have also been demonstrated to be effective in genome editing *(see,* e.g., Hendel et al., Nature Biotechnol., 985-91 (2015)).

In some embodiments, the crRNA further comprises a sequence capable of binding to the tracrRNA. Upon binding, the partially double-stranded structure is cleaved by RNase III, the resulting crRNA/tracrRNA hybrid directs the Cas9 endonuclease to recognize and cleave a target DNA sequence.

In some embodiments, the target DNA sequence is approximate to a "protospacer adjacent motif" ("PAM") that is specific for a Cas endonuclease. PAM sequences appear throughout a given genome. CRISPR endonucleases of various prokaryotic species have unique PAM sequence requirements. Non-limiting examples of PAM sequences include: 5'-NGG (Streptococcus pyogenes), 5'-NNAGAA (Streptococcus thermophilus CRISPR1), 5'-NGGNG (Streptococcus thermophilus CRISPR3) and 5'-NNNGATT (Neisseria meningiditis). Some endonucleases, e.g., Cas9 endonucleases, are associated with G-rich PAM sites, e.g., 5'-NGG, and perform blunt-end cleaving of the target DNA at a location that is 3 nucleotides upstream (5') of the PAM site.

In some embodiments, the gene editing system comprises:
a) a wild-type or modified type II Cas endonuclease or a polynucleotide encoding the wild-type or modified type II Cas endonuclease; and
b) a crRNA.

Cpfl-associated CRISPR arrays are processed into mature crRNAs without the requirement of a tracrRNA. Cpf1 endonucleases, are associated with T-rich PAM sites, e.g., 5'-TTN. Cpfl can also recognize a 5'-CTA PAM motif. Cpfl cleaves the target DNA by introducing an offset or staggered double-strand break with a 4- or 5-nucleotide 5' overhang, for example, cleaving a target DNA with a 5-nucleotide offset or staggered cut located 18 nucleotides downstream (3') of the PAM site on the coding strand and 23 nucleotides downstream from the PAM site on the complimentary strand. The 5-nucleotide overhang that results from such offset cleavage allows more precise genome editing by DNA insertion by homologous recombination than by insertion at blunt-end cleaved DNA. *See,* e.g., Zetsche et al., Cell 163:759-71 (2015).

In some embodiments, the gene editing system activates or represses transcription of a target gene. In some embodiments, the gene editing system comprises:
a) a chimeric protein comprising dCas9 and one or more effector domains; and
b) one or more sgRNAs.

In some embodiments, the chimeric protein represses the expression of the target protein (CRISPRi). In some embodiments, the chimeric protein activates the expression of the target protein (CRISPRa). In some embodiments, the chimeric protein methylates a DNA sequence recognized by the sgRNA. In some embodiments, the chimeric protein demethylates a DNA sequence recognized by the sgRNA.

An effector domain comprises a biologically active portion of an effector protein (e.g., transcriptional activator or repressor). In some embodiments, the gene editing system comprises 1 effector domain. In some embodiments, the gene editing system comprises at least 2 effector domains, e.g., 2, 3 or 4 effector domains. In some embodiments, the effector domain comprises KRAB. In some embodiments, the effector domain comprises VP64. In some embodiments, the effector domain comprises VP64, p65 and Rta. In some embodiments, the dCas9 is D10A. In some embodiments, the dCas9 is H840A.

Because dCas9 is catalytically inactive, dCas9 does not cut the target DNA but interferes with transcription by steric hindrance. The dCas9 chimeric protein (e.g., dCas9-VPR), guided by the one or more gRNAs to the upstream sequence of a transcriptional start site (TSS) of the target gene, modulates transcription of the target gene. *See,* e.g., Gilbert et al., CRISPR-Mediated Modular RNA-Guided Regulation of Transcription in Eukaryotes, Cell 154, 442-51 (2013); Cheng et al., Multiplexed activation of endogenous genes by CRISPR-on, an RNA-guided transcriptional activator system, Cell Res. 23: 1163-71 (2013); Gilbert et al., Genome-Scale CRISPR-Mediated Control of Gene Repression and activation, Cell 159: 647-61 (2014); Tanenbaum et al., A protein-tagging system for signal amplification in gene expression and fluorescence imaging, Cell 159: 635-46 (2014); Konermann et al., Genome-scale transcriptional activation by an engineered CRISPR-Cas9 complex, Nature 517: 583-88 (2015); Chavez et al., Highly efficient Cas9-mediated transcriptional programming, Nat. Methods. 12: 326-28 (2015); Zalatan et al., Engineering complex synthetic transcriptional programs with CRISPR RNA scaffolds, Cell 160: 339-50 (2015); Horlbeck et al., Compact and highly active next-generation libraries for CRISPR-mediated gene repression and activation, eLife. 5: e19760 (2016); Chavez et al., Comparison of Cas9 activators in multiple species, Nat Methods. 7: 563-67 (2016).

CRISPR technology for editing the genes of eukaryotes is disclosed in US Patent Application Publications 2016/0138008A1 and US2015/0344912A1, and in US Patents 8,697,359, 8,771,945, 8,945,839, 8,999,641, 8,993,233, 8,895,308, 8,865,406, 8,889,418, 8,871,445, 8,889,356, 8,932,814, 8,795,965, and 8,906,616. Cpfl endonuclease and corresponding guide RNAs and PAM sites are disclosed in US Patent Application Publication 2016/0208243 A1. CRISPR technology for generating mtDNA dysfunction in the mitochondrial genome is disclosed in Jo et al., BioMed Res. Int. 2015: 305716 (2015). Co-delivery of Cas9 and sgRNA with nanoparticles is disclosed in Mout et al., ACS Nano 11(3): 2452-58 (2017).

In some embodiments, the agent comprises a transposon-based gene editing system. An example of a suitable transposon-based gene editing system for use in the disclosure provided herein is the Gene Writer system described in International Publication Number WO 2020/047124, published on March 5, 2020, the contents of which are incorporated herein by referenced in their entirety.

In some embodiments, the agent comprises a transcription activator-like effector nuclease (TALEN) system. TALEN-based systems comprise a protein comprising a TAL effector DNA binding domain and an enzymatic domain. They are made by fusing a TAL effector DNA-binding domain to a DNA cleavage domain (a nuclease which cuts DNA strands). The FokI restriction enzyme described above is an exemplary enzymatic domain suitable for use in TALEN-based gene-regulating systems.

TAL effectors are proteins that are secreted by Xanthomonas bacteria via their type III secretion system when they infect plants. The DNA binding domain contains a repeated, highly conserved, 33-34 amino acid sequence with divergent 12th and 13th amino acids. These two positions, referred to as the Repeat Variable Diresidue (RVD), are highly variable and strongly correlated with specific nucleotide recognition. Therefore, the TAL effector domains can be engineered to bind specific target DNA sequences by selecting a combination of repeat segments containing the appropriate RVDs. The nucleic acid specificity for RVD combinations is as follows: HD targets cytosine, NI targets adenine, NG targets thymine, and NN targets guanine (though, in some embodiments, NN can also bind adenine with lower specificity)

In some embodiments, the TAL effector domains bind to a target DNA sequence that is at least 90%, 95%, 96%, 97%, 98%, or 99% identical, or is 100% identical to a target DNA sequence of the target protein. In some embodiments, the TAL effector domains bind to a target DNA sequence that is at least 90%, 95%, 96%, 97%, 98%, or 99% identical, or is 100% identical to a target DNA sequence defined by a set of genomic coordinates.

In some embodiments, the gene-regulating system comprises two or more TAL effector-fusion proteins each comprising a TAL effector domain, wherein at least one of the TAL effector domains binds to a target DNA sequence of the target protein. In some embodiments, at least one of the two or more TAL effector domains binds to a target DNA sequence that is at least 90%, 95%, 96%, 97%, 98%, or 99% identical, or is 100% identical to a target DNA sequence defined by a set of genomic coordinates.

Methods and compositions for assembling the TAL-effector repeats are known in the art. See e.g., Cermak et al, Efficient design and assembly of custom TALEN and other TAL effector-based constructs for DNA targeting, Nucleic Acids Res 39(12): e82 (2011). Plasmids for constructions of the TAL-effector repeats are commercially available from e.g., Addgene.

In some embodiments, the agent comprises a zinc finger nuclease (ZFN) system. ZFN domains can be generated using commercially available plasmids, such as, for example, plasmid pairs (CSTZFN-1KT COMPOZR^{®} Custom Zinc Finger Nuclease (ZFN) R-3257609) from Sigma Aldrich (St. Louis, MO). Plasmids can be prepared using the commercial system following manufacturer's protocol (e.g., NEB Monarch Miniprep (Cat# T1010), New England Biolabs, Ipswich, MA).

In some embodiments, the agent comprises a vector designed for delivering conventional gene therapy (e.g., gene knockout or knock-in via homologous recombination). Non-limiting examples of said vectors include retrovirus (e.g., Lentivirus 5), adenovirus, adeno-associated virus, Herpes Simplex Virus, nanoparticles and DNA Transposons.

### D. Small Molecule Agents

In some embodiments, the agent comprises a small molecule. In some embodiments, the small molecule binds to the target protein. In some embodiments, the small molecule binds to a protein capable of modulating the expression or activity of the target protein. In some embodiments, the small molecule is an inhibitor of the target protein (e.g., a direct inhibitor, an indirect inhibitor). In some embodiments, the small molecule is an activator of the target protein (e.g., a direct activator, and indirect activator).

Examples of small molecules include organic compounds, organometallic compounds, inorganic compounds, and salts of organic, organometallic or inorganic compounds. Atoms in a small molecule are typically linked together via covalent and/or ionic bonds. In certain embodiments, the small molecule is a small organic molecule. The arrangement of atoms in a small organic molecule may represent a chain (*e.g.* a carbon-carbon chain or a carbon-heteroatom chain), or may represent a ring containing carbon atoms, *e.g.* benzene or a polycyclic system, or a combination of carbon and heteroatoms, *i.e.,* heterocycles such as a pyrimidine or quinazoline. Although small molecules can have a wide range of molecular weights, they generally include molecules that are less than about 5,000 daltons. For example, such small molecules can be less than about 1000 daltons and, preferably, are less than about 750 daltons or, more preferably, are less than about 500 daltons. Small molecules can be found in nature (*e.g*., identified, isolated, purified) and/or produced synthetically (*e.g*., by traditional organic synthesis, bio-mediated synthesis, or a combination thereof). See *e.g*. Ganesan, Drug Discov. Today 7(1): 47-55 (January 2002); Lou, Drug Discov. Today, 6(24): 1288-1294 (December 2001). Examples of naturally occurring small molecules include, but are not limited to, hormones, neurotransmitters, nucleotides, amino acids, sugars, lipids, and their derivatives.

In particular embodiments, the agent comprises a proteolysis targeting chimera (PROTAC).

A small molecule suitable for use in the compositions, kits and methods of this disclosure can be identified by a person of ordinary skill in the art using any of the screening methods disclosed herein.

### E. Therapeutic Cells and Cell-Based Therapies

In some embodiments, the agent comprises a therapeutic cell. In certain embodiments, the therapeutic cell expresses and/or is engineered to express a target protein (e.g., a target protein in the Sequence Listing or a variant thereof), a polypeptide (e.g., an antibody, an antigen-binding fragment or a polypeptide that comprises an amino acid sequence that is at least 70% identical to at least a portion of the target protein), a polynucleotide (e.g., a recombinant DNA, an RNA such as mRNA or siRNA), and/or a gene editing system (e.g., a CRISPR/Cas system) described herein.

In some embodiments, a polypeptide disclosed herein (e.g., an antibody or antigen-binding fragment) is incorporated into a cell-based therapy. In some embodiments, the polypeptide is an engineered T cell receptor. In some embodiments, the polypeptide is a chimeric antigen receptor (CAR) (e.g., expressed on a T (CAR-T) cell, natural killer (CAR-NK) cell, or macrophage (CAR-M) cell). In some embodiments, the CAR comprises a transmembrane domain and an antigen-recognition moiety, wherein the antigen-recognition moiety binds the target protein. In certain embodiments, the polypeptide is expressed by a therapeutic cell (e.g., a CAR-T, CAR-NK or CAR-M cell). In particular embodiments, the polypeptide is a cytokine receptor expressed on the membrane of a CAR-T, CAR-NK or CAR-M cell. In more particular embodiments, the polypeptide is a cytokine secreted from a CAR-T, CAR-NK or CAR-M cell.

A therapeutic cell suitable for use in the compositions, kits and methods of this disclosure can be generated, identified and/or enriched with methods known to a person of ordinary skill in the art. Non-limiting examples of said methods include purifying, propagating and/or differentiating cells from a subject (e.g., a human) to a specific cell product; engineering a somatic cell for gene therapy; cell immortalization; *ex vivo* gene modification of a cell (e.g., using viral vector and/or lipid nanoparticle delivery technologies); *in vivo* gene modification of a cell (e.g., using viral vector and/or lipid nanoparticle delivery technologies); genome editing; cell plasticity technologies; gene modifications; and flow cytometry. In some embodiments, the therapeutic cell is autologous or syngeneic. In other embodiments, the therapeutic cell is allogeneic.

### Expression Vectors and Hosts

In another aspect, the disclosure provides an expression vector comprising a polynucleotide described herein.

The term "expression vector" refers to a replicable nucleic acid from which one or more proteins can be expressed when the expression vector is transformed into a suitable expression host cell.

In some embodiments, the expression vector comprises an expression control polynucleotide sequence operably linked to the polynucleotide, a polynucleotide sequence encoding a selectable marker, or both. In some embodiments, the expression control polynucleotide sequence comprises a promoter sequence, an enhancer sequence, or both. In some embodiments, the expression control polynucleotide sequence comprises an inducible promoter sequence. The term "promoter" refers to a region of DNA to which RNA polymerase binds and initiates the transcription of a gene. The term "operably linked" means that the nucleic acid is positioned in the recombinant polynucleotide, e.g., vector, in such a way that enables expression of the nucleic acid under control of the element (e.g., promoter) to which it is linked. The term "selectable marker element" is an element that confers a trait suitable for artificial selection. Selectable marker elements can be negative or positive selection markers. Non-limiting examples of expression vectors for use with bacterial, fungal, yeast, and mammalian cellular hosts are described in Molecular Cloning: A Laboratory Manual (Michael R. Green & Joseph Sambrook eds., 4th ed. 2012).

In another aspect, the disclosure provides an expression host cell comprising any one or more of the polynucleotides or expression vectors described herein.

The term "expression host cell" refers to a cell useful for receiving, maintaining, reproducing and/or amplifying a vector.

Non-limiting examples of expression host cells include mammalian cells such as hybridoma cells, Baby Hamster Kidney fibroblasts (BHK cells), Chinese hamster ovary (CHO) cells, COS cells, HeLa cells, and human embryonic kidney (HEK), yeast cells such as *Pichia pastoris* cells, or bacterial cells such as DH5α, etc. *See,* e.g., Mammalian Cell Cultures for Biologics Manufacturing (Weichang Zhou & Anne Kantardjieff eds., 2014) for processes of host cell culture for production of protein therapeutics; Protein Biotechnology: Isolation, Characterization, and Stabilization (Felix Franks eds., 2013) and Protein Purification Protocols (Paul Cutler eds., 2010) for purification of protein therapeutics; and Therapeutic Protein Drug Products: Practical Approaches to formulation in the Laboratory, Manufacturing, and the Clinic (Brian K Meyer eds., 2012) for formulation of protein therapeutics.

A polynucleotide or expression vector described herein can be introduced into a suitable or desired host cell using techniques known in the art, including transformation, electroporation, and transduction. The introduced nucleic acid can be extrachromosomal in the host cell, or integrated into the host cell's genome.

### Pharmaceutical Compositions

In another aspect, the disclosure provides a pharmaceutical composition, wherein the pharmaceutical composition comprises an agent disclosed herein, and a pharmaceutically acceptable carrier. As used herein, the term "pharmaceutical composition" refers to a composition having pharmacological activity or other direct effect in mitigating, treating, or preventing cancer, or a finished dosage form or formulation thereof.

In some embodiments, the composition (e.g., pharmaceutical composition) comprises pharmaceutically acceptable carriers, excipients, stabilizers, diluents or tonifiers (Remington's Pharmaceutical Sciences 16th edition, Osol, A. Ed. (1980)). Suitable pharmaceutically acceptable carriers, excipients, or stabilizers are nontoxic to recipients at the dosages and concentrations employed. Non-limiting examples of pharmaceutically acceptable carriers, excipients, stabilizers, diluents or tonifiers include buffers (e.g., phosphate, citrate, histidine), antioxidants (e.g., ascorbic acid or methionine), preservatives, proteins (e.g., serum albumin, gelatin or immunoglobulins); hydrophilic polymers, amino acids, carbohydrates (e.g., monosaccharides, disaccharides, glucose, mannose or dextrins); chelating agents (e.g., EDTA), sugars (e.g., sucrose, mannitol, trehalose or sorbitol), salt-forming counter-ions (e.g., sodium), metal complexes (e.g., Zn-protein complexes); non-ionic surfactants (e.g., Tween), PLURONICS^{™} and polyethylene glycol (PEG).

In some embodiments, the agent of the pharmaceutical compositions (e.g., polypeptide, polynucleotide, or small molecule) is modified, e.g., conjugated to a heterologous moiety. The term "conjugated" refers to attached, via a covalent or noncovalent interaction. Conjugation can employ any of suitable linking agents; non-limiting examples include peptide linkers, compound linkers, and chemical cross-linking agents.

In some embodiments, the heterologous moiety is a marker (e.g., a fluorescent or radioactive marker), a molecule that stabilizes the agent, a molecule that targets the agent (e.g., to a particular cell or tissue, to facilitate or prevent from crossing the blood brain barrier), or a combination thereof.

In some embodiments, the heterologous moiety is polyethylene glycol (PEG), hexadecanoic acid, hydrogels, nanoparticles, multimerization domains and carrier peptides. In some embodiments, the nanoparticle is a lipid nanoparticle. In some embodiments, the nanoparticle is a polymer nanoparticle. In some embodiments, the polymer is an amphiphilic polymer. In other embodiments, the polymer is a hydrophobic or hydrophilic polymer. Non-limiting examples of polymers include poly(lactic acid)-poly(ethylene glycol), poly(lactic-co-glycolic acid)-poly(ethylene glycol), poly(lactic-co-glycolic) acid (PLGA), poly(lactic-co-glycolic acid)-d-α-tocopheryl polyethylene glycol succinate, poly(lactic-co-glycolic acid)-ethylene oxide fumarate, poly(glycolic acid)-poly(ethylene glycol), polycaprolactone-poly(ethylene glycol), or any salts thereof. In some embodiments, the polymer nanoparticle comprises poly(lactic-co-glycolic) acid (PLGA).

In some embodiments, the composition (e.g., pharmaceutical composition) is formulated for a suitable administration schedule and route. Non-limiting examples of administration routes include oral, rectal, mucosal, intravenous, intramuscular, subcutaneous and topical, etc. In some embodiments, the composition (e.g., pharmaceutical composition) is stored in the form of an aqueous solution or a dried formulation (e.g., lyophilized). In some embodiments, the composition is formulated to be administered by infusion (e.g., intravenous infusion).

In some embodiments, the composition is formulated to be administered with one or more additional therapeutic agents (e.g., with a second therapeutic agent) as a combination therapy. As used herein, a "combination therapy" or "administered in combination" means that two (or more) different agents or treatments are administered to a subject as part of a defined treatment regimen for a particular disease or condition. Non-limiting examples of additional agents or treatments include biologics (e.g., antibodies, peptides), cell therapies, gene therapies, immunotherapies, and small molecules impacting immune-mediated diseases or conditions.

The treatment regimen defines the doses and periodicity of administration of each agent such that the effects of the separate agents on the subject overlap. In some embodiments, the two or more agents are administered in a sequential manner as part of a prescribed regimen. In other embodiments, the delivery of the two or more agents is simultaneous or concurrent. In some embodiments, the two or more agents are co-formulated. In some embodiments, administration of two or more agents or treatments in combination is such that the reduction in a symptom, or other parameter related to the disorder is greater than what would be observed with one agent or treatment delivered alone or in the absence of the other. The effect of the two treatments can be partially additive, wholly additive, or greater than additive (e.g., synergistic). Each of the two or more therapeutic agents can be administered by any appropriate route including, but not limited to, oral routes, intravenous routes, intramuscular routes, and direct absorption through mucous membrane tissues. The two or more therapeutic agents can be administered by the same route or by different routes.

In some embodiments, the agent or pharmaceutical composition of the disclosure is delivered by a viral vector, e.g., by contacting a cell with a viral vector, locally administered (e.g., injected) to a tumor, or systemically (e.g., intravenously or orally) administered to a subject (e.g., a human patient).

Viral genomes provide a rich source of vectors that can be used for the efficient delivery of exogenous genes into a mammalian cell. Viral genomes are particularly useful vectors for gene delivery because the polynucleotides contained within such genomes are typically incorporated into the nuclear genome of a mammalian cell by generalized or specialized transduction. These processes occur as part of the natural viral replication cycle, and do not require added proteins or reagents to induce gene integration. Non-limiting examples of viral vectors include retrovirus (e.g., Retroviridae family viral vector), adenovirus (e.g., Ad5, Ad26, Ad34, Ad35, and Ad48), parvovirus (e.g., adeno-associated viruses), coronavirus, negative strand RNA viruses such as orthomyxovirus (e.g., influenza virus), rhabdovirus (e.g., rabies and vesicular stomatitis virus), paramyxovirus (e.g., measles and Sendai), positive strand RNA viruses, such as picornavirus and alphavirus, and double stranded DNA viruses including adenovirus, herpesvirus (e.g., Herpes Simplex virus types 1 and 2, Epstein-Barr virus, cytomegalovirus, replication deficient herpes virus), and poxvirus (e.g., vaccinia, modified vaccinia Ankara (MVA), fowlpox and canarypox). Additional non-limiting examples include Norwalk virus, togavirus, flavivirus, reoviruses, papovavirus, hepadnavirus, human papilloma virus, human foamy virus, and hepatitis virus, for example. Non-limiting examples of retroviruses include: avian leukosis-sarcoma, avian C-type viruses, mammalian C-type, B-type viruses, D-type viruses, oncoretroviruses, HTLV-BLV group, lentivirus, alpharetrovirus, gammaretrovirus, spumavirus *(see,* e.g., Coffin JM. Retroviridae: The viruses and their replication. In: Fields BN, Knipe DM, Howley PM et al, eds. Fundamental Virology. 3rd ed. Philadelphia: Lippincott-Raven Publishers, 1996:763-843). Additional non-limiting examples include murine leukemia viruses, murine sarcoma viruses, mouse mammary tumor virus, bovine leukemia virus, feline leukemia virus, feline sarcoma virus, avian leukemia virus, human T-cell leukemia virus, baboon endogenous virus, Gibbon ape leukemia virus, Mason Pfizer monkey virus, simian immunodeficiency virus, simian sarcoma virus, Rous sarcoma virus and lentiviruses. Additional non-limiting examples of vectors are described, for example, in US Patent No. 5,801,030, the teachings of which are incorporated herein by reference.

In some embodiments, the agent or pharmaceutical composition of the disclosure is formulated to be delivered by a membrane-based carrier *in vivo, in vitro, ex vivo,* or *in situ.* In some embodiments, the membrane-based carrier is a cell-based carrier (e.g., mammalian such as human cells). In some embodiments, the membrane-based carrier is a vesicle-based carrier. In some embodiments, the membrane-based carrier comprises one or more vectors described herein (e.g., a plasmid, virus, viral-like particle or a virosome).

In some embodiments, the agent or pharmaceutical composition of the disclosure is formulated to be delivered by one or more liposomes. Liposomes are spherical vesicle structures composed of a uni- or multilamellar lipid bilayer surrounding internal aqueous compartments and a relatively impermeable outer lipophilic phospholipid bilayer. Liposomes may be anionic, neutral or cationic. Liposomes are biocompatible, nontoxic, can deliver both hydrophilic and lipophilic drug molecules, protect their cargo from degradation by plasma enzymes, and transport their load across biological membranes and the blood brain barrier (BBB) *(see,* e.g., Spuch and Navarro, J Drug Deliv. 2011: 469679 (2011)).

Vesicles can be made from several different types of lipids; however, phospholipids are most commonly used to generate liposomes as drug carriers. Methods for preparation of multilamellar vesicle lipids are known in the art (see for example U.S. Pat. No. 6,693,086, the teachings of which relating to multilamellar vesicle lipid preparation are incorporated herein by reference). Although vesicle formation can be spontaneous when a lipid film is mixed with an aqueous solution, it can also be expedited by applying force in the form of shaking by using a homogenizer, sonicator, or an extrusion apparatus *(see,* e.g., Spuch and Navarro, J Drug Deliv. 2011: 469679 (2011)). Extruded lipids can be prepared by extruding through filters of decreasing size, as described in Templeton et al., Nature Biotech, 15: 647-52 (1997), the teachings of which relating to extruded lipid preparation are incorporated herein by reference.

In some embodiments, the agent or pharmaceutical composition of the disclosure is formulated to be delivered by lipid nanoparticles (LNPs). In one embodiment, the LNP preparation comprising the agent or pharmaceutical composition of the disclosure has one or more of the following characteristics: (a) the LNP preparation comprises a cationic lipid, a neutral lipid, a cholesterol, and a PEG lipid, (b) the LNP preparation has a mean particle size of between 80 nm and 160 nm.

Nanostructured lipid carriers (NLCs) are modified solid lipid nanoparticles (SLNs) that retain the characteristics of the SLN, improve drug stability and loading capacity, and prevent drug leakage. Polymer nanoparticles (PNPs) are an important component of drug delivery. These nanoparticles can effectively direct drug delivery to specific targets and improve drug stability and controlled drug release. Lipid-polymer nanoparticles (PLNs), a new type of carrier that combines liposomes and polymers, may also be employed. These nanoparticles possess the complementary advantages of PNPs and liposomes. A PLN is composed of a core-shell structure; the polymer core provides a stable structure, and the phospholipid shell offers good biocompatibility. As such, the two components increase the drug encapsulation efficiency rate, facilitate surface modification, and prevent leakage of water-soluble drugs. *See,* e.g., Li et al., Nanomaterials 7(6): 122 (2017).

In some embodiments, the agent or pharmaceutical composition of the disclosure is formulated to be delivered by a carbohydrate carrier (e.g., an anhydride- modified phytoglycogen or glycogen-type material). Non-limiting examples of carbohydrate carriers include phytoglycogen octenyl succinate, phytoglycogen beta-dextrin and anhydride-modified phytoglycogen beta-dextrin.

In some embodiments, the agent or pharmaceutical composition of the disclosure is formulated to be delivered by a protein carrier (e.g., a protein covalently linked to the circular polyribonucleotide). Non-limiting examples of protein carriers include human serum albumin (HSA), low-density lipoprotein (LDL), high- density lipoprotein (HDL) and globulin.

In some embodiments, the agent or pharmaceutical composition of the disclosure is formulated to be delivered by a cationic carrier (e.g., a cationic lipopolymer or transfection reagent). Non-limiting examples of cationic carriers include lipofectamine, polyethylenimine, poly(trimethylenimine), poly(tetramethylenimine), polypropylenimine, aminoglycoside-polyamine, dideoxy-diamino-b-cyclodextrin, spermine, spermidine, poly(2-dimethylamino)ethyl methacrylate, poly(lysine), poly(histidine), poly(arginine), cationized gelatin, dendrimers, chitosan, 1,2-Dioleoyl-3- Trimethylammonium-Propane(DOTAP), N-[1-(2,3-dioleoyloxy) propyl]-N,N,N- trimethylammonium chloride (DOTMA), 1-[2-(oleoyloxy)ethyl]-2-oleyl-3-(2- hydroxyethyl)imidazolinium chloride (DOTIM), 2,3-dioleyloxy-N-[2(sperminecarboxamido) ethyl]-N,N-dimethyl-l-propanaminium trifluoroacetate (DOSPA), 3B-[N- (N\N'-Dimethylaminoethane)-carbamoyl]Cholesterol Hydrochloride (DC-Cholesterol HC1), diheptadecylamidoglycyl spermidine (DOGS), N,N-distearyl-N,N- dimethylammonium bromide (DDAB), N-(l,2-dimyristyloxyprop-3-yl)-N,N-dimethyl-N- hydroxyethyl ammonium bromide (DMRIE) and N,N-dioleyl-N,N-dimethylammonium chloride (DODAC).

In some embodiments, the agent or pharmaceutical composition of the disclosure is formulated to be delivered by exosomes, adipocytes and/or red blood cells. *See,* e.g., Ha et al., Acta Pharm Sin B. 6(4): 287-96 (2016).

In some embodiments, the agent or pharmaceutical composition of the disclosure is formulated to be delivered by one or more Fusosomes. Fusosomes have been engineered to confer target cell specificity for the fusion and payload delivery, allowing the creation of delivery vehicles with programmable cell specificity. See, e.g., Patent Application WO2020014209, the teachings of which relating to fusosome design, preparation, and usage are incorporated herein by reference.

In some embodiments, the agent or pharmaceutical composition of the disclosure is formulated to be delivered by *ex vivo* differentiated red blood cells. See, e.g., WO2015073587; WO2017123646; WO2017123644; WO2018102740; WO2016183482; WO2015153102; WO2018151829; WO2018009838; Shi et al., PNAS, 111(28): 10131-36 (2014); US Patent 9,644,180; Huang et al., Nature Communications 8: 423 (2017).

In some embodiments, the agent or pharmaceutical composition of the disclosure is formulated to be delivered by one or more microsomes, virus-like particles (VLPs) or plant nanovesicles and plant messenger packs (PMPs). *See,* e.g., WO2011097480, WO2013070324, WO2017004526 and WO2020041784.

In some embodiments, the agent or pharmaceutical composition of the disclosure is formulated to be delivered by one or more anellosomes. The making and use of anellosomes used for delivery of therapeutic products is described in US Pat. 11,166,996, the teachings of which relating to anellosome design, preparation, and usage are incorporated herein by reference.

### Methods of Modulating Target Proteins

In another aspect, the disclosure provides a method of modulating the expression or activity of a target protein identified in the Sequence Listing, or a variant thereof in a cell (a target cell, a cell of a target tissue), comprising contacting the cell (e.g., *in vitro, ex vivo,* or *in vivo)* with an agent that comprises and/or modulates the expression or activity of a target protein identified herein, or a pharmaceutical composition comprising the agent.

In some embodiments, the target cell is a cancer cell (e.g., a metastatic cancer cell), a cell (e.g., stromal cell) in a tumor micro-environment, or a combination thereof. In particular embodiments, the target cell is a metastatic cancer cell.

In some embodiments, the target cell is implicated and/or involved in inflammation. In certain embodiments, the target cell is an epithelial cell, an endothelial cell, a stem cell, a non-immune cell, or a combination thereof.

In some embodiments, the target cell is implicated and/or involved in fibrosis, aging, and/or senescence. In certain embodiments, the target cell is an epithelial cell, an endothelial cell, a stem cell, a non-immune cell, or a combination thereof.

In some embodiments, the target cell is an immune cell. In certain embodiments, the target cell is an effector T cell, a helper T cell, a Th1 cell, a Th2 cell, a Th17 cell, a B cell, a natural killer (NK) cell, an innate lymphoid cell (e.g., an ILC1 cell, an ILC2 cell, an ILC3 cell), a macrophage (e.g., an M1 macrophage, an M2 macrophage), a monocyte, and/or an antigen presenting cell (e.g., a dendritic cell), or a combination of the foregoing.

In some embodiments, a target protein of the disclosure is used to mediate depletion of a cell population (e.g., a population of cancer cells, such as tumor cells; a population of immune cells). In some embodiments, a target protein of the disclosure facilitates cell targeting (e.g., delivering a therapeutic in a cell type-specific manner), for example, as a binder of a surface marker.

The target tissue may be any tissue of the body.

In certain embodiments, the target tissue comprises a tumor, a tumor microenvironment, a site of metastasis, or a combination thereof.

In some embodiments, the target tissue is an immune tissue. In some embodiments, the target cell is a non-immune tissue. In some embodiments, the target tissue comprises a lymph node, a spleen, a secondary lymphoid organ, a tertiary lymphoid organ, a barrier tissue, skin, gut, an airway, a wound, an immune tissue, a non-immune tissue, or a combination of the foregoing.

In certain embodiments, the effective amount is sufficient to reduce expression of the target protein in the target cell and/or target tissue. In some embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In certain embodiments, the effective amount is sufficient to increase expression of the target protein in the target cell and/or target tissue. In some embodiments, said increase is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said increase is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%. In some embodiments, said increase is by about 1-100 fold, e.g., by about: 1-75, 1-50, 1-25, 1-20, 1-15, 1-10, 1-8, 1-6, 1-5, 1-4, 1-3 or 1-2 fold.

In some embodiments, the effective amount is sufficient to modulate nuclear factor kappa B (NF-κB) signaling, growth-factor signaling, cell death (e.g., apoptosis), cell cycle (e.g., mitosis), cell migration, inflammation, or a combination of the foregoing. In some embodiments, the effective amount is sufficient to modulate a signaling pathway involving the Janus kinase (JAK)-signal transducer family (e.g., JAK1, JAK2, JAK3 and TYK2), a member of the signal transducer and activator of transcription (STAT) protein family (e.g., STAT1, STAT3), a member of the protein kinase B family (e.g., RAC-alpha, RAC-beta or RAC-gamma serine/threonine-protein kinase), a member of the interferon regulatory factor (IRF) family, a mitogen-activated protein kinase (MAPK), or a combination of the foregoing.

### Methods of Diagnosis and Treatment

In another aspect, the disclosure provides a method of detecting a disease or condition, or predicting a likelihood of (or risk level for) developing a disease or condition in a subject, comprising quantifying an expression or activity of a target protein in a sample from the subject, wherein the level of expression or activity of the target protein in the sample is indicative of the likelihood of developing the disease or condition in the subject, wherein the disease or condition is selected from aging, senescence, fibrosis, autoimmunity, cancer (e.g., tumor), an infection, an immunological disease (e.g., inflammation and/or an autoimmune disease), or a combination thereof.

In another aspect, the disclosure provides a method of classifying a subject based on a predicted likelihood of developing a disease or condition, comprising quantifying an expression or activity of a target protein in a sample from the subject; predicting the likelihood of developing the disease or condition based on the expression or activity of the target protein in the sample; and classifying the patient based on the predicted likelihood, wherein the disease or condition is selected from aging, senescence, fibrosis, autoimmunity, cancer (e.g., tumor), an infection, an immunological disease (e.g., inflammation and/or an autoimmune disease), or a combination thereof.

In another aspect, the disclosure provides a method of stratifying a set of subjects having a disease or condition, comprising: quantifying an expression and/or activity of a target protein in samples from individual subjects in the set; and stratifying the set of subjects for treatment according to the individual subjects' levels of the expression and/or activity of the target protein in the samples, wherein the disease or condition is selected from aging, senescence, fibrosis, autoimmunity, cancer (e.g., tumor), an infection, an immunological disease (e.g., inflammation and/or an autoimmune disease), or a combination thereof.

In some embodiments, a higher expression or activity level of a target protein in a sample from the subject, relative to an appropriate control (e.g., reference standard) is indicative of the disease or condition or a likelihood of developing the disease or condition. In some embodiments, a lower expression or activity level of a target protein in a sample from the subject, relative to an appropriate control (e.g., reference standard) is indicative of the disease or condition or a likelihood of developing the disease or condition.

In some embodiments, the method further comprises administering to a subject who is determined to have or predicted to have a likelihood (or be at risk) of developing the disease or condition, an effective amount of an agent disclosed herein or a pharmaceutical composition disclosed herein.

In some embodiments, the method further comprises administering to a subject who is determined to have or predicted to have a likelihood of developing the disease or condition, an effective amount of an agent disclosed herein or a pharmaceutical composition disclosed herein.

In another aspect, the disclosure provides a method of preparing a sample that is useful for detecting a likelihood of developing the disease or condition in a subject, comprising:
a) obtaining or having obtained a sample from the subject;
b) adding a protease inhibitor, a control peptide, a standard peptide, or a combination thereof to the sample to prepare a sample that is useful for detecting a likelihood of developing cancer;
c) quantifying an expression or activity of a target protein in the sample prepared in step b); and
d) wherein the disease or condition is selected from aging, senescence, fibrosis, autoimmunity, cancer (e.g., tumor), an infection, an immunological disease (e.g., inflammation and/or an autoimmune disease), or a combination thereof.

In another aspect, the disclosure provides a method of treating a disease or condition in a subject in need thereof, comprising administering to the subject an effective amount of an agent disclosed herein or a pharmaceutical composition disclosed herein.

In another aspect, the disclosure provides a method of treating a disease or condition in a subject in need thereof, comprising administering to the subject an effective amount of an agent disclosed herein or a pharmaceutical composition disclosed herein, wherein the subject has an altered level of expression and/or activity of a target protein disclosed herein.

"Treatment" and "treating," as used herein, refer to the medical management of a subject with the intent to improve, ameliorate, stabilize (i.e., not worsen), prevent or cure a disease, pathological condition, or disorder. "Treatment" includes active treatment (treatment directed to improve the disease, pathological condition, or disorder), causal treatment (treatment directed to the cause of the associated disease, pathological condition, or disorder), palliative treatment (treatment designed for the relief of symptoms), preventative treatment (treatment directed to minimizing or partially or completely inhibiting the development of the associated disease, pathological condition, or disorder); and supportive treatment (treatment employed to supplement another therapy). Treatment also includes diminishing the extent of the disease or condition; preventing spread of the disease or condition; delaying or slowing the progress of the disease or condition; ameliorating or palliating the disease or condition; and remission (whether partial or total), whether detectable or undetectable. "Ameliorating" or "palliating" a disease or condition means that the extent and/or undesirable clinical manifestations of the disease, disorder, or condition are lessened and/or time course of the progression is slowed or lengthened, as compared to the extent or time course in the absence of treatment. "Treatment" also includes prolonging survival as compared to expected survival if not receiving treatment. Those in need of treatment include those already with the condition or disorder, as well as those prone to have the condition or disorder or those in which the condition or disorder is to be prevented.

In some embodiments, the subject is an animal. In other embodiments the subject is a bird, e.g., a hen, rooster, turkey or parrot. In some embodiments, the subject is a mammal. In some embodiments the subject is a non-human mammal. Non-limiting examples of a non-human mammal include cattle (e.g., dairy or beef cattle), sheep, goat, pig, horse, dog, cat, mouse, rat, etc. In some embodiments, the subject is a human. In some embodiments, the human is a neonate. In some embodiments, the human is a pediatric patient. In some embodiments, the human is a juvenile. In some embodiments, the human is an adult. In some embodiments, the human is less than 18 years old. In some embodiments, the human is at least 18 years old. In some embodiments, the human is between 18 and 25 years old. In some embodiments, the human is at least 25 years old, e.g., at least 30, 35, 40, 45, 50, 55, 60, 65, 70, 75, or 80 years old.

As used herein, the term "effective amount," "therapeutically effective amount," or "sufficient amount" refers to a quantity sufficient to, when administered to a subject (e.g., a mammal such as a human cancer patient), effect treatment (e.g., produce beneficial or desired results), including effects at cellular, tissue or clinical levels, etc. As such, the term depends upon the context in which it is being applied. For example, in the context of treating cancer, it is an amount of an agent sufficient to achieve a response as compared to the response obtained without administration of the agent. The amount of a given composition described herein that will correspond to such an amount will vary depending upon various factors, such as the given agent, the pharmaceutical formulation, the route of administration, the type of disease or disorder, the identity of the subject (e.g., age, sex, weight) or host being treated, and the like, but can nevertheless be routinely determined by one skilled in the art. In some embodiments, a "therapeutically effective amount" of a composition of the present disclosure is an amount that results in a beneficial or desired result in a subject (e.g., as compared to a control). A therapeutically effective amount of a composition of the present disclosure may be readily determined by one of ordinary skill by routine methods known in the art. Dosage regimen may be adjusted to provide the optimum therapeutic response.

A therapeutic agent described herein can be administered via a variety of routes of administration, including, for example, oral, dietary, topical, transdermal, rectal, parenteral (*e.g.,* intra-arterial, intravenous, intramuscular, subcutaneous injection, intradermal injection), intravenous infusion and inhalation (*e.g.,* intrabronchial, intranasal or oral inhalation, intranasal drops) routes of administration, depending on the compound and the particular disease or condition to be treated. Administration can be local or systemic as indicated. The preferred mode of administration can vary depending on the particular compound chosen.

In some embodiments, the method further comprises administering a therapeutically effective amount of one or more additional therapeutic agents (e.g., a second therapeutic agent) to the subject.

Administration of two or more therapeutic agents encompasses co-administration of the therapeutic agents in a substantially simultaneous manner, such as in a pharmaceutical combination. Alternatively, such administration encompasses co-administration in multiple containers, or separate containers (*e.g.,* capsules, powders, and liquids) for each therapeutic agent. Such administration also encompasses use of the therapeutic agents in a sequential manner, either at approximately the same time or at different times. When two or more therapeutic agents are administered, the therapeutic agents can be administered via the same administration route or via different administration routes.

In another aspect, the disclosure provides a method of modulating the expression or activity of a target protein identified in the Sequence Listing, or a variant thereof in a cell, comprising contacting the cell with an agent disclosed herein or a pharmaceutical composition disclosed herein. In some embodiments, the cell is in a subject.

In another aspect, the disclosure provides a method of identifying an agent that modulates the expression and/or activity of a target protein (e.g., a target protein in the Sequence Listing), comprising:
a) contacting a sample (e.g., a biological sample, such as cells or a tissue) comprising the target protein with an agent (e.g., a candidate agent to be tested for its ability to modulate expression and/or activity of the target); and
b) determining whether the agent modulates the expression or activity of the target protein,
wherein a difference in the expression or activity of the target protein that has been contacted with the agent compared to a reference indicates that the agent modulates the expression or activity of the target protein.

In some embodiments, a difference of at least about 10% in the expression or activity of the protein that has been contacted with the agent compared to the reference indicates that the agent modulates the expression or activity of the protein. In some embodiments, the difference is at least about: 15%, 20%, 25%, 30%, 35%, 40%, 45% or 50% or more.

In some embodiments, a decrease in the expression or activity of the target protein that has been contacted with the agent compared to the reference indicates the agent inhibits the expression or activity of the target protein. In some embodiments, an increase in the expression or activity of the protein compared to the reference indicates the agent activates the expression or activity of the protein.

### Indications

### Cancer

A wide variety of cancers is treatable according to the methods described herein. In some embodiments, the cancer comprises a solid tumor (*e.g.,* a tumor of the breast, lung, prostate, colon, bladder, ovary, kidney, stomach, colon, rectum, testes, head and/or neck, pancreas, brain, skin). Accordingly, in some embodiments, the cancer is a solid tumor cancer. Solid tumor cancers that can be treated according to the methods described herein include breast cancer, lung cancer, prostate cancer, colon cancer, bladder cancer, ovarian cancer, renal cancer, gastric cancer, colon cancer, rectal cancer, colorectal cancer, testicular cancer, head and neck cancer, pancreatic cancer, brain cancer and skin cancer. In some embodiments, the cancer is a hematologic cancer (*e.g.,* leukemia, lymphoma, myeloma). Hematologic cancers that can be treated according to the methods described herein include leukemias (*e.g.,* acute leukemias, chronic leukemias), lymphomas (*e.g.,* B-cell lymphoma, T-cell lymphoma) and multiple myeloma.

Examples of cancers treatable according to the methods described herein include Acute Lymphoblastic Leukemia (ALL); Acute Myeloid Leukemia (AML); Adrenocortical Carcinoma; Adrenocortical Carcinoma, Childhood; AIDS-Related Cancer (*e.g.,* Kaposi Sarcoma, AIDS-Related Lymphoma, Primary CNS Lymphoma); Anal Cancer; Appendix Cancer; Astrocytomas, Childhood; Atypical Teratoid/Rhabdoid Tumor, Childhood, Central Nervous System; Basal Cell Carcinoma of the Skin; Bile Duct Cancer; Bladder Cancer; Bladder Cancer, Childhood; Bone Cancer (including Ewing Sarcoma, Osteosarcoma and Malignant Fibrous Histiocytoma); Brain Tumors/Cancer; Breast Cancer; Burkitt Lymphoma; Carcinoid Tumor (Gastrointestinal); Carcinoid Tumor, Childhood; Cardiac (Heart) Tumors, Childhood; Embryonal Tumors, Childhood; Germ Cell Tumor, Childhood; Primary CNS Lymphoma; Cervical Cancer; Childhood Cervical Cancer; Cholangiocarcinoma; Chordoma, Childhood; Chronic Lymphocytic Leukemia (CLL); Chronic Myelogenous Leukemia (CML); Chronic Myeloproliferative Neoplasms; Colorectal Cancer; Childhood Colorectal Cancer; Craniopharyngioma, Childhood; Cutaneous T-Cell Lymphoma (*e.g.,* Mycosis Fungoides and Sézary Syndrome); Ductal Carcinoma In Situ (DCIS); Embryonal Tumors, Central Nervous System, Childhood; Endometrial Cancer (Uterine Cancer); Ependymoma, Childhood; Esophageal Cancer; Childhood Esophageal Cancer; Esthesioneuroblastoma; Ewing Sarcoma; Extracranial Germ Cell Tumor, Childhood; Extragonadal Germ Cell Tumor; Eye (Ocular) Cancer; Childhood Intraocular Melanoma; Intraocular Melanoma; Retinoblastoma; Fallopian Tube Cancer; Fibrous Histiocytoma of Bone, Malignant, and Osteosarcoma; Gallbladder Cancer; Gastric (Stomach) Cancer; Childhood Gastric (Stomach) Cancer; Gastrointestinal Carcinoid Tumor; Gastrointestinal Stromal Tumors (GIST); Childhood Gastrointestinal Stromal Tumors; Germ Cell Tumors; Childhood Central Nervous System Germ Cell Tumors (*e.g.,* Childhood Extracranial Germ Cell Tumors, Extragonadal Germ Cell Tumors, Ovarian Germ Cell Tumors, Testicular Cancer); Gestational Trophoblastic Disease; Hairy Cell Leukemia; Head and Neck Cancer; Heart Tumors, Childhood; Hepatocellular (Liver) Cancer; Histiocytosis, Langerhans Cell; Hodgkin Lymphoma; Hypopharyngeal Cancer; Intraocular Melanoma; Childhood Intraocular Melanoma; Islet Cell Tumors, Pancreatic Neuroendocrine Tumors; Kaposi Sarcoma; Kidney (Renal Cell) Cancer; Langerhans Cell Histiocytosis; Laryngeal Cancer; Leukemia; Lip and Oral Cavity Cancer; Liver Cancer; Lung Cancer (Non-Small Cell and Small Cell); Childhood Lung Cancer; Lymphoma; Male Breast Cancer; Malignant Fibrous Histiocytoma of Bone and Osteosarcoma; Melanoma; Childhood Melanoma; Melanoma, Intraocular (Eye); Childhood Intraocular Melanoma; Merkel Cell Carcinoma; Mesothelioma, Malignant; Childhood Mesothelioma; Metastatic Cancer; Metastatic Squamous Neck Cancer with Occult Primary; Midline Tract Carcinoma With NUT Gene Changes; Mouth Cancer; Multiple Endocrine Neoplasia Syndromes; Multiple Myeloma/Plasma Cell Neoplasms; Mycosis Fungoides; Myelodysplastic Syndromes, Myelodysplastic/Myeloproliferative Neoplasms; Myelogenous Leukemia, Chronic (CML); Myeloid Leukemia, Acute (AML); Myeloproliferative Neoplasms, Chronic; Nasal Cavity and Paranasal Sinus Cancer; Nasopharyngeal Cancer; Neuroblastoma; Non-Hodgkin Lymphoma; Non-Small Cell Lung Cancer; Oral Cancer, Lip and Oral Cavity Cancer and Oropharyngeal Cancer; Osteosarcoma and Malignant Fibrous Histiocytoma of Bone; Ovarian Cancer; Childhood Ovarian Cancer; Pancreatic Cancer; Childhood Pancreatic Cancer; Pancreatic Neuroendocrine Tumors; Papillomatosis (Childhood Laryngeal); Paraganglioma; Childhood Paraganglioma; Paranasal Sinus and Nasal Cavity Cancer; Parathyroid Cancer; Penile Cancer; Pharyngeal Cancer; Pheochromocytoma; Childhood Pheochromocytoma; Pituitary Tumor; Plasma Cell Neoplasm/Multiple Myeloma; Pleuropulmonary Blastoma; Pregnancy and Breast Cancer; Primary Central Nervous System (CNS) Lymphoma; Primary Peritoneal Cancer; Prostate Cancer; Rectal Cancer; Recurrent Cancer; Renal Cell (Kidney) Cancer; Retinoblastoma; Rhabdomyosarcoma, Childhood; Salivary Gland Cancer; Sarcoma (*e.g.,* Childhood Rhabdomyosarcoma, Childhood Vascular Tumors, Ewing Sarcoma, Kaposi Sarcoma, Osteosarcoma (Bone Cancer), Soft Tissue Sarcoma, Uterine Sarcoma); Sézary Syndrome; Skin Cancer; Childhood Skin Cancer; Small Cell Lung Cancer; Small Intestine Cancer; Soft Tissue Sarcoma; Squamous Cell Carcinoma of the Skin; Squamous Neck Cancer with Occult Primary, Metastatic; Stomach (Gastric) Cancer; Childhood Stomach (Gastric) Cancer; T-Cell Lymphoma, Cutaneous (*e.g.,* Mycosis Fungoides and Sèzary Syndrome); Testicular Cancer; Childhood Testicular Cancer; Throat Cancer (*e.g.,* Nasopharyngeal Cancer, Oropharyngeal Cancer, Hypopharyngeal Cancer); Thymoma and Thymic Carcinoma; Thyroid Cancer; Transitional Cell Cancer of the Renal Pelvis and Ureter; Ureter and Renal Pelvis, Transitional Cell Cancer; Urethral Cancer; Uterine Cancer, Endometrial; Uterine Sarcoma; Vaginal Cancer; Childhood Vaginal Cancer; Vascular Tumors; Vulvar Cancer; and Wilms Tumor and Other Childhood Kidney Tumors.

Metastases of the aforementioned cancers can also be treated in accordance with the methods described herein. In some embodiments, the cancer is a metastatic cancer.

In some embodiments, the cancer is selected from lung cancer, breast cancer, Hodgkin's lymphoma, diffuse large B-cell lymphoma, follicular lymphoma, primary central nervous system lymphoma, chronic lymphocytic leukemia, epithelial ovarian cancer, prostate cancer, squamous cell carcinoma, non-melanoma skin cancer, nasal polyps, basal cell carcinoma, keratinocyte cancer, multiple myeloma, serous invasive ovarian cancer, hepatocellular carcinoma, small cell lung carcinoma, adenocarcinoma, lung adenocarcinoma, non-small cell lung cancer, ovarian cancer or colorectal cancer.

In some embodiments, the treatment:
a) inhibits cancer cell growth, proliferation, metastasis, invasion or migration, or a combination of the foregoing;
b) promotes cancer cell death;
c) induces cancer cell autophagy,
or a combination of the foregoing.

In some embodiments, the effective amount is sufficient to:
a) inhibit cancer cell growth, proliferation, metastasis, invasion or migration, or a combination of the foregoing;
b) promote cancer cell death;
c) induce cancer cell autophagy,
d) or a combination of the foregoing.

In some embodiments, the effective amount is sufficient to reduce cancer (e.g., tumor) growth, proliferation, metastasis, invasion, migration, autophagy or a combination of the foregoing. In certain embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to reduce cancer cell proliferation or tumor growth in the subject. In some embodiments, the reduction in cancer cell proliferation or tumor growth is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In some embodiments, the reduction in cancer cell proliferation or tumor growth is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to modulate (e.g., increase or reduce) tumor autophagy, for example, by increasing at least one tumor-inhibiting function of autophagy and/or reducing at least one tumor-promoting function of autophagy.

In some embodiments, the effective amount is sufficient to increase cancer autophagy. In certain embodiments, said increase is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said increase is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to reduce cancer autophagy. In certain embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to prevent death of a subject thereby reducing the cancer (e.g., tumor) death rate. In certain embodiments, the reduction in cancer (e.g., tumor) death rate is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, the reduction in cancer (e.g., tumor) death rate is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to modulate (e.g., increases or reduce) expression of the target protein in a cancer (e.g., tumor) cell.

In some embodiments, the effective amount is sufficient to modulate the body's response to cancer, for example, by inhibiting the growth of cancer, reducing malignancy of cancer, inhibiting metastasis of cancer, promoting remission, modulating (increasing and/or decreasing) immune-mediated responses related to cancer, or a combination of the foregoing.

### Immuno-Oncology

In some embodiments, the disclosure provides for treatments that leverage the immune system. In some embodiments, the methods relate to immuno-oncology (e.g., cancer immunotherapy). In still further embodiments, the immune system is the innate immune system. In some embodiments, the immune system is the adaptive immune system. In still further embodiments, the treatment relates to humoral immunity or antibody-mediated immunity. In some embodiments, the treatment relates to cell-mediated immunity, such as cancer. In some embodiments, the methods relate to treatment at or before early-stage disease progression, while in other embodiments, the methods relate to treatment at or prevention of late-stage disease progression. In still further embodiments, the immune-oncological effect results from stimulation of the immune system.

In some embodiments, the effective amount is sufficient to modulate (e.g., increase) the subject's immune system against cancer. In certain embodiments, the effective amount is sufficient to modulate (e.g., increase or decrease):
a) an immune cell-related readout, an immune-cell activation, degranulation, maturation, migration of an immune cell (e.g., a macrophage, a monocyte or a dendritic cell), polarization, proliferation and recruitment;
b) lymph-node activation, innervation, differentiation, egress, homing;
c) cytokine production;
d) antibody-dependent cellular cytotoxicity (ADCC) and/or antibody-dependent cellular phagocytosis (ADCP);
e) antigen presentation;
f) target protein expression,
g) or a combination of the foregoing.

In some embodiments, the modulation is an increase. In certain embodiments, said increase is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said increase is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the modulation is a reduction. In certain embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments (e.g., immuno-oncology specific treatment), the effective amount is sufficient to modulate (e.g., increase or decrease) an immune cell-related readout, migration of an immune cell (e.g., an antigen presenting cell (such as a dendritic cell and/or a macrophage) and/or a T cell), proliferation of an immune cell, recruitment of an immune cell (e.g., an antigen presenting cell (such as a dendritic cell and/or a macrophage), a monocyte, a T cell, and/or a B cell), lymph node homing of an immune cell (e.g., a dendritic cell and/or a T cell), lymph node egress of an immune cell (e.g., a dendritic cell and/or a T cell), differentiation of an immune cell, activation of an immune cell, polarization of an immune cell, cytokine production (e.g., increase pro-inflammatory cytokine, decrease pro-inflammatory cytokine, increase anti-inflammatory cytokine, decrease anti-inflammatory cytokine), degranulation of an immune cell, maturation of an immune cell, ADCC of an immune cell, ADCP of an immune cell, antigen presentation, tumor homing of an immune cell (e.g. a T cell); increase tumor egress of an immune cell (e.g., a regulatory T cell) decrease tumor egress of an immune cell (e.g., a CD8+ T cell), target protein expression, or a combination of the foregoing.

### Immunity/Inflammation

In some embodiments, the methods of the disclosure relate to immunity. In some embodiments, the immunity is related to bacteria, parasites, viruses, fungi, and/or cancer cells. In some embodiments, the immunity is autoimmune and is the result of the immune system attacking self-molecules. In some embodiments, the methods relate to treatment of immunological tolerance.

In some embodiments of the disclosure, the methods relate to treatment of inflammation. In some embodiments, the inflammation is acute or of relatively short duration, lasting minutes to hours. In still other embodiments, the inflammation is chronic or of longer duration, lasting weeks to months, and possibly years. In some embodiments, the methods disclosed herein relate to the treatment or prevention of conditions associated with inflammation. In certain embodiments, such conditions include but are not limited to flushed skin, pain or tenderness, swelling, heat, fatigue, fever, joint pain or stiffness, mouth sores, rashes. In some embodiments, inflammation is resultant from another disease or disorder.

In some embodiments, the disclosed methods relate to the treatment of autoimmune diseases. In some embodiments, the autoimmune disease may include but is not limited to disease of the joint and muscles (e.g., psoriatic arthritis, rheumatoid arthritis, Sjogren's syndrome, systemic lupus erythematosus), diseases of the digestive track (e.g., Crohn's disease, celiac disease, ulcerative colitis, inflammatory bowel diseases), diseases of the endocrine system (e.g., Graves' disease, Hashimoto's thyroiditis, Addison's disease), diseases of the skin (e.g., dermatomyositis, psoriasis, scleroderma), disease of the nervous system (e.g., chronic inflammatory demyelinating polyneuropathy, Guillain-Barre syndrome, multiple sclerosis), and other diseases (e.g., myasthenia gravis, autoimmune vasculitis, pernicious anemia, vasculitis, autoimmune lymphoproliferative syndrome, type 1 diabetes).

In some embodiments, the disease or condition is an inflammatory disease and/or an autoimmune disease. A wide variety of inflammatory and/or autoimmune diseases are treatable according to the methods described herein. In some embodiments, the inflammatory and/or autoimmune disease comprises Alzheimer's disease, asthma, endometriosis, inflammatory bowel disease (IBD) (e.g., Crohn's disease and ulcerative colitis), multiple sclerosis (MS), non-fatty liver disease (e.g., nonalcoholic fatty liver disease (NAFLD)), obesity, Parkinson's disease cancer, psoriasis, rheumatoid arthritis (RA), scleroderma, systemic lupus erythematosus (SLE), type 1 diabetes, type 2 diabetes, or a combination thereof.

In some embodiments, the target protein activates an immune response. In certain embodiments, the target protein inhibits an immune response. In some embodiments, the immune response is an innate immune response (e.g., a humoral and/or a cell-mediated immune response). In certain embodiments, the immune response is an adaptive immune response (e.g., a humoral and/or a cell-mediated immune response). Non-limiting examples of immune responses includes T-cell mediated immune responses (e.g., cytokine production and cellular cytotoxicity), B-cell mediated immune responses, humoral immune responses, and activation of cytokine responsive cells (e.g., macrophages).

In some embodiments, the target protein enhances a signal involved in T cell activation and/or survival. In certain embodiments, the target protein activates a stimulatory checkpoint molecule. Non-limiting examples of stimulatory checkpoint molecules include CD27, CD28, CD40, CD122, CD137, OX40, glucocorticoid-induced TNFR family related gene (GITR), inducible T-cell costimulator (ICOS). In particular embodiments, the target protein is an agonist to CD28.

In some embodiments, the target protein reduces a signal involved in T cell anergy and/or exhaustion. In certain embodiments, the target protein inhibits an inhibitory checkpoint molecule. Non-limiting examples of inhibitory checkpoint molecules include programmed cell death protein 1 (PD-1), PD-L1, PD-L2, T-cell immunoglobulin domain and mucin domain 3 (TIM-3), lymphocyte activation gene-3 (LAG-3), cytotoxic T-lymphocyte-associated protein 4 (CTLA-4), Adenosine A2A receptor (A2AR), B7-H3 (CD276), B7-H4 (VTCN1), B and T lymphocyte attenuator (BTLA), Indoleamine 2,3-dioxygenase (IDO), killer-cell immunoglobulin-like receptor (KIR), nicotinamide adenine dinucleotide phosphate NADPH oxidase isoform 2 (NOX2), V-domain Ig suppressor of T cell activation (VISTA), sialic acid-binding immunoglobulin-type lectin 7 (SIGLECs 7) and sialic acid-binding immunoglobulin-type lectin 9 (SIGLECs 9). In particular embodiments, the target protein is an inhibitor to PD-1.

In some embodiments, the effective amount is sufficient to modulate (e.g., increase or decrease):
a) development of high endothelial venules (HEVs) and/or tertiary lymphoid organs (TLOs);
b) immune-cell activation, degranulation, differentiation, maturation, migration of an immune cell (e.g., a macrophage, a monocyte or a dendritic cell), polarization, proliferation, and/or recruitment;
c) immune-cell lymph-node egress and/or homing;
d) immune-cell tumor egress and/or homing;
e) cytokine production;
f) antigen presentation;
g) target protein expression; or
h) auto-antibody levels,
or a combination thereof.

In some embodiments, the effective amount is sufficient to increase:
a) development of HEVs and/or TLOs;
b) immune-cell activation, degranulation, differentiation, maturation, migration of an immune cell (e.g., a macrophage, a monocyte or a dendritic cell), polarization, proliferation, and/or recruitment;
c) immune-cell lymph-node egress and/or homing;
d) immune-cell tumor egress and/or homing;
e) cytokine production;
f) antigen presentation;
g) target protein expression; or
h) auto-antibody levels,
or a combination thereof.

In certain embodiments, said increase is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said increase is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to decrease:
a) development of HEVs and/or TLOs;
b) immune-cell activation, degranulation, differentiation, maturation, migration of an immune cell (e.g., a macrophage, a monocyte or a dendritic cell), polarization, proliferation, and/or recruitment;
c) immune-cell lymph-node egress and/or homing;
d) immune-cell tumor egress and/or homing;
e) cytokine production;
f) antigen presentation;
g) target protein expression; or
h) auto-antibody levels,
or a combination thereof.

In certain embodiments, said decrease is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said decrease is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to:
a) increase organ function;
b) modulate (e.g., increase or decrease) inflammation;
c) decrease the level of auto-antibody;
d) decrease rate and/or number of relapses and/or flare-ups;
e) decrease viral load; or
f) decrease (e.g., control) infection,
or a combination of the foregoing.

In some embodiments, the effective amount is sufficient to increase organ function, inflammation, or a combination thereof. In certain embodiments, said increase is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said increase is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to decrease inflammation, the level of auto-antibody, rate and/or number of relapses and/or flare-ups, viral load or infection, or a combination of the foregoing. In certain embodiments, said decrease is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said decrease is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the target protein activates an immune cell. In some embodiments, the target protein inhibits an immune cell. Immune cells are cells that play a role in the immune response. Immune cells are of hematopoietic origin, including lymphocytes (e.g., B cells and T cells), natural killer cells, and myeloid cells (e.g., basophils, eosinophils, granulocytes, macrophages, mast cells, and monocytes). The target protein can be expressed on a cancer cell (e.g., a metastatic cancer cell), in the tumor microenvironment (e.g., on a stromal cell), or on a non-malignant cell (e.g., an immune cell).

In some embodiments, the effective amount is sufficient to increase an immune response. In certain embodiments, said increase is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said increase is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to reduce an immune response. In certain embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to reduce an inflammatory response. In certain embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to reduce autoimmunity. In certain embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In certain embodiments, the effective amount is sufficient to modulate expression of the target protein in an immune cell.

In some embodiments (e.g., immune and/or inflammation treatment), the effective amount is sufficient to modulate (e.g., increase or decrease) migration of an immune cell (e.g., an antigen presenting cell (such as a dendritic cell and/or a macrophage) and/or a T cell), proliferation of an immune cell, recruitment of an immune cell (e.g., an antigen presenting cell (such as a dendritic cell and/or a macrophage), a monocyte, a T cell, and/or a B cell), lymph node homing of an immune cell (e.g., a dendritic cell and/or a T cell), lymph node egress of an immune cell (e.g., a dendritic cell and/or a T cell), differentiation of an immune cell, activation of an immune cell, polarization of an immune cell, cytokine production (e.g., increase pro-inflammatory cytokine, decrease pro-inflammatory cytokine, increase anti-inflammatory cytokine, decrease anti-inflammatory cytokine), degranulation of an immune cell, maturation of an immune cell, antigen presentation, target protein expression, inflammation, an auto-antibody level; to increase an organ function; to decrease the rate and/or number of relapses or flare-ups, viral load; to control infection or a combination of the foregoing.

### Aging

The methods described herein are applicable to the treatment of aging, age-related conditions, and/or age-related disorders or diseases. In some embodiments, the methods described herein include treatment of general health, body temperature, body weight, body height, waist circumference, reproductive ability, body fat, heart rate, blood pressure, pulse rate, blood oxygen level, respiratory rate, breathing pattern, blood glucose level, blood pH, cardiac output, cardiac rhythm, as well as concentration of certain substances in the blood. In some embodiments, treatment may be assessed by measurement of complete blood count, red blood cells, white blood cells, platelets, hemoglobin, hematocrit, mean corpuscular volume, basic metabolic panel, blood glucose, calcium, electrolyte test, kidney function, blood enzyme tests, troponin, creatine kinase, lipoprotein panel, total cholesterol, LDL cholesterol, HDL cholesterol, triglycerides, coagulation panel, and/or bone marrow tests.

In some embodiments, the effective amount is sufficient to slow aging. In certain embodiments, said slowing is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said slowing is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

### Senescence

In some embodiments, the methods disclosed herein are for the treatment of senescence. In still further embodiments, the method disclosed herein relate the treatment of senescent associated diseases or disorders. In some embodiments, the disease or disorder includes but it not limited to diabetes, metabolic syndrome, and obesity. In other embodiments, the disease or disorder is related to photosensitivity or photoaging. In still further embodiments, the disease or disorder may be selected from arthritis, Alzheimer's disease, asthma, blindness, cancer, chronic bronchitis, chronic kidney disease, chronic obstructive pulmonary disease, coronary heart disease, deep vein thrombosis, dementia, depression, diabetes, epilepsy, heart failure, high cholesterol, hypertension, motor neuron disease, multiple sclerosis, osteoporosis, Paget's disease of bone, Parkinson's diseases, shingles, and stroke.

In some embodiments, the effective amount is sufficient to reduce senescence. In certain embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

### Fibrosis

In some embodiments, the methods of the disclosure relate to the treatment of fibrosis. In some embodiments, the methods relate to treatment of conditions related to or resultant from fibrosis. In some embodiments, the fibrosis is pulmonary fibrosis, liver fibrosis, skin fibrosis, renal fibrosis, pancreas fibrosis, systemic sclerosis, cardiac fibrosis, mediastinal fibrosis, bone marrow fibrosis, retroperitoneal cavity fibrosis, and/or macular degeneration.

In some embodiments, the effective amount is sufficient to reduce fibrosis. In certain embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

### Infections

In some embodiments, the disclosure relates to treatment of infections. In some aspects, the infection is a bacterial infection, a protozoan infection, a viral infection, a fungal infection, or another pathogenic infection. In some embodiments, the infection is AIDS or HIV, viral hepatitis (e.g. hepatitis A, hepatitis B, hepatitis C), tuberculosis, salmonella, Lyme disease, meningococcal disease, influenza, measles, mumps, rubella (e.g. German measles), pneumonia, a sexually transmitted disease (e.g. syphilis, chlamydia, gonorrhea), chronic sinusitis, whooping cough, pertussis, or a combination thereof.

Unless otherwise defined, all terms of art, notations and other scientific terms or terminology used herein are intended to have the meanings commonly understood by those of skill in the art to which this disclosure pertains. In some cases, terms with commonly understood meanings are defined herein for clarity and/or for ready reference, and the inclusion of such definitions herein should not necessarily be construed to represent a substantial difference over what is generally understood in the art. It will be further understood that terms, such as those defined in commonly-used dictionaries, should be interpreted as having a meaning that is consistent with their meaning in the context of the relevant art and/or as otherwise defined herein.

The terminology used herein is for the purpose of describing particular embodiments only and is not intended to be limiting.

As used herein, the indefinite articles "a," "an" and "the" should be understood to include plural reference unless the context clearly indicates otherwise.

Throughout this specification and the claims which follow, unless the context requires otherwise, the word "comprise," and variations such as "comprises" and "comprising", will be understood to imply the inclusion of, *e.g.,* a stated integer or step or group of integers or steps, but not the exclusion of any other integer or step or group of integer or step. When used herein, the term "comprising" can be substituted with the term "containing" or "including."

As used herein, "consisting of' excludes any element, step, or ingredient not specified in the claim element. When used herein, "consisting essentially of" does not exclude materials or steps that do not materially affect the basic and novel characteristics of the claim. Any of the terms "comprising," "containing," "including," and "having," whenever used herein in the context of an aspect or embodiment of the disclosure, can in some embodiments, be replaced with the term "consisting of," or "consisting essentially of" to vary scopes of the disclosure.

As used herein, the conjunctive term "and/or" between multiple recited elements is understood as encompassing both individual and combined options. For instance, where two elements are conjoined by "and/or," a first option refers to the applicability of the first element without the second. A second option refers to the applicability of the second element without the first. A third option refers to the applicability of the first and second elements together. Any one of these options is understood to fall within the meaning, and, therefore, satisfy the requirement of the term "and/or" as used herein. Concurrent applicability of more than one of the options is also understood to fall within the meaning, and, therefore, satisfy the requirement of the term "and/or."

When a list is presented, unless stated otherwise, it is to be understood that each individual element of that list, and every combination of that list, is a separate embodiment. For example, a list of embodiments presented as "A, B, or C" is to be interpreted as including the embodiments, "A," "B," "C," "A or B," "A or C," "B or C," or "A, B, or C."

### Exemplification

### Example 1: Validation of a target protein as a checkpoint by overexpression

This example demonstrates the ability of a target protein of the disclosure to act as a checkpoint immune regulator. In this example, human target proteins (including positive controls PD-1 and CTLA-4) cDNAs are cloned into the pLVX-TetOne vector (Clontech) under a doxycycline (dox) inducible TRE3G promoter. Human checkpoint ligands PD-L1 and PD-L2 cDNAs are cloned into the pLVX-IRES-Puro vector (Clontech) under expression of a constitutive CMV promoter. The T-cell line is built by infecting Jurkat cells with lentiviral vectors pLVX-TetOne-target protein. The B-cell line is built by infecting Raji cells with lentiviral vectors pLVX-IRES-Puro-target protein or pLVX-IRES-Puro-PD-L2. The anti-CD3 beads are made in-house by coating anti-Human CD3 antibodies from eBioscience to Dynabeads M-450 Epoxy beads from Invitrogen, using coating protocol from Invitrogen.

On day 1, target protein expression is induced in the Jurkat and Raji cell lines by adding dox up to 250 ng/mL concentration. 2 days post dox induction, the Jurkat and Raji cells are washed and diluted to 1x10⁶ cells in RPMI + 10% FBS. Anti-CD3 beads are added to the Jurkat cells and in parallel, control cells (dox negative) are used to assess the IL-2 production by non-suppressed T-cells. The Jurkat cells and beads [and antibodies] are incubated at room temperature for 30 min. 500 µl of the Raji cells are added to the Jurkat and beads. The final ratio is 500k Jurkat: 500k Raji: 2000k beads or 1: 1:4. Using a multichannel pipette, 200 µl of cell:bead mix is transferred into 96-well plate (U-bottom). This is kept in the incubator at 37°C for 22 hrs.

The next day, plates are centrifuged, and the supernatant is transferred to a new 96-well plate for IL-2 ELISA (R&D Systems #D2050). A decrease in detection of IL-2 is expected in T cells that are exhausted. This is how checkpoint targets proteins are selected.

### Example 2. Validation of a secreted target protein that stimulates or inhibits Cytokine

**release.** CBA (Cytometry) experiment looking at pro-inflammatory cytokines produced by PBMCs treated with secreted peptides.

This example demonstrates the validation of a Target Protein, SEQ ID NO: 33885, as an anti-inflammatory immune modulator, suitable for the treatment of autoimmune diseases such as Psoriasis, Arthritis, and Multiple Sclerosis.

Peripheral Blood Mononuclear Cells were seeded +/- the T-cell activator CD3/CD8 and +/- peptides for 24-48 hours. Cytokines were measured from the supernatant after incubation by CBA Cytometer.

### Materials and methods:

*Thawing PBMCs:* PBMCs were thawed by pre-warming TexMACS media (Miltenyi Biotech) in 20ml aliquots- 1-50ml conical per donor. After the media was warmed to 37°C, cells were removed from liquid nitrogen and brought to the tissue culture hood. For each donor, 1ml of media from the warmed aliquot was added to the frozen vial and mixed up and down. The media plus thawed cells were then added back to the 50ml conical. This was repeated until all the cells were thawed and added to the 50ml tube. Continue with other donors if used. Spin cells for 5min at 400xg. Supernatant was aspirated and cells resuspended at ~10*10^6cells/ml (see vial for cell number).

*PBMC Plating:* Frozen PBMCs from normal, healthy, human volunteers (StemCell Technologies) were thawed and plated at 250,000 cells/well. After PBMCs were thawed and resuspended at 10*0^6cells/ml, cells were then counted (Countess III, Thermo Scientific) and adjusted based on counts to 10*10^6cell/well. Take the number of PBMCs determined by number of wells for the experiment, for example, 10 wells would need 2.5*10^6 cells total or 0.250ml. The cells needed for the experiment are then transferred to another conical tube. Warmed TexMACS media is added so that the final concentration is 250,000 cells/190 ul media, for example,10 wells would need 1.9 ml (190ul x 10 wells) of media total minus 0.250 ml of cells (1.9ml - 0.250ml) or 1.65 ml of media added to the 0.250ml of cells. Add 190 ul of cells plus media to the 96 well U-bottom plate. Place in incubator until dilutions are made and ready.

*Peptide Dilutions:* Ideally, all peptides should be at a starting concentration of at least 10mM, especially for those in DMSO (ATCC) to reach a final concentration of 1 uM (H2O) or 0.5uM (DMSO). Dilutions of all test articles should be made at 20X the final concentration, for example, a final concentration of 1 uM peptide would need to be made at 20 uM.

*Reagent Dilution:* Dilutions are made in TexMACS media, enough for 10 ul/well in triplicate (30ul of a 20x). Activator - CD3/CD28 (TransAct; Miltenyi Biotech) or cytokine will be at 21X and added after the 45-minute incubation with peptides. Be sure to include relevant controls - Positive: TransAct + Cells, Negative: Vehicle + Cells. Dilutions should also be made in a ultra-low binding plate (Corning).

*Assay Procedure: Peptide Treatment:* Start assay by thawing the cells and plating in a 96 well, U-bottom tissue culture plate (Corning) as described above. Cell plates are placed in a tissue culture incubator (5% CO2, 37°C). While cells are in the incubator, start making dilutions (optimally 1hr or less) as described above. Cell plates are removed from the incubator and 10 ul of the 20X dilutions are added to the relevant wells. After the addition of inhibitors, cell plates are placed in the tissue culture incubator for 45 minutes. The plates are removed from the incubator and 10ul of 21x TransAct is added to the relevant wells*. Plates are then returned to the incubator and incubated for 24 hours. After 24 hours, plates are spun for 5 minutes at 400xg.

** Activation:* This assay can be run in two different formats - Activation and Inhibition. The procedure is the same except no TransAct is needed in the peptide wells for Activation. TransAct is only needed in the control wells. The purpose of this procedure is to determine the possible response from peptides alone.

*Cytometric Bead Array (CEA):* After the spin, supernatants (~150ul) are transferred to a new, ultra-low binding U-bottom plate. Supernatants are then frozen at -20°C until thawed for CBA (BD) to determine levels of human IL-2, TNFa, IL-1b, IP-10, IL-10, and IFNg. CBA was run according to the manufacturers protocol.

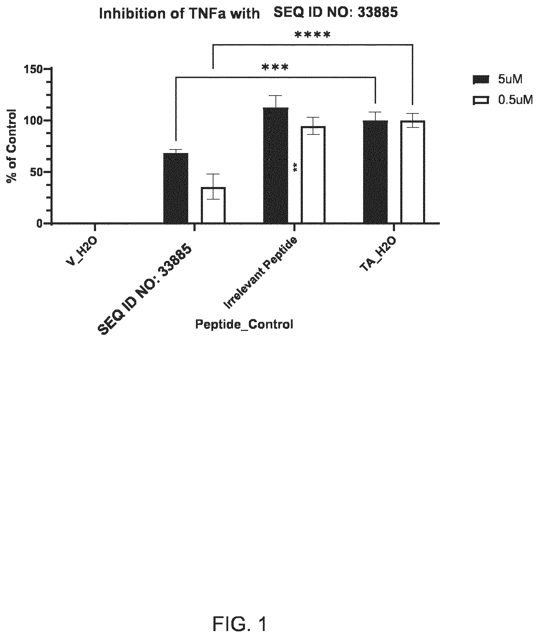

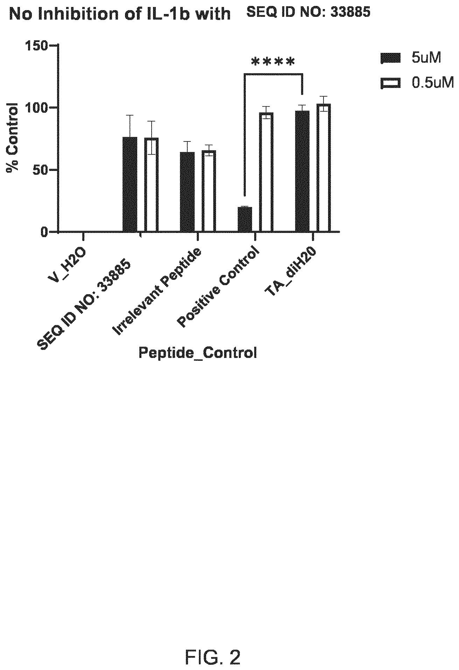

SEQ ID NO: 33885 significantly blocks the release of TNF-alpha from activated PBMCs but does not block the release of another inflammatory cytokine, IL-1b. Specific inhibition of TNF-alpha release will decrease the inflammatory response (FIG. 1 and FIG. 2).

**Table of Hits: X = a hit with significant activation or inhibition of relevant cytokine release. Inhibition occurs when the peptide is applied with anti-CD3/CD28 T-cell activator. Activation occurs when the peptide is applied to PBMCs alone (see Materials and Methods). Significance = P=0.05**

| | **Activation by Analyte** | | | | |
|---|---|---|---|---|---|
| | **IL-2** | **IL-1b** | **IP-10** | **IL-10** | **TNF** |
| SEQ ID NO: 35558 | | | X | X | |
| SEQ ID NO: 37047 | | | X | | |
| SEQ ID NO: 38042 | | | X | | |
| SEQ ID NO: 37375 | X | | | | |
| SEQ ID NO: 37477 | | X | | | |
| SEQ ID NO: 38393 | | | X | | |
| SEQ ID NO: 33741 | | | X | | |
| SEQ ID NO: 33169 | | | X | | |
| SEQ ID NO: 33678 | | | X | | |
| SEQ ID NO: 35480 | | | X | | |
| SEQ ID NO: 36902 | | | X | | |
| SEQ ID NO: 37192 | | | X | | |
| | | | | | |

| | **Inhibition by Analyte** | | | | |
|---|---|---|---|---|---|
| | **IL-2** | **TNF** | **IL-1b** | **IP-10** | **IL-10** |
| SEQ ID NO: 37394 | | | X | | |
| SEQ ID NO: 33934 | X | X | | | |
| SEQ ID NO: 37363 | | | X | | |
| SEQ ID NO: 35558 | | X | X | | |
| SEQ ID NO: 34837 | | X | X | X | |
| SEQ ID NO: 34837 | | | | | X |
| SEQ ID NO: 37043 | X | X | | | |
| SEQ ID NO: 33885 | X | X | X | | |
| SEQ ID NO: 33885 | X | X | X | X | |
| SEQ ID NO: 35373 | X | X | X | X | |

### Example 3. Validation of a target protein as a modulator of the innate immune response.

This example demonstrates the validation of a Target Protein, SEQ ID NO: 37413, as a modulator of the innate immune response. Specifically, the Target Protein inhibits multiple toll-like receptors from responding to their specific agonists. Thus, the target protein is suitable for the treatment of autoimmune diseases such as Lupus, Psoriasis, Arthritis, and Multiple Sclerosis. As a toll-like receptor (TLR) inhibitor, SEQ ID NO: 37413 will be especially useful for the treatment of lupus.

To determine the effect of the Target Protein on TLR activity, the Target Protein, irrelevant proteins and controls were applied +/- established TLR agonists to a commercial reporter cell line which monitors the two main signal transduction pathways that respond to TLR activation. To determine which immune cells are most responsive to the Target Proteins, the Target Proteins were applied to Peripheral Blood Mononuclear Cells (PBMCs) and then 24 hours later the PBMCs were subjected to single cell RNA-seq using cell hashing.

### Methods and materials:

*Dual Reporter Assay: From example:* NF-kB-SEAP and IRF-Lucia luciferase reporter monocyte (THP1) cells (InvivoGen) are cultured in RPMI 1640 media supplemented with HEPES buffer (10 m*M*), sodium pyruvate (1 m*M*), glucose (4.5 g/L), fetal bovine serum (10%), penicillin (100 U/mL), streptomycin (100 µg/mL), and 2-mercaptoethanol (0.05 m*M*). THP1 cells are treated with 100 ng/mL LPS (Sigma-Aldrich) for 24 hrs, the target protein (final conc. 10 µM), or left untreated.

After treatment, THP1 cells are cultured for 24 hrs and then assayed for reporter activity. Both reporter proteins are measurable in the cell culture supernatant when using QUANTI-Blue (InvivoGen), a SEAP detection reagent, and QUANTI-Luc (InvivoGen), a luciferase detection reagent. QUANTI-Blue is a colorimetric enzyme assay developed to determine any alkaline phosphatase activity in a biological sample, such as cell culture supernatant. QUANTI-Luc is a lyophilized assay reagent containing all the components required to quantitatively measure the activity of Lucia Luciferase other coelenterazine-utilizing luciferases. Increased reporter activity after treatment with target protein is observed in cells where the target protein is immune stimulating. Additionally, decreased reporter activity after treatment with the target protein is observed in cells where the target protein is immune dampening.

*scRNA-seq: From example:* Shifts in cell populations after treatment with Target Proteins are assessed by single cell RNA-seq (scRNA-seq). Target proteins (e.g., listed in the Sequence Listing) are synthesized through Fluorenylmethyloxycarbonyl (Fmoc) protection group chemistry using Solid Phase Peptide Synthesis (SPPS). Human primary blood mononuclear cells are seeded at 1x10 PBMC in each well of 12-well plate with 1 ml of RPMI-1640 (10% FBS). PBMCs are treated with 1 µg/mL LPS (Sigma-Aldrich) for 24 hrs, plus or minus target proteins for a final concentration of 10 µM or left untreated. The single cell suspensions are harvested and centrifuged at 400 x g for 5 mins at 4°C. The media is discarded, and the cells are resuspended with 1 ml of cell staining buffer (BioLegend). 5 µl of Human TruStain FcX (BioLegend) is added to the cells and incubated at 4°C for 10 mins. 1 µg of single cell hashing antibody (BioLegend TotalSeq) is added to cells along with ~50 µl of cell staining buffer to make 100 µl total volume. This is incubated for 4°C for 30 mins then washed three times at 400 x g. 10 different cell hashing samples are pooled in one tube with desired cell number (~1000 cells/µl).

Single cells are processed through Chromium Next GEM Single Cell 3' _Reagent Kit (*Dual Index*) (*10X Genomics CG000317 Rev C*) following the provided protocol from 10X Genomics. Briefly, samples are processed through GEM generation and barcoding, post GEM-RT Cleanup and cDNA amplification, 3' gene expression library construction, cell surface protein library construction and finally sequencing.

Post sequencing, reads are aligned to the human reference genome (GENCODE34/GRCH38). Reads are demultiplexed using DNA-barcoded antibodies and run through quality control (*g*. doublets are removed, singlet cells are selected with less than 15% mitochondria contamination at RNA levels and reads contain higher than 500 genes). The raw data is normalized. Principal component analysis is performed, and cell clusters are annotated. Differential gene expression analysis is performed between controls and treatment with target protein. Shifts in cell population dynamics observed by principal component analysis and/or shifts in gene expression post treatment with target protein in supernatant validates that target protein acts as a circulating factor.

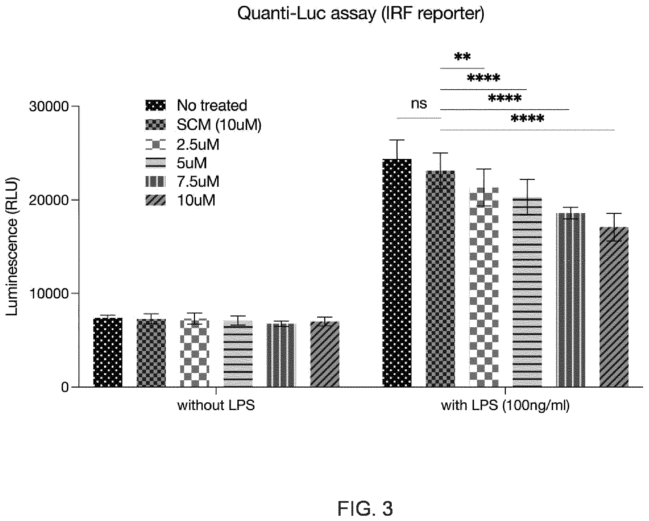

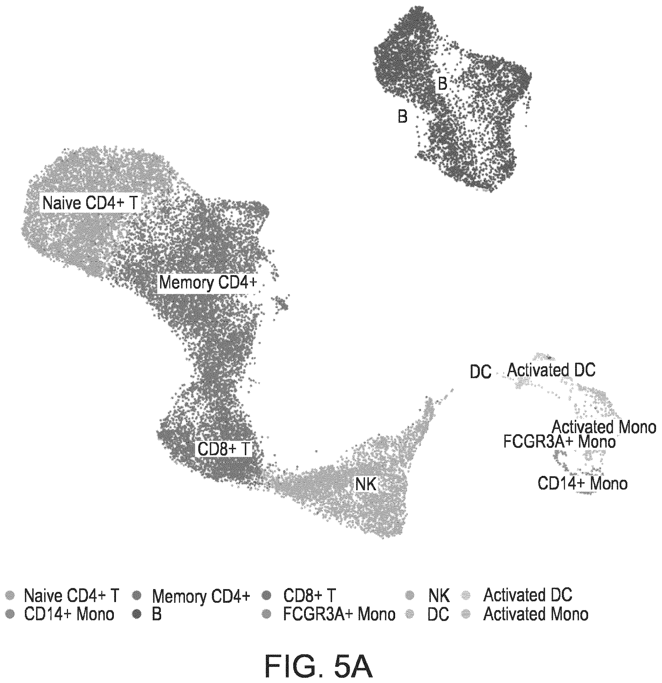

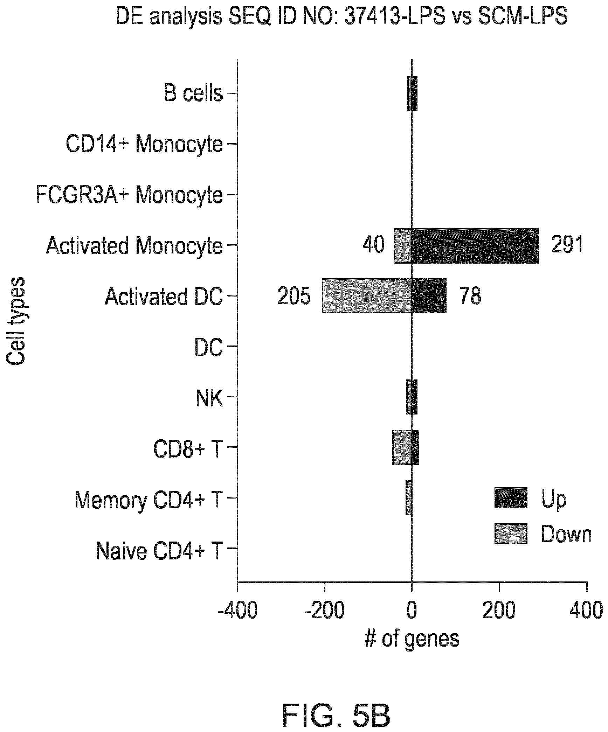

SEQ ID NO: 37413 significantly blocks the IRF signal transduction pathway response from several TLRs. Up-regulation of the IRF pathway is linked to many autoimmune diseases, especially to lupus. Consistent with a connection to innate immune related diseases like lupus, SEQ ID NO: 37413 strongly affected active monocytes and dendritic cells while having relatively small effects on T-cells, B-cells, and NK-cells (FIG. 3, FIG. 4, FIG. 5A, FIG. 5B).

| **ORF ID** | **Relative activity** |
|---|---|
| SEQ ID NO: 33341 | +++ |

### Example 4.

Inhibition of NK cell function by blocking function of co-stimulatory ligands and cytokines is a promising therapeutics strategy to treat allo-immune response and graft rejection. This example demonstrates the validation of Target Proteins, SEQ ID NO: 33482, SEQ ID NO: 37776 and SEQ ID NO: 35050 as novel co-stimulatory ligands that potentiate NK-mediated cytotoxicity in human B cell lymphoma.

CRISPR system was used to generate a pooled KO library comprising 4,708 individual novel proteins within 2,127 lncRNAs and 998 known proteins in Raji cells (human B cell lymphoma). To understand the role of these proteins in modulation of NK cell cytotoxicity we conducted a cell killing assay using primary human NK cells. We examined the change in the initial library representation in Raji cells with the library representation after 24 hours of co-incubation with NK cells. We used known regulators of NK cell cytotoxicity as reference to establish hit significance. The novel co-stimulators (potentiators) of NK-mediated cytotoxicity comprise targets that significantly inhibit Raji cell killing upon CRISPR KO, similar to known controls - ICAM1 and FAS.

### Materials and methods:

*CRISPR KO generation.* CRISPR guide RNA library has been designed at ProFound Therapeutics to target 4,708 distinct predicted novel proteins localized within 2,127 lncRNA, 998 known proteins, including positive (inhibitors, for example B2M, TAP1, TAP2, HLA-DM) and negative (co-stimulators, for example, ICAM1, FAS, IL7, DGKE) of NK cell function, and 900 neutral controls (non-targeting and intergenic guide RNAs). The CRISPR guide RNA library was packaged into lentivirus, transduced into Raji Cas9 line at the MOI of 0.3 and selected using puromycin selection as described in [Sanjana NE, Shalem O, Zhang F. Nat. Methods. 2014 Aug; 11(8):783-4.

*NK isolation and expansion.* Primary human NK cells were isolated from frozen PBMCs using EasySep human NK cell isolation kit (StemCell, catalog # 17955). Primary human NK cells were expanded using immunoCult NK cell expansion kit (StemCell, catalog # 100-0711) following manufacturer protocol.

*Screen setup.* The screen was performed using 4 biological replicates. The NK cytotoxicity assay was performed by co-culturing 20 million Raji CRISPR KO library cells with 60 million primary NK cells for 24 hours in T75 tissue culture flask. NK cells were removed using positive CD56 selection kit (StemCell, catalog # 17855). Dead cells were removed using EasySep dead cell removal (Annexin V) kit (StemCell, catalog # 17899). Remaining Raji CRISPR KO library cells have been processed to extract genomic DNA. NGS library prep and illumina sequencing was performed on these cells and Raji cells prior to exposure to NK as described in [Sanjana NE, Shalem O, Zhang F. Nat. Methods. 2014 Aug; 11(8):783-4.

*Data analysis.* Screen analysis and interpretation was performed using ProFound Tx custom data analysis tools and confirmed with MAGeCK-VISPR as described in [Li W, Koster J, Xu H, Chen CH, Xiao T, Brown M, Liu S. Genome Biology 2015(281)].

We found several lncRNA-encoded proteins that upon loss of function inhibit Raji cell killing in the presence of primary NK cells. The effect size is comparable to control proteins, known modulators of NK cell function such as components of ICAM1 and FAS. SEQ ID NO: 33482, SEQ ID NO: 37776 and SEQ ID NO: 35050 are examples of novel proteins with strong phenotypes suggesting their role in potentiation of NK cell cytotoxicity and predicted cell surface localization.

### Additional novel proteins with validated co-stimulatory effect on NK cytotoxicity with predicted cell surface localization:

SEQ ID NO: 34013, SEQ ID NO: 34021, SEQ ID NO: 33482, SEQ ID NO: 35972, SEQ ID NO: 36456, SEQ ID NO: 37776, SEQ ID NO: 33345, SEQ ID NO: 35531, SEQ ID NO: 36975, SEQ ID NO: 37561, SEQ ID NO: 35050, SEQ ID NO: 34728, SEQ ID NO: 34076

### Additional novel proteins with validated co-stimulatory effect on NK cytotoxicity with predicted secretion:

SEQ ID NO: 37151, SEQ ID NO: 37187, SEQ ID NO: 37199, SEQ ID NO: 38297, SEQ ID NO: 34018, SEQ ID NO: 37016, SEQ ID NO: 33395, SEQ ID NO: 33372, SEQ ID NO: 38018, SEQ ID NO: 36168, SEQ ID NO: 34490, SEQ ID NO: 35219, SEQ ID NO: 38378, SEQ ID NO: 37411, SEQ ID NO: 37782, SEQ ID NO: 36984, SEQ ID NO: 37938, SEQ ID NO: 34395, SEQ ID NO: 34586, SEQ ID NO: 34589, SEQ ID NO: 35295, SEQ ID NO: 36972, SEQ ID NO: 37564, SEQ ID NO: 37158, SEQ ID NO: 38247, SEQ ID NO: 38249, SEQ ID NO: 37863, SEQ ID NO: 33904, SEQ ID NO: 38163, SEQ ID NO: 38189, SEQ ID NO: 38195, SEQ ID NO: 38196, SEQ ID NO: 38206

### Example 5.

The impact of NK cell dysfunction in tumor microenvironment is becoming universally recognized, for example, in cancer patients who are refractory to T-cell checkpoint therapies. Identification of novel inhibitory ligands upregulated on tumor cells to induce NK cell disfunction presents a promising therapeutic opportunity whereby antagonists of these inhibitory ligands of NK cells, such as blocking antibodies, could reactivate NK cytotoxicity and promote cancer cell killing.

This example demonstrates the validation of Target Proteins, SEQ ID NO: 2013, SEQ ID NO: 35586 and SEQ ID NO: 36545 as novel inhibitory ligands that suppress NK-mediated cytotoxicity in human B cell lymphoma.

CRISPR system was used to generate a pooled KO library comprising 4,708 individual novel proteins within 2,127 lncRNAs and 998 known proteins in Raji cells (human B cell lymphoma). To understand the role of these proteins in modulation of NK cell cytotoxicity we conducted a cell killing assay using primary human NK cells. We examined the change in the initial library representation in Raji cells with the library representation after 24 hours of co-incubation with NK cells. We used known regulators of NK cell cytotoxicity as reference to establish hit significance. The novel inhibitors of NK-mediated cytotoxicity comprise targets that significantly potentiate Raji cell killing upon CRISPR KO, similar to known controls - B2M and TAP1.

### Materials and methods:

*CRISPR KO generation.* CRISPR guide RNA library has been designed at ProFound Therapeutics to target 4,708 distinct predicted novel proteins localized within 2,127 lncRNA, 998 known proteins, including positive (inhibitors, for example B2M, TAP1, TAP2, HLA-DM) and negative (co-stimulators, for example, ICAM1, FAS, IL7, DGKE) of NK cell function, and 900 neutral controls (non-targeting and intergenic guide RNAs). The CRISPR guide RNA library was packaged into lentivirus, transduced into Raji Cas9 line at the MOI of 0.3 and selected using puromycin selection as described in [Sanjana NE, Shalem O, Zhang F. Nat. Methods. 2014 Aug; 11(8):783-4.

*NK isolation and expansion.* Primary human NK cells were isolated from frozen PBMCs using EasySep human NK cell isolation kit (StemCell, catalog # 17955). Primary human NK cells were expanded using immunoCult NK cell expansion kit (StemCell, catalog # 100-0711) following manufacturer protocol.

*Screen setup.* The screen was performed using 4 biological replicates. The NK cytotoxicity assay was performed by co-culturing 20 million Raji CRISPR KO library cells with 60 million primary NK cells for 24 hours in T75 tissue culture flask. NK cells were removed using positive CD56 selection kit (StemCell, catalog # 17855). Dead cells were removed using EasySep dead cell removal (Annexin V) kit (StemCell, catalog # 17899). Remaining Raji CRISPR KO library cells have been processed to extract genomic DNA. NGS library prep and illumina sequencing was performed on these cells and Raji cells prior to exposure to NK as described in [Sanjana NE, Shalem O, Zhang F. Nat. Methods. 2014 Aug; 11(8):783-4.

*Data analysis.* Screen analysis and interpretation has been performed using ProFound Tx custom data analysis tools and confirmed with MAGeCK-VISPR as described in [Li W, Koster J, Xu H, Chen CH, Xiao T, Brown M, Liu S. Genome Biology 2015(281)].

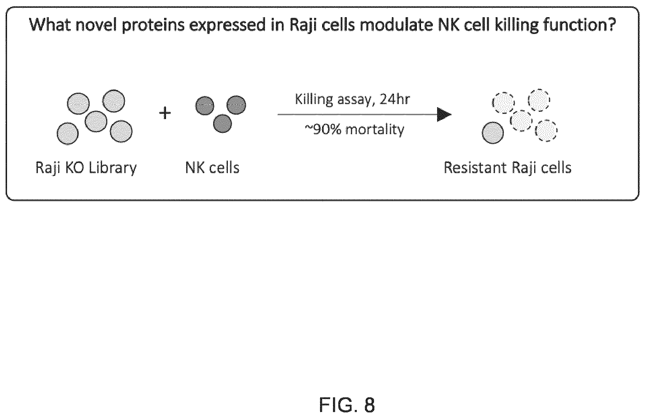

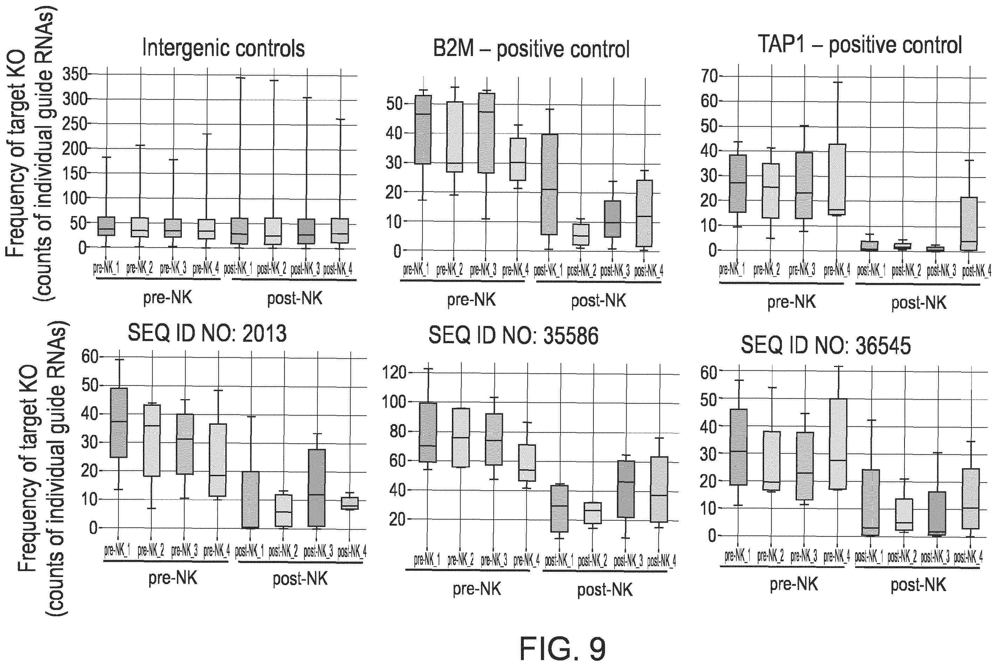

We found several lncRNA encoded proteins that upon loss of function potentiate Raji cell killing in the presence of primary NK cells. The effect size of these is comparable to control proteins, known modulators of NK cell function such as components of MHC (B2M, TAP1, TAP2). SEQ ID NO: 2013, SEQ ID NO: 35586 and SEQ ID NO: 36545 are examples of novel proteins with strong phenotypes and predicted cell surface localization (FIG. 8 and FIG. 9).

### Additional novel proteins with validated inhibitory effect on NK cytotoxicity with predicted cell surface localization:

SEQ ID NO: 35361, SEQ ID NO: 33359, SEQ ID NO: 33807, SEQ ID NO: 33817, SEQ ID NO: 35236, SEQ ID NO: 36386, SEQ ID NO: 37561

### Additional novel proteins with validated inhibitory effect on NK cytotoxicity with predicted secretion:

SEQ ID NO: 38261, SEQ ID NO: 33454, SEQ ID NO: 33510, SEQ ID NO: 33225, SEQ ID NO: 33247, SEQ ID NO: 36705, SEQ ID NO: 38128, SEQ ID NO: 35759, SEQ ID NO: 36769, SEQ ID NO: 36771, SEQ ID NO: 34633, SEQ ID NO: 36461, SEQ ID NO: 37622, SEQ ID NO: 37640, SEQ ID NO: 38369, SEQ ID NO: 37377, SEQ ID NO: 37958, SEQ ID NO: 35900, SEQ ID NO: 37565, SEQ ID NO: 37099, SEQ ID NO: 33521, SEQ ID NO: 33548, SEQ ID NO: 33683, SEQ ID NO: 33704, SEQ ID NO: 34374, SEQ ID NO: 35661, SEQ ID NO: 37816

The teachings of all patents, published applications and references cited herein are incorporated by reference in their entirety.

While example embodiments have been particularly shown and described, it will be understood by those skilled in the art that various changes in form and details may be made therein without departing from the scope of the embodiments encompassed by the appended claims.

### EMBODIMENTS

1. An agent that comprises and/or modulates the expression or activity of a target protein identified in the Sequence Listing, or a variant thereof.
2. The agent of embodiment 1, wherein the agent comprises the target protein.
3. The agent of embodiment 1, wherein the agent modulates the expression of the target protein.
4. The agent of embodiment 2 or 3, wherein the agent modulates the expression of a gene or gene transcript encoding the target protein.
5. The agent of embodiment 1, wherein the agent modulates the activity of the target protein.
6. The agent of any one of embodiments 2-5, comprising a polypeptide, a polynucleotide, or a small molecule.
7. The agent of any one of embodiments 2-6, wherein the agent decreases the expression or activity of the target protein.
8. The agent of embodiment 7, wherein the agent comprises an inhibitor of the target protein.
9. The agent of embodiment 8, wherein the inhibitor is a polypeptide.
10. The agent of embodiment 9, wherein the polypeptide is an antagonist antibody, or an antigen-binding fragment thereof, that binds to the target protein.
11. The agent of embodiment 8, wherein the inhibitor is a polynucleotide.
12. The agent of embodiment 11, wherein the polynucleotide comprises a nucleotide sequence that is complementary to at least a portion of a gene or gene transcript encoding the target protein.
13. The agent of embodiment 11 or 12, wherein the polynucleotide comprises DNA.
14. The agent of embodiment 11 or 12, wherein the polynucleotide comprises RNA.
15. The agent of embodiment 11 or 12, wherein the polynucleotide is a small interfering RNA (siRNA), a short hairpin RNA (shRNA), an antisense DNA, an antisense RNA, a microRNA (miRNA), an antagomir, a guide RNA (gRNA), a locked nucleic acid (LNA) or a morpholino nucleic acid (MNA).
16. The agent of embodiment 8, wherein the inhibitor is a small molecule.
17. The agent of embodiment 16, wherein the small molecule binds to the target protein, thereby decreasing the activity of the target protein.
18. The agent of any one of embodiments 1-6, wherein the agent increases the expression or activity of the target protein.
19. The agent of embodiment 18, wherein the agent is an isolated polypeptide comprising the amino acid sequence of the target protein, or a variant thereof comprising an amino acid sequence having at least 80% sequence identity to the amino acid sequence of the target protein.
20. The agent of embodiment 19, wherein the isolated polypeptide or variant thereof is a recombinant protein or a synthetic protein.
21. The agent of embodiment 18, wherein the agent is a polynucleotide encoding the target protein or a variant thereof having at least 80% sequence identity to the amino acid sequence of the target protein.
22. The agent of embodiment 21, wherein the polynucleotide comprises DNA.
23. The agent of embodiment 21 or 22, wherein the polynucleotide comprises a vector.
24. The agent of embodiment 21, wherein the polynucleotide comprises RNA.
25. The agent of embodiment 24, wherein the RNA is messenger RNA (mRNA) and/or circular RNA (circRNA).
26. The agent of embodiment 18, wherein the agent comprises an activator of the target protein.
27. The agent of embodiment 26, wherein the activator is a polypeptide.
28. The agent of embodiment 27, wherein the polypeptide is an agonist antibody, or an antigen-binding fragment thereof, that binds to the target protein.
29. The agent of embodiment 26, wherein the activator is a small molecule.
30. The agent of embodiment 29, wherein the small molecule binds to the target protein, thereby increasing the activity of the target protein.
31. The agent of any one of embodiments 1-8, 18 and 26, comprising a gene editing system.
32. The agent of embodiment 31, wherein the gene editing system is a CRISPR/Cas system, a transposon-based gene editing system, and a transcription activator-like effector nuclease (TALEN) system.
33. The agent of any one of embodiments 1-32, wherein the target protein is translated from a non-coding RNA.
34. The agent of embodiment 33, wherein the non-coding RNA is a long intergenic non-coding RNA (lincRNA).
35. The agent of any one of embodiments 1-32, wherein the target protein is translated from a non-exonic element in an unprocessed precursor mRNA (pre-mRNA).
36. The agent of embodiment 35, wherein the non-exonic element is an intron in a pre-mRNA.
37. The agent of embodiment 35, wherein the non-exonic element is a 5'-untranslated region (5'-UTR) in a pre-mRNA.
38. The agent of embodiment 35, wherein the non-exonic element is a 3'-untranslated region (3'-UTR) in a pre-mRNA.
39. A pharmaceutical composition comprising the agent of any one of embodiments 1-38, and a pharmaceutically acceptable carrier.
40. The pharmaceutical composition of embodiment 39, wherein the pharmaceutically acceptable carrier is a vector.
41. A method for modulating the expression or activity of a target protein identified in the Sequence Listing, or a variant thereof in a cell, comprising contacting the cell with the agent of any one of embodiments 1-38 or the pharmaceutical composition of embodiment 39 or 40.
42. The method of embodiment 41, wherein the agent decreases the expression or activity of the target protein in the cell.
43. The method of embodiment 41, wherein the agent increases the expression or activity of the target protein in the cell.
44. The method of any one of embodiments 41-43, wherein the cell is in a subject, and wherein the cell is:
   a) a cancer cell, a cell in a tumor micro-environment, or a combination thereof;
   b) an epithelial cell, an endothelial cell, a stem cell, a non-immune cell, or a combination thereof;
   c) an immune cell, or
   a combination of the foregoing.
45. The method of embodiment 44, wherein the subject has a disease or condition selected from aging, senescence, fibrosis, autoimmunity, cancer, an infection, an immunological disease, or a combination thereof
46. The method of any one of embodiments 41-45, wherein the agent modulates the expression or activity of the target protein by at least 10%.
47. The method of any one of embodiments 41-46, wherein the agent modulates the expression of a gene encoding the target protein by at least 10%, thereby modulating the expression or activity of the target protein.
48. A method of predicting a likelihood of developing a disease or condition in a subject, comprising quantifying an expression or activity of a target protein identified in the Sequence Listing in a sample from the subject, wherein the level of expression or activity of the target protein in the sample is indicative of the likelihood of developing cancer in the subject, wherein the disease or condition is selected from aging, senescence, fibrosis, autoimmunity, cancer, an infection, an immunological disease, or a combination thereof.
49. A method of preparing a sample that is useful for detecting a likelihood of developing a disease or condition in a subject, comprising:
   a) obtaining or having obtained a sample from the subject;
   b) adding a protease inhibitor, a control peptide, a standard peptide, or a combination thereof to the sample to prepare a sample that is useful for detecting a likelihood of developing cancer; and
   c) quantifying an expression or activity of a target protein identified in the Sequence Listing in the sample prepared in step b),
   wherein the disease or condition is selected from aging, senescence, fibrosis, autoimmunity, cancer, an infection, an immunological disease, or a combination thereof.
50. The method of embodiment 48 or 49, further comprising administering to the subject an effective amount of the agent of any one of embodiments 1-38, or the pharmaceutical composition of embodiment 39 or 40 if the subject is predicted to have a likelihood of developing cancer.
51. A method of treating a disease or condition in a subject in need thereof, comprising administering to the subject an effective amount of the agent of any one of embodiments 1-38, or the pharmaceutical composition of embodiment 39 or 40, wherein the disease or condition is selected from aging, senescence, fibrosis, autoimmunity, cancer, an infection, an immunological disease, or a combination thereof.
52. A method for identifying an agent that modulates the expression or activity of a target protein identified in the Sequence Listing, or a variant thereof, comprising:
   a) contacting a protein identified in the Sequence Listing, or a variant thereof with an agent; and
   b) determining whether the agent modulates the expression or activity of the target protein,
   wherein a difference in the expression or activity of the target protein that has been contacted with the agent compared to a reference for the expression or activity of the target protein indicates that the agent modulates the expression or activity of the target protein.
53. The method of embodiment 52, wherein a difference of at least 10% in the expression or activity of the target protein that has been contacted with the agent compared to the reference indicates that the agent modulates the expression or activity of the target protein.
54. The method of embodiment 52 or 53, wherein a decrease in the expression or activity of the target protein that has been contacted with the agent compared to the reference indicates the agent inhibits the expression or activity of the target protein.
55. The method of embodiment 52 or 53, wherein an increase in the expression or activity of the target protein compared to the reference indicates the agent activates the expression or activity of the target protein.

## Claims

1. An agent that comprises and/or modulates the expression or activity of a target protein comprising the amino acid sequence of SEQ ID NO: 33482 or a variant thereof.

2. The agent of claim 1, wherein the agent comprises the target protein, and/or wherein the agent modulates the expression of a gene or gene transcript encoding the target protein.

3. The agent of claim 1 or 2 , comprising a polypeptide, a polynucleotide, or a small molecule.

4. The agent of any one of claims 1-3, wherein the agent increases the expression or activity of the target protein, optionally wherein the agent is:
an isolated polypeptide comprising the amino acid sequence of the target protein, or a variant thereof comprising an amino acid sequence having at least 80% sequence identity to the amino acid sequence of the target protein, optionally wherein the isolated polypeptide or variant thereof is a recombinant protein or a synthetic protein; or
a polynucleotide encoding the target protein or a variant thereof having at least 80% sequence identity to the amino acid sequence of the target protein, optionally wherein
the polynucleotide comprises:
DNA and/or a vector; or
RNA, optionally wherein the RNA is messenger RNA (mRNA) or circular RNA (circRNA).

5. The agent of claim 4, wherein the agent comprises an activator of the target protein, optionally wherein the activator is:
a polypeptide, optionally wherein the polypeptide is an agonist antibody, or an antigen-binding fragment thereof, that binds to the target protein; or
a small molecule, optionally wherein the small molecule binds to the target protein, thereby increasing the activity of the target protein.

6. The agent of any one of claims 1-5, comprising a gene editing system, optionally wherein the gene editing system is a CRISPR/Cas system, a transposon-based gene editing system, or a transcription activator-like effector nuclease (TALEN) system.

7. A pharmaceutical composition comprising the agent of any one of claims 1-6 and a pharmaceutically acceptable carrier, optionally wherein the pharmaceutically acceptable carrier is a vector.

8. An ex vivo method for modulating the expression or activity of a target protein comprising the amino acid sequence of SEQ ID NO: 33482 or a variant thereof in a cell, comprising contacting the cell with the agent of any one of claims 1-6 or the pharmaceutical composition of claim 7;
optionally wherein the cell is:
a) a cancer cell, a cell in a tumor micro-environment, or a combination thereof;
b) an epithelial cell, an endothelial cell, a stem cell, a non-immune cell, or a combination thereof;
c) an immune cell; or
d) a combination of the foregoing; and
optionally wherein the agent modulates the expression or activity of the target protein by at least 10%, optionally wherein the agent modulates the expression of a gene encoding the target protein by at least 10%, thereby modulating the expression or activity of the target protein.

9. The method of claim 8, wherein the agent increases the expression or activity of the target protein in the cell.

10. A method of predicting a likelihood of developing a disease or condition in a subject, comprising quantifying an expression or activity of a target protein comprising the amino acid sequence of SEQ ID NO: 33482 in a sample from the subject, wherein the level of expression or activity of the target protein in the sample is indicative of the likelihood of developing the disease or condition in the subject, wherein the disease or condition is selected from aging, senescence, fibrosis, autoimmunity, cancer, an infection, an immunological disease, or a combination thereof;
optionally wherein the method further comprises use of an effective amount of the agent of any one of claims 1-6, or the pharmaceutical composition of claim 7 for administering to the subject if the subject is predicted to have a likelihood of developing the disease or condition.

11. A method of preparing a sample that is useful for detecting a likelihood of developing a disease or condition in a subject, comprising:
a) obtaining or having obtained a sample from the subject;
b) adding a protease inhibitor, a control peptide, a standard peptide, or a combination thereof to the sample to prepare a sample that is useful for detecting a likelihood of developing cancer; and
c) quantifying an expression or activity of a target protein identified in the Sequence Listing in the sample prepared in step b),
wherein the disease or condition is selected from aging, senescence, fibrosis, autoimmunity, cancer, an infection, an immunological disease, or a combination thereof;
optionally wherein the method further comprises use of an effective amount of the agent of any one of claims 1-6, or the pharmaceutical composition of claim 7 for administering to the subject if the subject is predicted to have a likelihood of developing the disease or condition.

12. The agent of any one of claims 1-6, or the pharmaceutical composition of claim 7, for use in a method of treating a disease or condition in a subject in need thereof, wherein the disease or condition is selected from aging, senescence, fibrosis, autoimmunity, cancer, an infection, an immunological disease, or a combination thereof.

13. A method for identifying an agent that modulates the expression or activity of a target protein comprising the amino acid sequence of SEQ ID NO: 33482 or a variant thereof, comprising:
a) contacting the protein or the variant thereof with the agent; and
b) determining whether the agent modulates the expression or activity of the target protein,
wherein a difference in the expression or activity of the target protein that has been contacted with the agent compared to a reference for the expression or activity of the target protein indicates that the agent modulates the expression or activity of the target protein;
optionally wherein a difference of at least 10% in the expression or activity of the target protein that has been contacted with the agent compared to the reference indicates that the agent modulates the expression or activity of the target protein; and
optionally wherein a decrease in the expression or activity of the target protein that has been contacted with the agent compared to the reference indicates the agent inhibits the expression or activity of the target protein, or an increase in the expression or activity of the target protein compared to the reference indicates the agent activates the expression or activity of the target protein.

14. A method for potentiating natural killer (NK) cell cytotoxicity, comprising contacting a NK cell with the agent of any one of claims 1-6 or the pharmaceutical composition of claim 7.

15. The method of any one of claims 8 and 9-12, or the agent or pharmaceutical composition for use of claim 12, wherein the cancer is a hematologic cancer, optionally wherein the hematologic cancer is a lymphoma, and optionally wherein the lymphoma is a B cell lymphoma.
